# EUROPEAN PATENT APPLICATION

(11) **EP 3 750 560 A2**
(43) Date of publication of application: **16.12.2020**
(21) Application number: 20173044.7
(22) Date of filing: 08.10.2013
(51) Int. Cl.: A61K 39/395, C07K 16/28, A61P 25/28, A61K 39/00, A61K 31/56, A61K 38/21, A61P 25/00, A61P 29/00, A61P 43/00, A61P 27/02

(54) **COMBINATION THERAPIES AND USES FOR TREATMENT OF DEMYELINATING DISORDERS**

(30) Priority: 09.10.2012 US 201261711638 P
(62) Divisional of application: 13780458.9
(71) Applicant: Biogen MA Inc., Cambridge, MA 02142 (US)
(72) Inventor: CADAVID,, Diego, Concord,, MA Massachusetts 01742 (US); MI,, Sha, Belmont,, MA Massachusetts 02478 (US)
(74) Representative: Miller, David James

(57) **Abstract**

Methods and compositions for enhancing one or more of: myelination, re-myelination, oligodendrocyte numbers, or neuroaxonal protection, while ameliorating an inflammatory condition in a human subject are disclosed. In certain embodiments, the methods and compositions described herein include a reparative agent (*e.g*., a LINGO-1 antagonist) and an immunomodulatory agent, in combination. Thus, methods, compositions and kits described herein can be useful for treating a CNS demyelinating disease.

SEQUENCE LISTING

<110>  Biogen MA Inc.

<120>  COMBINATION THERAPIES AND USES FOR TREATMENT OF DEMYELINATING
       DISORDERS

<130>  P45082EP-D1-PCT

<140>  To be assigned
<141>  2013-10-08

<150>  US 61/711,638
<151>  2012-10-09

<160>  276

<170>  PatentIn version 3.5

<210>  1
<211>  119
<212>  PRT
<213>  Artificial

<220>
<223>  VH sequence of Li62 antibody

<400>  1

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  2
<211>  5

<212>  PRT
<213>  Artificial

<220>
<223>  VH CDR1 sequence of Li62 antibody

<400>  2

Ile Tyr Pro Met Phe
1               5


<210>  3
<211>  17
<212>  PRT
<213>  Artificial

<220>
<223>  VH CDR2 sequence of Li62 antibody

<400>  3

Trp Ile Gly Pro Ser Gly Gly Ile Thr Lys Tyr Ala Asp Ser Val Lys
1               5                   10                  15


Gly


<210>  4
<211>  10
<212>  PRT
<213>  Artificial

<220>
<223>  VH CDR3 sequence of Li62 antibody

<400>  4

Glu Gly His Asn Asp Trp Tyr Phe Asp Leu
1               5                   10


<210>  5
<211>  118
<212>  PRT
<213>  Artificial

<220>
<223>  VH sequence of Li81 antibody

<400>  5

<210>  6
<211>  5
<212>  PRT
<213>  Artificial

<220>
<223>  VH CDR1 sequence of Li81 antibody

<400>  6

Ala Tyr Glu Met Lys
1               5

<210>  7
<211>  17
<212>  PRT
<213>  Artificial

<220>
<223>  VH CDR2 sequence of Li81 antibody

<400>  7

Val Ile Gly Pro Ser Gly Gly Phe Thr Phe Tyr Ala Asp Ser Val Lys
1               5                   10                  15

Gly

<210>  8
<211>  9
<212>  PRT
<213>  Artificial

<220>
<223>  VH CDR3 sequence of Li81 antibody

<400>  8

Glu Gly Asp Asn Asp Ala Phe Asp Ile
1               5


<210>  9
<211>  107
<212>  PRT
<213>  Artificial

<220>
<223>  VL sequence of Li62 antibody

<400>  9

Asp Ile Gln Met Thr Gln Ser Pro Ser Phe Leu Ser Ala Ser Val Gly
1               5                   10                  15


Asp Ser Val Ala Ile Thr Cys Arg Ala Ser Gln Asp Ile Ser Arg Tyr
            20                  25                  30


Leu Ala Trp Tyr Gln Gln Arg Pro Gly Lys Ala Pro Lys Leu Leu Ile
        35                  40                  45


Tyr Asp Ala Ser Asn Leu Gln Thr Gly Val Pro Ser Arg Phe Ser Gly
    50                  55                  60


Ser Gly Ser Gly Thr Asp Phe Thr Phe Thr Ile Thr Ser Leu Gln Pro
65                  70                  75                  80


Glu Asp Phe Gly Thr Tyr Tyr Cys Gln Gln Tyr Asp Thr Leu His Pro
                85                  90                  95


Ser Phe Gly Pro Gly Thr Thr Val Asp Ile Lys
            100                 105


<210>  10
<211>  11
<212>  PRT
<213>  Artificial

<220>
<223>  VL CDR1 sequence of Li62 antibody

<400>  10

Arg Ala Ser Gln Asp Ile Ser Arg Tyr Leu Ala
1               5                   10


<210>  11
<211>  7
<212>  PRT
<213>  Artificial

<220>
<223>  VL CDR2 sequence of Li62 antibody

<400>  11

Asp Ala Ser Asn Leu Gln Thr
1               5


<210>  12
<211>  9
<212>  PRT
<213>  Artificial

<220>
<223>  VL CDR3 sequence of Li62 antibody

<400>  12

Gln Gln Tyr Asp Thr Leu His Pro Ser
1               5


<210>  13
<211>  108
<212>  PRT
<213>  Artificial

<220>
<223>  VL sequence of Li81 antibody

<400>  13

Asp Ile Gln Met Thr Gln Ser Pro Ala Thr Leu Ser Leu Ser Pro Gly
1               5                   10                  15


Glu Arg Ala Thr Leu Ser Cys Arg Ala Ser Gln Ser Val Ser Ser Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Arg Ser Asn Trp Pro Met
                85                  90                  95


Tyr Thr Phe Gly Gln Gly Thr Lys Leu Glu Ile Lys
            100                 105


<210>  14
<211>  11

<212>  PRT
<213>  Artificial

<220>
<223>  VL CDR1 sequence of Li81 antibody

<400>  14

Arg Ala Ser Gln Ser Val Ser Ser Tyr Leu Ala
1               5                   10


<210>  15
<211>  7
<212>  PRT
<213>  Artificial

<220>
<223>  VL CDR2 sequence of Li81 antibody

<400>  15

Asp Ala Ser Asn Arg Ala Thr
1               5


<210>  16
<211>  10
<212>  PRT
<213>  Artificial

<220>
<223>  VL CDR3 sequence of Li81 antibody

<400>  16

Gln Gln Arg Ser Asn Trp Pro Met Tyr Thr
1               5                   10


<210>  17
<211>  10
<212>  PRT
<213>  Artificial

<220>
<223>  VH CDR3 sequence of Li62 variant B06 antibody

<400>  17

Glu Gly Tyr Tyr Asp Trp Tyr Phe Asp Gln
1               5                   10


<210>  18
<211>  10
<212>  PRT
<213>  Artificial

<220>
<223>  VH CDR3 sequence of Li62 variant B12 antibody

<400>  18

Glu Gly Gln Tyr Asp Trp Tyr Phe Asp Val
1               5                   10

<210>  19
<211>  10
<212>  PRT
<213>  Artificial

<220>
<223>  VH CDR3 sequence of Li62 variant F06 antibody

<400>  19

Glu Gly Asp Tyr Asp Trp Tyr Phe Asp Leu
1               5                   10

<210>  20
<211>  10
<212>  PRT
<213>  Artificial

<220>
<223>  VH CDR3 sequence of Li62 variant B01 antibody

<400>  20

Glu Gly Gln Tyr Asp Trp Tyr Phe Glu Leu
1               5                   10

<210>  21
<211>  10
<212>  PRT
<213>  Artificial

<220>
<223>  VH CDR3 sequence of Li62 variant D09 antibody

<400>  21

Glu Ala Asp Ile Asp Trp Phe Phe Asp Leu
1               5                   10

<210>  22
<211>  10
<212>  PRT
<213>  Artificial

<220>
<223>  VH CDR3 sequence of Li62 variant D12 antibody

<400>  22

Glu Gly His Tyr Asp Trp Tyr Phe Asp Leu
1               5                   10

<210>  23
<211>  10

<212>  PRT
<213>  Artificial

<220>
<223>  VH CDR3 sequence of Li62 variant F01 antibody

<400>  23

Glu Gly Arg Tyr Asp Trp Tyr Phe Asp Pro
1               5                   10


<210>  24
<211>  10
<212>  PRT
<213>  Artificial

<220>
<223>  VH CDR3 sequence of Li62 variant F02 antibody

<400>  24

Glu Gly Asp Tyr Asp Trp Tyr Phe Gly Leu
1               5                   10


<210>  25
<211>  10
<212>  PRT
<213>  Artificial

<220>
<223>  VH CDR3 sequence of Li62 variant F06 antibody

<400>  25

Glu Gly Arg Tyr Asp Trp Tyr Phe Asp Leu
1               5                   10


<210>  26
<211>  10
<212>  PRT
<213>  Artificial

<220>
<223>  VH CDR3 sequence of Li62 variant F10 antibody

<400>  26

Glu Ser His Ile Asp Arg Tyr Phe Asp Leu
1               5                   10


<210>  27
<211>  10
<212>  PRT
<213>  Artificial

<220>
<223>  VH CDR3 sequence of Li62 variant G08 antibody

<400>  27

Glu Gly Gln Tyr Asp Trp Tyr Phe Asp Val
1               5                   10

<210>  28
<211>  10
<212>  PRT
<213>  Artificial

<220>
<223>  VH CDR3 sequence of Li62 variant H08 antibody

<400>  28

Glu Gly His Tyr Asn Gly Tyr Phe Asp Leu
1               5                   10

<210>  29
<211>  10
<212>  PRT
<213>  Artificial

<220>
<223>  VH CDR3 sequence of Li62 variant C10 antibody

<400>  29

Glu Gly Tyr Tyr Asp Trp Tyr Phe Asp Leu
1               5                   10

<210>  30
<211>  10
<212>  PRT
<213>  Artificial

<220>
<223>  VH CDR3 sequence of Li62 variant C02 antibody

<400>  30

Glu Gly Thr Tyr Asp Trp Tyr Leu Asp Leu
1               5                   10

<210>  31
<211>  10
<212>  PRT
<213>  Artificial

<220>
<223>  VH CDR3 sequence of Li62 variant D05 antibody

<400>  31

Glu Gly Tyr Tyr Asp Trp Tyr Phe Glu Leu
1               5                   10

<210>  32
<211>  10

<212>  PRT
<213>  Artificial

<220>
<223>  VH CDR3 sequence of Li62 variant F02 antibody

<400>  32

Glu Gly Leu Ile Asp Trp Phe Phe Asp Gln
1               5                   10


<210>  33
<211>  10
<212>  PRT
<213>  Artificial

<220>
<223>  VH CDR3 sequence of Li62 variant C10 antibody

<400>  33

Glu Gly Gln Phe Asp Trp Tyr Phe Asp Leu
1               5                   10


<210>  34
<211>  10
<212>  PRT
<213>  Artificial

<220>
<223>  VH CDR3 sequence of Li62 variant H08 antibody

<400>  34

Glu Gly Thr Tyr Asp Trp Tyr Phe Asp Leu
1               5                   10


<210>  35
<211>  9
<212>  PRT
<213>  Artificial

<220>
<223>  VH CDR3 sequence of Li81 variant F09 antibody

<400>  35

Glu Gly Glu Asn Asp Ala Phe Asp Val
1               5


<210>  36
<211>  9
<212>  PRT
<213>  Artificial

<220>
<223>  VH CDR3 sequence of Li81 variant G02 antibody

<400>  36

Glu Gly Asp Asn Asp Ala Tyr Asp Thr
1               5

<210>  37
<211>  9
<212>  PRT
<213>  Artificial

<220>
<223>  VH CDR3 sequence of Li81 variant H03 antibody

<400>  37

Glu Gly Thr Asn Asp Ala Phe Asp Ile
1               5

<210>  38
<211>  9
<212>  PRT
<213>  Artificial

<220>
<223>  VH CDR3 sequence of Li81 variant A12 antibody

<400>  38

Glu Gly Asp Asn Asp Ala Phe Asp Ser
1               5

<210>  39
<211>  9
<212>  PRT
<213>  Artificial

<220>
<223>  VH CDR3 sequence of Li81 variant C02 antibody

<400>  39

Glu Gly Asp Asn Asp Ala Phe Asp Thr
1               5

<210>  40
<211>  9
<212>  PRT
<213>  Artificial

<220>
<223>  VH CDR3 sequence of Li81 variant C11 antibody

<400>  40

Glu Gly Asp Asn Asp Ala Tyr Asp Arg
1               5

<210>  41
<211>  9

<212>  PRT
<213>  Artificial

<220>
<223>  VH CDR3 sequence of Li81 variant D11 antibody

<400>  41

Glu Gly Asp Asn Asp Val Phe Asp Ser
1               5


<210>  42
<211>  9
<212>  PRT
<213>  Artificial

<220>
<223>  VH CDR3 sequence of Li81 variant E05 antibody

<400>  42

Glu Gly Asp Asp Asp Val Phe Asp Met
1               5


<210>  43
<211>  9
<212>  PRT
<213>  Artificial

<220>
<223>  VH CDR3 sequence of Li81 variant H04 antibody

<400>  43

Glu Gly Tyr Asn Asp Ala Phe Asp Phe
1               5


<210>  44
<211>  9
<212>  PRT
<213>  Artificial

<220>
<223>  VH CDR3 sequence of Li81 variant B04 antibody

<400>  44

Glu Gly Asp Asp Asp Ala Tyr Asp Met
1               5


<210>  45
<211>  9
<212>  PRT
<213>  Artificial

<220>
<223>  VH CDR3 sequence of Li81 variant A02 antibody

<400>  45

Glu Gln Asp Tyr Asp Thr Tyr Asp Leu
1               5

<210>  46
<211>  9
<212>  PRT
<213>  Artificial

<220>
<223>  VH CDR3 sequence of Li81 variant B12 antibody

<400>  46

Glu Gly Asp Asp Asp Ala Phe Asp Thr
1               5

<210>  47
<211>  9
<212>  PRT
<213>  Artificial

<220>
<223>  VH CDR3 sequence of Li81 variant H06 antibody

<400>  47

Glu Ala Asp Asp Asp Ala Phe Asp Ile
1               5

<210>  48
<211>  9
<212>  PRT
<213>  Artificial

<220>
<223>  VH CDR3 sequence of Li81 variant H08 antibody

<400>  48

Glu Gly Glu Asn Asp Ala Phe Asp Met
1               5

<210>  49
<211>  9
<212>  PRT
<213>  Artificial

<220>
<223>  VH CDR3 sequence of Li81 variant E07 antibody

<400>  49

Glu Gly Glu Tyr Asp Thr Tyr Asp Ile
1               5

<210>  50
<211>  447

<212>  PRT
<213>  Artificial

<220>
<223>  Aglycosylated Li81 heavy chain

<400>  50

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Ala Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445

<210>  51
<211>  614
<212>  PRT
<213>  Homo sapiens

<400>  51

Met Leu Ala Gly Gly Val Arg Ser Met Pro Ser Pro Leu Leu Ala Cys
1               5                   10                  15

Trp Gln Pro Ile Leu Leu Leu Val Leu Gly Ser Val Leu Ser Gly Ser
            20                  25                  30

Ala Thr Gly Cys Pro Pro Arg Cys Glu Cys Ser Ala Gln Asp Arg Ala
        35                  40                  45

Val Leu Cys His Arg Lys Arg Phe Val Ala Val Pro Glu Gly Ile Pro
    50                  55                  60

Thr Glu Thr Arg Leu Leu Asp Leu Gly Lys Asn Arg Ile Lys Thr Leu
65                  70                  75                  80

Asn Gln Asp Glu Phe Ala Ser Phe Pro His Leu Glu Glu Leu Glu Leu
                85                  90                  95

Asn Glu Asn Ile Val Ser Ala Val Glu Pro Gly Ala Phe Asn Asn Leu
            100                 105                 110

Phe Asn Leu Arg Thr Leu Gly Leu Arg Ser Asn Arg Leu Lys Leu Ile
        115                 120                 125

Pro Leu Gly Val Phe Thr Gly Leu Ser Asn Leu Thr Lys Leu Asp Ile
    130                 135                 140

Ser Glu Asn Lys Ile Val Ile Leu Leu Asp Tyr Met Phe Gln Asp Leu
145                 150                 155                 160

Tyr Asn Leu Lys Ser Leu Glu Val Gly Asp Asn Asp Leu Val Tyr Ile
                165                 170                 175

Ser His Arg Ala Phe Ser Gly Leu Asn Ser Leu Glu Gln Leu Thr Leu
            180                 185                 190

Glu Lys Cys Asn Leu Thr Ser Ile Pro Thr Glu Ala Leu Ser His Leu
        195                 200                 205

His Gly Leu Ile Val Leu Arg Leu Arg His Leu Asn Ile Asn Ala Ile
    210                 215                 220

Arg Asp Tyr Ser Phe Lys Arg Leu Tyr Arg Leu Lys Val Leu Glu Ile
225                 230                 235                 240

Ser His Trp Pro Tyr Leu Asp Thr Met Thr Pro Asn Cys Leu Tyr Gly
                245                 250                 255

Leu Asn Leu Thr Ser Leu Ser Ile Thr His Cys Asn Leu Thr Ala Val
            260                 265                 270

Pro Tyr Leu Ala Val Arg His Leu Val Tyr Leu Arg Phe Leu Asn Leu
        275                 280                 285

Ser Tyr Asn Pro Ile Ser Thr Ile Glu Gly Ser Met Leu His Glu Leu
    290                 295                 300

Leu Arg Leu Gln Glu Ile Gln Leu Val Gly Gly Gln Leu Ala Val Val
305                 310                 315                 320

Glu Pro Tyr Ala Phe Arg Gly Leu Asn Tyr Leu Arg Val Leu Asn Val
                325                 330                 335

Ser Gly Asn Gln Leu Thr Thr Leu Glu Glu Ser Val Phe His Ser Val
            340                 345                 350

Gly Asn Leu Glu Thr Leu Ile Leu Asp Ser Asn Pro Leu Ala Cys Asp
        355                 360                 365

Cys Arg Leu Leu Trp Val Phe Arg Arg Arg Trp Arg Leu Asn Phe Asn
    370                 375                 380

Arg Gln Gln Pro Thr Cys Ala Thr Pro Glu Phe Val Gln Gly Lys Glu
385                 390                 395                 400

Phe Lys Asp Phe Pro Asp Val Leu Leu Pro Asn Tyr Phe Thr Cys Arg
                405                 410                 415

Arg Ala Arg Ile Arg Asp Arg Lys Ala Gln Gln Val Phe Val Asp Glu
            420                 425                 430

Gly His Thr Val Gln Phe Val Cys Arg Ala Asp Gly Asp Pro Pro Pro
        435                 440                 445

Ala Ile Leu Trp Leu Ser Pro Arg Lys His Leu Val Ser Ala Lys Ser
    450                 455                 460

Asn Gly Arg Leu Thr Val Phe Pro Asp Gly Thr Leu Glu Val Arg Tyr
465                 470                 475                 480

Ala Gln Val Gln Asp Asn Gly Thr Tyr Leu Cys Ile Ala Ala Asn Ala
                485                 490                 495

Gly Gly Asn Asp Ser Met Pro Ala His Leu His Val Arg Ser Tyr Ser

500                 505                 510

Pro Asp Trp Pro His Gln Pro Asn Lys Thr Phe Ala Phe Ile Ser Asn
        515                 520                 525

Gln Pro Gly Glu Gly Glu Ala Asn Ser Thr Arg Ala Thr Val Pro Phe
    530                 535                 540

Pro Phe Asp Ile Lys Thr Leu Ile Ile Ala Thr Thr Met Gly Phe Ile
545                 550                 555                 560

Ser Phe Leu Gly Val Val Leu Phe Cys Leu Val Leu Leu Phe Leu Trp
                565                 570                 575

Ser Arg Gly Lys Gly Asn Thr Lys His Asn Ile Glu Ile Glu Tyr Val
            580                 585                 590

Pro Arg Lys Ser Asp Ala Gly Ile Ser Ser Ala Asp Ala Pro Arg Lys
        595                 600                 605

Phe Asn Met Lys Met Ile
    610

<210>  52
<211>  1845
<212>  DNA
<213>  Homo sapiens

<400>  52
atgctggcgg ggggcgtgag gagcatgccc agccccctcc tggcctgctg gcagcccatc      60

ctcctgctgg tgctgggctc agtgctgtca ggctcggcca cgggctgccc gccccgctgc     120

gagtgctccg cccaggaccg cgctgtgctg tgccaccgca agcgctttgt ggcagtcccc     180

gagggcatcc ccaccgagac gcgcctgctg gacctaggca agaaccgcat caaaacgctc     240

aaccaggacg agttcgccag cttcccgcac ctggaggagc tggagctcaa cgagaacatc     300

gtgagcgccg tggagcccgg cgccttcaac aacctcttca acctccggac gctgggtctc     360

cgcagcaacc gcctgaagct catcccgcta ggcgtcttca ctggcctcag caacctgacc     420

aagctggaca tcagcgagaa caagattgtt atcctgctgg actacatgtt tcaggacctg     480

tacaacctca agtcactgga ggttggcgac aatgacctcg tctacatctc tcaccgcgcc     540

ttcagcggcc tcaacagcct ggagcagctg acgctggaga aatgcaacct gacctccatc     600

cccaccgagg cgctgtccca cctgcacggc ctcatcgtcc tgaggctccg gcacctcaac     660

atcaatgcca tccgggacta ctccttcaag aggctctacc gactcaaggt cttggagatc     720

tcccactggc cctacttgga caccatgaca cccaactgcc tctacggcct caacctgacg     780

tccctgtcca tcacacactg caatctgacc gctgtgccct acctggccgt ccgccaccta 840

gtctatctcc gcttcctcaa cctctcctac aaccccatca gcaccattga gggctccatg 900

ttgcatgagc tgctccggct gcaggagatc cagctggtgg gcgggcagct ggccgtggtg 960

gagccctatg ccttccgcgg cctcaactac ctgcgcgtgc tcaatgtctc tggcaaccag 1020

ctgaccacac tggaggaatc agtcttccac tcggtgggca acctggagac actcatcctg 1080

gactccaacc cgctggcctg cgactgtcgg ctcctgtggg tgttccggcg ccgctggcgg 1140

ctcaacttca accggcagca gcccacgtgc gccacgcccg agtttgtcca gggcaaggag 1200

ttcaaggact tccctgatgt gctactgccc aactacttca cctgccgccg cgcccgcatc 1260

cgggaccgca aggcccagca ggtgtttgtg gacgagggcc acacggtgca gtttgtgtgc 1320

cgggccgatg gcgacccgcc gcccgccatc ctctggctct caccccgaaa gcacctggtc 1380

tcagccaaga gcaatgggcg gctcacagtc ttccctgatg gcacgctgga ggtgcgctac 1440

gcccaggtac aggacaacgg cacgtacctg tgcatcgcgg ccaacgcggg cggcaacgac 1500

tccatgcccg cccacctgca tgtgcgcagc tactcgcccg actggcccca tcagcccaac 1560

aagaccttcg ctttcatctc caaccagccg ggcgagggag aggccaacag cacccgcgcc 1620

actgtgcctt tccccttcga catcaagacc ctcatcatcg ccaccaccat gggcttcatc 1680

tctttcctgg gcgtcgtcct cttctgcctg gtgctgctgt ttctctggag ccggggcaag 1740

ggcaacacaa agcacaacat cgagatcgag tatgtgcccc gaaagtcgga cgcaggcatc 1800

agctccgccg acgcgccccg caagttcaac atgaagatga tatga 1845

<210> 53
<211> 119
<212> PRT
<213> Artificial

<220>
<223> VH sequence of Li62 variant B06 antibody

<400> 53

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Tyr Tyr Asp Trp Tyr Phe Asp Gln Trp Gly Arg Gly
            100                 105                 110

Thr Leu Val Thr Val Ser Ser
        115

<210>  54
<211>  119
<212>  PRT
<213>  Artificial

<220>
<223>  VH sequence of Li62 variant B12 antibody

<400>  54

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Gln Tyr Asp Trp Tyr Phe Asp Val Trp Gly Arg Gly
            100                 105                 110

Thr Leu Val Thr Val Ser Ser
        115

<210>  55
<211>  119
<212>  PRT
<213>  Artificial

<220>
<223>  VH sequence of Li62 variant F06 antibody

<400>  55

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Asp Tyr Asp Trp Tyr Phe Asp Leu Trp Gly Arg Gly
            100                 105                 110

Thr Leu Val Thr Val Ser Ser
        115


<210>  56
<211>  119
<212>  PRT
<213>  Artificial

<220>
<223>  VH sequence of Li62 variant B01 antibody

<400>  56

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Gln Tyr Asp Trp Tyr Phe Glu Leu Trp Gly Arg Gly
            100                 105                 110

Thr Leu Val Thr Val Ser Ser
        115

<210>  57
<211>  119
<212>  PRT
<213>  Artificial

<220>
<223>  VH sequence of Li62 variant D09 antibody

<400>  57

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Ala Asp Ile Asp Trp Phe Phe Asp Leu Trp Gly Arg Gly
            100                 105                 110

Thr Leu Val Thr Val Ser Ser
        115

<210>  58
<211>  119
<212>  PRT
<213>  Artificial

<220>
<223>  VH sequence of Li62 variant D12 antibody

<400>  58

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Tyr Asp Trp Tyr Phe Asp Leu Trp Gly Arg Gly
            100                 105                 110

Thr Leu Val Thr Val Ser Ser
        115


<210>  59
<211>  119
<212>  PRT
<213>  Artificial

<220>
<223>  VH sequence of Li62 variant F01 antibody

<400>  59

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Arg Tyr Asp Trp Tyr Phe Asp Pro Trp Gly Arg Gly
            100                 105                 110

Thr Leu Val Thr Val Ser Ser
        115

<210>  60
<211>  119
<212>  PRT
<213>  Artificial

<220>
<223>  VH sequence of Li62 variant F02 antibody

<400>  60

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Asp Tyr Asp Trp Tyr Phe Gly Leu Trp Gly Arg Gly
            100                 105                 110

Thr Leu Val Thr Val Ser Ser
        115

<210>  61
<211>  119
<212>  PRT
<213>  Artificial

<220>
<223>  VH sequence of Li62 variant F06 antibody

<400>  61

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Arg Tyr Asp Trp Tyr Phe Asp Leu Trp Gly Arg Gly
            100                 105                 110

Thr Leu Val Thr Val Ser Ser
        115


<210>  62
<211>  119
<212>  PRT
<213>  Artificial

<220>
<223>  VH sequence of Li62 variant F10 antibody

<400>  62

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Ser His Ile Asp Arg Tyr Phe Asp Leu Trp Gly Arg Gly
            100                 105                 110

Thr Leu Val Thr Val Ser Ser
        115

<210>  63
<211>  119
<212>  PRT
<213>  Artificial

<220>
<223>  VH sequence of Li62 variant G08 antibody

<400>  63

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Gln Tyr Asp Trp Tyr Phe Asp Val Trp Gly Arg Gly
            100                 105                 110

Thr Leu Val Thr Val Ser Ser
        115

<210>  64
<211>  119
<212>  PRT
<213>  Artificial

<220>
<223>  VH sequence of Li62 variant H08 antibody

<400>  64

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Tyr Asn Gly Tyr Phe Asp Leu Trp Gly Arg Gly
            100                 105                 110

Thr Leu Val Thr Val Ser Ser
        115


<210>  65
<211>  119
<212>  PRT
<213>  Artificial

<220>
<223>  VH sequence of Li62 variant C10 antibody

<400>  65

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Tyr Tyr Asp Trp Tyr Phe Asp Leu Trp Gly Arg Gly
            100                 105                 110

Thr Leu Val Thr Val Ser Ser
        115

<210>  66
<211>  119
<212>  PRT
<213>  Artificial

<220>
<223>  VH sequence of Li62 variant C02 antibody

<400>  66

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  67
<211>  119
<212>  PRT
<213>  Artificial

<220>
<223>  VH sequence of Li62 variant D05 antibody

<400>  67

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Tyr Tyr Asp Trp Tyr Phe Glu Leu Trp Gly Arg Gly
            100                 105                 110

Thr Leu Val Thr Val Ser Ser
        115


<210>  68
<211>  119
<212>  PRT
<213>  Artificial

<220>
<223>  VH sequence of Li62 variant F02 antibody

<400>  68

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Leu Ile Asp Trp Phe Phe Asp Gln Trp Gly Arg Gly
            100                 105                 110

Thr Leu Val Thr Val Ser Ser
        115

<210>  69
<211>  119
<212>  PRT
<213>  Artificial

<220>
<223>  VH sequence of Li62 variant C10 antibody

<400>  69

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Gln Phe Asp Trp Tyr Phe Asp Leu Trp Gly Arg Gly
            100                 105                 110

Thr Leu Val Thr Val Ser Ser
        115

<210>  70
<211>  119
<212>  PRT
<213>  Artificial

<220>
<223>  VH sequence of Li62 variant H08 antibody

<400>  70

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Phe Asp Leu Trp Gly Arg Gly
            100                 105                 110

Thr Leu Val Thr Val Ser Ser
        115


<210>  71
<211>  118
<212>  PRT
<213>  Artificial

<220>
<223>  VH sequence of Li81 variant F09 antibody

<400>  71

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Glu Asn Asp Ala Phe Asp Val Trp Gly Gln Gly Thr
            100                 105                 110

Thr Val Thr Val Ser Ser
        115

<210>  72
<211>  118
<212>  PRT
<213>  Artificial

<220>
<223>  VH sequence of Li81 variant G02 antibody

<400>  72

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Tyr Asp Thr Trp Gly Gln Gly Thr
            100                 105                 110

Thr Val Thr Val Ser Ser
        115

<210>  73
<211>  118
<212>  PRT
<213>  Artificial

<220>
<223>  VH sequence of Li81 variant H03 antibody

<400>  73

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Thr Asn Asp Ala Phe Asp Ile Trp Gly Gln Gly Thr
            100                 105                 110

Thr Val Thr Val Ser Ser
        115


<210>  74
<211>  118
<212>  PRT
<213>  Artificial

<220>
<223>  VH sequence of Li81 variant A12 antibody

<400>  74

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ser Trp Gly Gln Gly Thr
            100                 105                 110

Thr Val Thr Val Ser Ser
        115

<210>  75
<211>  118
<212>  PRT
<213>  Artificial

<220>
<223>  VH sequence of Li81 variant C02 antibody

<400>  75

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Thr Trp Gly Gln Gly Thr
            100                 105                 110

Thr Val Thr Val Ser Ser
        115

<210>  76
<211>  118
<212>  PRT
<213>  Artificial

<220>
<223>  VH sequence of Li81 variant C11 antibody

<400>  76

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Tyr Asp Arg Trp Gly Gln Gly Thr
            100                 105                 110

Thr Val Thr Val Ser Ser
        115


<210>  77
<211>  118
<212>  PRT
<213>  Artificial

<220>
<223>  VH sequence of Li81 variant D11 antibody

<400>  77

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Val Phe Asp Ser Trp Gly Gln Gly Thr
            100                 105                 110

Thr Val Thr Val Ser Ser
        115

<210>  78
<211>  118
<212>  PRT
<213>  Artificial

<220>
<223>  VH sequence of Li81 variant E05 antibody

<400>  78

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asp Asp Val Phe Asp Met Trp Gly Gln Gly Thr
            100                 105                 110

Thr Val Thr Val Ser Ser
        115

<210>  79
<211>  118
<212>  PRT
<213>  Artificial

<220>
<223>  VH sequence of Li81 variant H04 antibody

<400>  79

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Tyr Asn Asp Ala Phe Asp Phe Trp Gly Gln Gly Thr
            100                 105                 110

Thr Val Thr Val Ser Ser
        115


<210>  80
<211>  118
<212>  PRT
<213>  Artificial

<220>
<223>  VH sequence of Li81 variant B04 antibody

<400>  80

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asp Asp Ala Tyr Asp Met Trp Gly Gln Gly Thr
            100                 105                 110

Thr Val Thr Val Ser Ser
        115

<210>  81
<211>  118
<212>  PRT
<213>  Artificial

<220>
<223>  VH sequence of Li81 variant A02 antibody

<400>  81

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gln Asp Tyr Asp Thr Tyr Asp Leu Trp Gly Gln Gly Thr
            100                 105                 110

Thr Val Thr Val Ser Ser
        115

<210>  82
<211>  118
<212>  PRT
<213>  Artificial

<220>
<223>  VH sequence of Li81 variant B12 antibody

<400>  82

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asp Asp Ala Phe Asp Thr Trp Gly Gln Gly Thr
            100                 105                 110

Thr Val Thr Val Ser Ser
        115


<210>  83
<211>  118
<212>  PRT
<213>  Artificial

<220>
<223>  VH sequence of Li81 variant H06 antibody

<400>  83

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Ala Asp Asp Asp Ala Phe Asp Ile Trp Gly Gln Gly Thr
            100                 105                 110

Thr Val Thr Val Ser Ser
        115

<210>  84
<211>  118
<212>  PRT
<213>  Artificial

<220>
<223>  VH sequence of Li81 variant H08 antibody

<400>  84

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Glu Asn Asp Ala Phe Asp Met Trp Gly Gln Gly Thr
            100                 105                 110

Thr Val Thr Val Ser Ser
        115

<210>  85
<211>  118
<212>  PRT
<213>  Artificial

<220>
<223>  VH sequence of Li81 variant E07 antibody

<400>  85

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Glu Tyr Asp Thr Tyr Asp Ile Trp Gly Gln Gly Thr
            100                 105                 110

Thr Val Thr Val Ser Ser
        115


<210>  86
<211>  447
<212>  PRT
<213>  Artificial

<220>
<223>  Li81 heavy chain

<400>  86

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445

<210>  87
<211>  6
<212>  PRT
<213>  Artificial

<220>
<223>  N-terminus of LINGO-1 polypeptide

<400>  87

Met Gln Val Ser Lys Arg
1               5

<210>  88
<211>  5
<212>  PRT
<213>  Artificial

<220>
<223>  LINGO-1 polypeptide

<220>
<221>  variant
<222>  (3)..(5)
<223>  can be arginine, histidine, glutamine, asparagine

<400>  88

Ile Thr Lys Lys Lys
1               5


<210>  89
<211>  5
<212>  PRT
<213>  Artificial

<220>
<223>  LINGO-1 polypeptide


<220>
<221>  variant
<222>  (3)..(5)
<223>  can be arginine, histidine, glutamine, asparagine

<400>  89

Ala Cys Lys Lys Lys
1               5


<210>  90
<211>  5
<212>  PRT
<213>  Artificial

<220>
<223>  LINGO-1 polypeptide


<220>
<221>  variant
<222>  (3)..(5)
<223>  can be arginine, histidine, glutamine, asparagine

<400>  90

Val Cys Lys Lys Lys
1               5


<210>  91
<211>  5
<212>  PRT
<213>  Artificial

<220>
<223>  LINGO-1 polypeptide


<220>
<221>  Variant
<222>  (3)..(5)
<223>  can be arginine, histidine, glutamine, asparagine

<400>  91

Ser Pro Lys Lys Lys
1               5

<210> 92
<211> 5
<212> PRT
<213> Artificial

<220>
<223> LINGO-1 polypeptide

<400> 92

Ser Pro Arg Lys His
1               5

<210> 93
<211> 5
<212> PRT
<213> Artificial

<220>
<223> LINGO-1 polypeptide

<400> 93

Ser Pro Arg Lys Lys
1               5

<210> 94
<211> 5
<212> PRT
<213> Artificial

<220>
<223> LINGO-1 polypeptide

<400> 94

Ser Pro Arg Lys Arg
1               5

<210> 95
<211> 5
<212> PRT
<213> Artificial

<220>
<223> LINGO-1 polypeptide

<400> 95

Ser Pro Lys Lys His
1               5

<210> 96
<211> 5
<212> PRT
<213> Artificial

<220>

<223>  LINGO-1 polypeptide

<400>  96

Ser Pro His Lys His
1               5

<210>  97
<211>  5
<212>  PRT
<213>  Artificial

<220>
<223>  LINGO-1 polypeptide

<400>  97

Ser Pro Arg Arg His
1               5

<210>  98
<211>  5
<212>  PRT
<213>  Artificial

<220>
<223>  LINGO-1 polypeptide

<400>  98

Ser Pro Arg His His
1               5

<210>  99
<211>  5
<212>  PRT
<213>  Artificial

<220>
<223>  LINGO-1 polypeptide

<400>  99

Ser Pro Arg Arg Arg
1               5

<210>  100
<211>  5
<212>  PRT
<213>  Artificial

<220>
<223>  LINGO-1 polypeptide

<400>  100

Ser Pro His His His
1               5

<210>  101
<211>  5
<212>  PRT
<213>  Artificial

<220>
<223>  LINGO-1 polypeptide

<400>  101

Ser Pro Lys Lys Lys
1               5


<210>  102
<211>  6
<212>  PRT
<213>  Artificial

<220>
<223>  LINGO-1 polypeptide

<400>  102

Leu Ser Pro Arg Lys His
1               5


<210>  103
<211>  6
<212>  PRT
<213>  Artificial

<220>
<223>  LINGO-1 polypeptide

<400>  103

Leu Ser Pro Arg Lys Lys
1               5


<210>  104
<211>  6
<212>  PRT
<213>  Artificial

<220>
<223>  LINGO-1 polypeptide

<400>  104

Leu Ser Pro Arg Lys Arg
1               5


<210>  105
<211>  6
<212>  PRT
<213>  Artificial

<220>

<223>  LINGO-1 polypeptide

<400>  105

Leu Ser Pro Lys Lys His
1               5


<210>  106
<211>  6
<212>  PRT
<213>  Artificial

<220>
<223>  LINGO-1 polypeptide

<400>  106

Leu Ser Pro His Lys His
1               5


<210>  107
<211>  6
<212>  PRT
<213>  Artificial

<220>
<223>  LINGO-1 polypeptide

<400>  107

Leu Ser Pro Arg Arg His
1               5


<210>  108
<211>  6
<212>  PRT
<213>  Artificial

<220>
<223>  LINGO-1 polypeptide

<400>  108

Leu Ser Pro Arg His His
1               5


<210>  109
<211>  6
<212>  PRT
<213>  Artificial

<220>
<223>  LINGO-1 polypeptide

<400>  109

Leu Ser Pro Arg Arg Arg
1               5

<210>  110
<211>  6
<212>  PRT
<213>  Artificial

<220>
<223>  LINGO-1 polypeptide

<400>  110

Leu Ser Pro His His His
1               5


<210>  111
<211>  6
<212>  PRT
<213>  Artificial

<220>
<223>  LINGO-1 polypeptide

<400>  111

Leu Ser Pro Lys Lys Lys
1               5


<210>  112
<211>  7
<212>  PRT
<213>  Artificial

<220>
<223>  LINGO-1 polypeptide

<400>  112

Trp Leu Ser Pro Arg Lys His
1               5


<210>  113
<211>  7
<212>  PRT
<213>  Artificial

<220>
<223>  LINGO-1 polypeptide

<400>  113

Trp Leu Ser Pro Arg Lys Lys
1               5


<210>  114
<211>  7
<212>  PRT
<213>  Artificial

<220>

<223>  LINGO-1 polypeptide

<400>  114

Trp Leu Ser Pro Arg Lys Arg
1               5


<210>  115
<211>  7
<212>  PRT
<213>  Artificial

<220>
<223>  LINGO-1 polypeptide

<400>  115

Trp Leu Ser Pro Lys Lys His
1               5


<210>  116
<211>  7
<212>  PRT
<213>  Artificial

<220>
<223>  LINGO-1 polypeptide

<400>  116

Trp Leu Ser Pro His Lys His
1               5


<210>  117
<211>  7
<212>  PRT
<213>  Artificial

<220>
<223>  LINGO-1 polypeptide

<400>  117

Trp Leu Ser Pro Arg Arg His
1               5


<210>  118
<211>  7
<212>  PRT
<213>  Artificial

<220>
<223>  LINGO-1 polypeptide

<400>  118

Trp Leu Ser Pro Arg His His
1               5

<210>  119
<211>  7
<212>  PRT
<213>  Artificial

<220>
<223>  LINGO-1 polypeptide

<400>  119

Trp Leu Ser Pro Arg Arg Arg
1               5


<210>  120
<211>  7
<212>  PRT
<213>  Artificial

<220>
<223>  LINGO-1 polypeptide

<400>  120

Trp Leu Ser Pro His His His
1               5


<210>  121
<211>  7
<212>  PRT
<213>  Artificial

<220>
<223>  LINGO-1 polypeptide

<400>  121

Trp Leu Ser Pro Lys Lys Lys
1               5


<210>  122
<211>  6
<212>  PRT
<213>  Artificial

<220>
<223>  LINGO-1 polypeptide

<400>  122

Ile Thr Pro Lys Arg Arg
1               5


<210>  123
<211>  5
<212>  PRT
<213>  Artificial

<220>

<223>  LINGO-1 polypeptide

<400>  123

Ala Cys His His Lys
1               5


<210>  124
<211>  5
<212>  PRT
<213>  Artificial

<220>
<223>  LINGO-1 polypeptide

<400>  124

Val Cys His His Lys
1               5


<210>  125
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  125

Xaa Xaa Arg Lys His
1               5


<210>  126
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  126

Xaa Xaa Arg Arg Arg
1               5


<210>  127

<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  127

Xaa Xaa Lys Lys Lys
1               5


<210>  128
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  128

Xaa Xaa His His His
1               5


<210>  129
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  129

Xaa Xaa Arg Lys Lys
1               5


<210>  130
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  130

Xaa Xaa Arg Lys Arg
1               5


<210>  131
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  131

Xaa Xaa Lys Lys His
1               5


<210>  132
<211>  5
<212>  PRT
<213>  Artificial

<220>
<223>  LINGO-1 polypeptide


<220>
<221>  variant
<222>  (1)..(2)
<223>  Xaa can be any naturally occurring amino acid

<400>  132

Xaa Xaa His Lys His
1               5


<210>  133
<211>  5
<212>  PRT
<213>  Artificial

<220>
<223>  LINGO-1 polypeptide

<220>
<221> variant
<222> (1)..(2)
<223> Xaa can be any naturally occurring amino acid

<400> 133

Xaa Xaa Arg Arg His
1               5


<210> 134
<211> 5
<212> PRT
<213> Artificial

<220>
<223> LINGO-1 polypeptide


<220>
<221> variant
<222> (1)..(2)
<223> Xaa can be any naturally occurring amino acid

<400> 134

Xaa Xaa Arg His His
1               5


<210> 135
<211> 5
<212> PRT
<213> Artificial

<220>
<223> LINGO-1 polypeptide


<220>
<221> variant
<222> (3)..(3)
<223> can be arginine, histidine, glutamine, asparagine

<220>
<221> variant
<222> (4)..(4)
<223> Xaa can be any naturally occurring amino acid

<220>
<221> variant
<222> (5)..(5)
<223> can arginine, histidine, glutamine, asparagine

<400> 135

Ile Thr Lys Xaa Lys
1               5

<210>  136
<211>  5
<212>  PRT
<213>  Artificial

<220>
<223>  LINGO-1 polypeptide


<220>
<221>  Variant
<222>  (3)..(3)
<223>  can be arginine, histidine, glutamine, asparagine

<220>
<221>  Variant
<222>  (4)..(4)
<223>  Xaa can be any naturally occurring amino acid

<220>
<221>  Variant
<222>  (5)..(5)
<223>  can be arginine, histidine, glutamine, asparagine

<400>  136

Ala Cys Lys Xaa Lys
1               5


<210>  137
<211>  5
<212>  PRT
<213>  Artificial

<220>
<223>  LINGO-1 polypeptide


<220>
<221>  Variant
<222>  (3)..(3)
<223>  can be arginine, histidine, glutamine, asparagine

<220>
<221>  Variant
<222>  (4)..(4)
<223>  Xaa can be any naturally occurring amino acid

<220>
<221>  Variant
<222>  (5)..(5)
<223>  can be arginine, histidine, glutamine, asparagine

<400>  137

Val Cys Lys Xaa Lys
1               5


<210>  138
<211>  5
<212>  PRT

<213>  Artificial

<220>
<223>  LINGO-1 polypeptide


<220>
<221>  Variant
<222>  (3)..(3)
<223>  can be arginine, histidine, glutamine, asparagine

<220>
<221>  Variant
<222>  (4)..(4)
<223>  Xaa can be any naturally occurring amino acid

<220>
<221>  Variant
<222>  (5)..(5)
<223>  can be arginine, histidine, glutamine, asparagine

<400>  138

Ser Pro Lys Xaa Lys
1               5


<210>  139
<211>  5
<212>  PRT
<213>  Artificial

<220>
<223>  LINGO-1 polypeptide

<400>  139

Ser Pro Arg Leu His
1               5


<210>  140
<211>  9
<212>  PRT
<213>  Artificial

<220>
<223>  LINGO-1 polypeptide

<400>  140

Arg Arg Ala Arg Ile Arg Asp Arg Lys
1               5


<210>  141
<211>  9
<212>  PRT
<213>  Artificial

<220>
<223>  LINGO-1 polypeptide

<400>  141

Lys Lys Val Lys Val Lys Glu Lys Arg
1               5


<210>  142
<211>  9
<212>  PRT
<213>  Artificial

<220>
<223>  LINGO-1 polypeptide

<400>  142

Arg Arg Leu Arg Leu Arg Asp Arg Lys
1               5


<210>  143
<211>  9
<212>  PRT
<213>  Artificial

<220>
<223>  LINGO-1 polypeptide

<400>  143

Arg Arg Gly Arg Gly Arg Asp Arg Lys
1               5


<210>  144
<211>  9
<212>  PRT
<213>  Artificial

<220>
<223>  LINGO-1 polypeptide

<400>  144

Arg Arg Ile Arg Ala Arg Asp Arg Lys
1               5


<210>  145
<211>  214
<212>  PRT
<213>  Artificial

<220>
<223>  Li33 Fab' light chain

<400>  145

Asp Ile Gln Met Thr Gln Ser Pro Gly Thr Leu Ser Leu Ser Pro Gly
1               5                   10                  15


Glu Arg Ala Thr Leu Ser Cys Arg Ala Ser Gln Ser Val Ser Ser Tyr

                20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Trp Pro Leu
                85                  90                  95

Thr Phe Gly Gly Gly Thr Lys Val Glu Ile Lys Arg Thr Val Ala Ala
                100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
        195                 200                 205

Phe Asn Arg Gly Glu Cys
    210


<210>  146
<211>  230
<212>  PRT
<213>  Artificial

<220>
<223>  Li33 Fab' heavy chain

<400>  146

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  147
<211>  230
<212>  PRT
<213>  Artificial

<220>
<223>  Li33 Heavy Chain Variant

<400>  147

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser His Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  148
<211>  230
<212>  PRT
<213>  Artificial

<220>
<223>  Li33 Heavy Chain Variant

<400>  148

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Phe Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  149
<211>  230
<212>  PRT
<213>  Artificial

<220>
<223>  Li33 Heavy Chain Variant

<400>  149

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Leu Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  150
<211>  230
<212>  PRT
<213>  Artificial

<220>
<223>  Li33 Heavy Chain Variant

<400>  150

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Met Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp

145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  151
<211>  230
<212>  PRT
<213>  Artificial

<220>
<223>  Li33 Heavy Chain Variant

<400>  151

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Leu Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230


<210>  152
<211>  230
<212>  PRT
<213>  Artificial

<220>
<223>  Li33 Heavy Chain Variant

<400>  152

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Ser Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 153
<211> 230
<212> PRT
<213> Artificial

<220>
<223> Li33 Heavy Chain Variant

<400> 153

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Thr Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  154
<211>  230
<212>  PRT
<213>  Artificial

<220>
<223>  Li33 Heavy Chain Variant

<400>  154

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val

35                  40                  45

Ser Trp Ile Gly Trp Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  155
<211>  230
<212>  PRT
<213>  Artificial

<220>
<223>  Li33 Heavy Chain Variant

<400>  155

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Val Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  156
<211>  230
<212>  PRT
<213>  Artificial

<220>
<223>  Li33 Heavy Chain Variant

<400>  156

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp His Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  157
<211>  230
<212>  PRT
<213>  Artificial

<220>
<223>  Li33 Heavy Chain Variant

<400>  157

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Ser Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  158
<211>  230
<212>  PRT
<213>  Artificial

<220>
<223>  Li33 Heavy Chain Variant

<400>  158

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Gln Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 159
<211> 230
<212> PRT
<213> Artificial

<220>
<223> Li33 Heavy Chain Variant

<400> 159

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Gly Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp

145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  160
<211>  230
<212>  PRT
<213>  Artificial

<220>
<223>  Li33 Heavy Chain Variant

<400>  160

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Met Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  161
<211>  230
<212>  PRT
<213>  Artificial

<220>
<223>  Li33 Heavy Chain Variant

<400>  161

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Asn Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230


<210>  162
<211>  230
<212>  PRT
<213>  Artificial

<220>
<223>  Li33 Heavy Chain Variant

<400>  162

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly His Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  163
<211>  230
<212>  PRT
<213>  Artificial

<220>
<223>  Li33 Heavy Chain Variant

<400>  163

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val

35                  40                  45

Ser Trp Ile Gly Pro Ser Gly Gly Leu Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  164
<211>  230
<212>  PRT
<213>  Artificial

<220>
<223>  Li33 Heavy Chain Variant

<400>  164

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Phe Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 165
<211> 214
<212> PRT
<213> Artificial

<220>
<223>  Li33 Light Chain Variant

<400>  165

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Ala Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  166
<211>  214

<212>  PRT
<213>  Artificial

<220>
<223>  Li33 Light Chain Variant

<400>  166

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Asp Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  167
<211>  214
<212>  PRT
<213>  Artificial

<220>
<223>  Li33 Light Chain Variant

<400>  167

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Leu Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  168
<211>  214
<212>  PRT
<213>  Artificial

<220>
<223>  Li33 Light Chain Variant

<400>  168

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Asn Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser

195                 200                 205

Phe Asn Arg Gly Glu Cys
    210

<210>  169
<211>  214
<212>  PRT
<213>  Artificial

<220>
<223>  Li33 Light Chain Variant

<400>  169

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Gly Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  170
<211>  214
<212>  PRT
<213>  Artificial

<220>
<223>  Li33 Light Chain Variant

<400>  170

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Gln Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 171
<211> 214
<212> PRT
<213> Artificial

<220>
<223> Li33 Light Chain Variant

<400> 171

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Val Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  172
<211>  214
<212>  PRT
<213>  Artificial

<220>
<223>  Li33 Light Chain Variant

<400>  172

Asp Ile Gln Met Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asp Lys Ser Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln

145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  173
<211>  230
<212>  PRT
<213>  Artificial

<220>
<223>  Li33 Heavy Chain Variant

<400>  173

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Gly Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  174
<211>  230
<212>  PRT
<213>  Artificial

<220>
<223>  Li33 Heavy Chain Variant

<400>  174

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Ile Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  175
<211>  230
<212>  PRT
<213>  Artificial

<220>
<223>  Li33 Heavy Chain Variant

<400>  175

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Asp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  176
<211>  230
<212>  PRT
<213>  Artificial

<220>
<223>  Li33 Heavy Chain Variant

<400>  176

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Met Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  177
<211>  230
<212>  PRT
<213>  Artificial

<220>
<223>  Li33 Heavy Chain Variant

<400>  177

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Leu Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 178
<211> 230
<212> PRT
<213> Artificial

<220>
<223> Li33 Heavy Chain Variant

<400> 178

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Thr Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  179
<211>  230

<212>  PRT
<213>  Artificial

<220>
<223>  Li33 Heavy Chain Variant

<400>  179

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Ile Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  180
<211>  230
<212>  PRT
<213>  Artificial

<220>
<223>  Li33 Heavy Chain Variant

<400>  180

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Gly Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  181
<211>  230
<212>  PRT
<213>  Artificial

<220>
<223>  Li33 Heavy Chain Variant

<400>  181

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Trp Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu

165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  182
<211>  230
<212>  PRT
<213>  Artificial

<220>
<223>  Li33 Heavy Chain Variant

<400>  182

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Tyr Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  183
<211>  230
<212>  PRT
<213>  Artificial

<220>
<223>  Li33 Heavy Chain Variant

<400>  183

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ser Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 184
<211> 230
<212> PRT
<213> Artificial

<220>
<223> Li33 Heavy Chain Variant

<400> 184

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Pro Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  185
<211>  230
<212>  PRT
<213>  Artificial

<220>
<223>  Li33 Heavy Chain Variant

<400>  185

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Val Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  186
<211>  230
<212>  PRT
<213>  Artificial

<220>
<223>  Li33 Heavy Chain Variant

<400>  186

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Thr Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210> 187
<211> 230
<212> PRT
<213> Artificial

<220>
<223> Li33 Heavy Chain Variant

<400> 187

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Gln Tyr Phe Asp Leu Trp Gly Arg Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro
225                 230

<210>  188
<211>  119

<212>  PRT
<213>  Artificial

<220>
<223>  Li62 VH Variant

<400>  188

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser His Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  189
<211>  119
<212>  PRT
<213>  Artificial

<220>
<223>  Li62 VH Variant

<400>  189

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Phe Ile Gly Pro Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  190
<211>  119
<212>  PRT
<213>  Artificial

<220>
<223>  Li62 VH Variant

<400>  190

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Leu Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  191
<211>  119

<212>  PRT
<213>  Artificial

<220>
<223>  Li62 VH Variant

<400>  191

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Met Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  192
<211>  119
<212>  PRT
<213>  Artificial

<220>
<223>  Li62 VH Variant

<400>  192

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Leu Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  193
<211>  119
<212>  PRT
<213>  Artificial

<220>
<223>  Li62 VH Variant

<400>  193

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Ser Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  194
<211>  119

<212>  PRT
<213>  Artificial

<220>
<223>  Li62 VH Variant

<400>  194

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Thr Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  195
<211>  119
<212>  PRT
<213>  Artificial

<220>
<223>  Li62 VH Variant

<400>  195

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Trp Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  196
<211>  119
<212>  PRT
<213>  Artificial

<220>
<223>  Li62 VH Variant

<400>  196

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Val Tyr Phe Asp Leu Trp Gly Arg Gly
            100                 105                 110

Thr Leu Val Thr Val Ser Ser
        115

<210>  197
<211>  119

<212>   PRT
<213>   Artificial

<220>
<223>   Li62 VH Variant

<400>   197

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp His Tyr Phe Asp Leu Trp Gly Arg Gly
            100                 105                 110

Thr Leu Val Thr Val Ser Ser
        115


<210>   198
<211>   119
<212>   PRT
<213>   Artificial

<220>
<223>   Li62 VH Variant

<400>   198

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Ser Tyr Phe Asp Leu Trp Gly Arg Gly
            100                 105                 110

Thr Leu Val Thr Val Ser Ser
        115

<210>  199
<211>  119
<212>  PRT
<213>  Artificial

<220>
<223>  Li62 VH Variant

<400>  199

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Gln Tyr Phe Asp Leu Trp Gly Arg Gly
            100                 105                 110

Thr Leu Val Thr Val Ser Ser
        115

<210>  200
<211>  119

<212>  PRT
<213>  Artificial

<220>
<223>  Li62 VH Variant

<400>  200

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Gly Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  201
<211>  119
<212>  PRT
<213>  Artificial

<220>
<223>  Li62 VH Variant

<400>  201

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Met Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  202
<211>  119
<212>  PRT
<213>  Artificial

<220>
<223>  Li62 VH Variant

<400>  202

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Asn Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  203
<211>  119

<212>  PRT
<213>  Artificial

<220>
<223>  Li62 VH Variant

<400>  203

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly His Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  204
<211>  119
<212>  PRT
<213>  Artificial

<220>
<223>  Li62 VH Variant

<400>  204

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Leu Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  205
<211>  119
<212>  PRT
<213>  Artificial

<220>
<223>  Li62 VH Variant

<400>  205

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Phe Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  206
<211>  119

<212>  PRT
<213>  Artificial

<220>
<223>  Li62 VH Variant

<400>  206

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Gly Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  207
<211>  119
<212>  PRT
<213>  Artificial

<220>
<223>  Li62 VH Variant

<400>  207

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Ile Ile Gly Pro Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  208
<211>  119
<212>  PRT
<213>  Artificial

<220>
<223>  Li62 VH Variant

<400>  208

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Asp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  209
<211>  119

<212>   PRT
<213>   Artificial

<220>
<223>   Li62 VH Variant

<400>   209

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Met Tyr Phe Asp Leu Trp Gly Arg Gly
            100                 105                 110

Thr Leu Val Thr Val Ser Ser
        115


<210>   210
<211>   119
<212>   PRT
<213>   Artificial

<220>
<223>   Li62 VH Variant

<400>   210

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Leu Tyr Phe Asp Leu Trp Gly Arg Gly
            100                 105                 110

Thr Leu Val Thr Val Ser Ser
        115

<210>  211
<211>  119
<212>  PRT
<213>  Artificial

<220>
<223>  Li62 VH Variant

<400>  211

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Thr Tyr Phe Asp Leu Trp Gly Arg Gly
            100                 105                 110

Thr Leu Val Thr Val Ser Ser
        115

<210>  212
<211>  119

<212>  PRT
<213>  Artificial

<220>
<223>  Li62 VH Variant

<400>  212

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Ile Tyr Phe Asp Leu Trp Gly Arg Gly
            100                 105                 110

Thr Leu Val Thr Val Ser Ser
        115


<210>  213
<211>  119
<212>  PRT
<213>  Artificial

<220>
<223>  Li62 VH Variant

<400>  213

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Gly Ser Gly Gly Ile Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  214
<211>  119
<212>  PRT
<213>  Artificial

<220>
<223>  Li62 VH Variant

<400>  214

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Trp Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  215
<211>  119

<212>  PRT
<213>  Artificial

<220>
<223>  Li62 VH Variant

<400>  215

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Tyr Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  216
<211>  119
<212>  PRT
<213>  Artificial

<220>
<223>  Li62 VH Variant

<400>  216

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ser Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  217
<211>  119
<212>  PRT
<213>  Artificial

<220>
<223>  Li62 VH Variant

<400>  217

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Pro Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  218
<211>  119

<212>  PRT
<213>  Artificial

<220>
<223>  Li62 VH Variant

<400>  218

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Val Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115


<210>  219
<211>  119
<212>  PRT
<213>  Artificial

<220>
<223>  Li62 VH Variant

<400>  219

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Thr Thr Lys Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Trp Tyr Phe Asp Leu Trp Gly Arg Gly
            100                 105                 110

Thr Leu Val Thr Val Ser Ser
        115

<210>  220
<211>  119
<212>  PRT
<213>  Artificial

<220>
<223>  Li62 VH Variant

<400>  220

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly His Asn Asp Gln Tyr Phe Asp Leu Trp Gly Arg Gly
            100                 105                 110

Thr Leu Val Thr Val Ser Ser
        115

<210>  221
<211>  108

<212>  PRT
<213>  Artificial

<220>
<223>  Li81 VL Variant

<400>  221

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Leu
                85                  90                  95

Tyr Thr Phe Gly Gln Gly Thr Lys Leu Glu Ile Lys
            100                 105


<210>  222
<211>  108
<212>  PRT
<213>  Artificial

<220>
<223>  Li81 VL Variant

<400>  222

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro

65                  70                  75                  80

Glu Asp Phe Ala Val Tyr Tyr Cys Gln Gln Arg Ser Asn Trp Pro Ile
                85                  90                  95

Tyr Thr Phe Gly Gln Gly Thr Lys Leu Glu Ile Lys
            100                 105

<210>  223
<211>  108
<212>  PRT
<213>  Artificial

<220>
<223>  Li81 VL Variant

<400>  223

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Gln
                85                  90                  95

Tyr Thr Phe Gly Gln Gly Thr Lys Leu Glu Ile Lys
            100                 105

<210>  224
<211>  108
<212>  PRT
<213>  Artificial

<220>
<223>  Li81 VL Variant

<400>  224

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Lys
                85                  90                  95

Tyr Thr Phe Gly Gln Gly Thr Lys Leu Glu Ile Lys
            100                 105

<210>  225
<211>  108
<212>  PRT
<213>  Artificial

<220>
<223>  Li81 VL Variant

<400>  225

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Ala
                85                  90                  95

Tyr Thr Phe Gly Gln Gly Thr Lys Leu Glu Ile Lys
            100                 105

<210>  226

<211>  108
<212>  PRT
<213>  Artificial

<220>
<223>  Li81 VL Variant

<400>  226

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Val
                85                  90                  95

Tyr Thr Phe Gly Gln Gly Thr Lys Leu Glu Ile Lys
            100                 105


<210>  227
<211>  108
<212>  PRT
<213>  Artificial

<220>
<223>  Li81 VL Variant

<400>  227

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Tyr
                85                  90                  95

Tyr Thr Phe Gly Gln Gly Thr Lys Leu Glu Ile Lys
            100                 105

<210>  228
<211>  108
<212>  PRT
<213>  Artificial

<220>
<223>  Li81 VL Variant

<400>  228

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Phe
                85                  90                  95

Tyr Thr Phe Gly Gln Gly Thr Lys Leu Glu Ile Lys
            100                 105

<210>  229
<211>  118
<212>  PRT
<213>  Artificial

<220>
<223>  Li81 VH Variant

<400>  229

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Leu Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser
        115

<210>  230
<211>  118
<212>  PRT
<213>  Artificial

<220>
<223>  Li81 VH Variant

<400>  230

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Ser Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser
        115

<210>  231
<211>  118
<212>  PRT
<213>  Artificial

<220>
<223>  Li81 VH Variant

<400>  231

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Thr Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser
        115

<210>  232
<211>  118
<212>  PRT
<213>  Artificial

<220>
<223>  Li81 VH Variant

<400>  232

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Trp Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser
        115

<210>  233
<211>  118
<212>  PRT
<213>  Artificial

<220>
<223>  Li81 VH Variant

<400>  233

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Gly Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser
        115

<210>  234
<211>  108
<212>  PRT
<213>  Artificial

<220>
<223>  Li81 VL Variant

<400>  234

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Ala Pro Met
                85                  90                  95

Tyr Thr Phe Gly Gln Gly Thr Lys Leu Glu Ile Lys
            100                 105

<210>  235
<211>  108
<212>  PRT
<213>  Artificial

<220>
<223>  Li81 VL Variant

<400>  235

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Asp Pro Met
                85                  90                  95

Tyr Thr Phe Gly Gln Gly Thr Lys Leu Glu Ile Lys
            100                 105

<210>  236
<211>  108
<212>  PRT
<213>  Artificial

<220>
<223>  Li81 VL Variant

<400>  236

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Leu Pro Met
                85                  90                  95

Tyr Thr Phe Gly Gln Gly Thr Lys Leu Glu Ile Lys
            100                 105

<210>  237
<211>  108
<212>  PRT
<213>  Artificial

<220>
<223>  Li81 VL Variant

<400>  237

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Asn Pro Met
                85                  90                  95

Tyr Thr Phe Gly Gln Gly Thr Lys Leu Glu Ile Lys
            100                 105


<210>  238
<211>  108
<212>  PRT
<213>  Artificial

<220>
<223>  Li81 VL Variant

<400>  238

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Gly Pro Met
                85                  90                  95

Tyr Thr Phe Gly Gln Gly Thr Lys Leu Glu Ile Lys
            100                 105

<210>  239
<211>  108
<212>  PRT
<213>  Artificial

<220>
<223>  Li81 VL Variant

<400>  239

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Gln Pro Met
                85                  90                  95

Tyr Thr Phe Gly Gln Gly Thr Lys Leu Glu Ile Lys
            100                 105

<210>  240
<211>  108
<212>  PRT
<213>  Artificial

<220>
<223>  Li81 VL Variant

<400>  240

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Val Pro Met
                85                  90                  95

Tyr Thr Phe Gly Gln Gly Thr Lys Leu Glu Ile Lys
            100                 105

<210>  241
<211>  108
<212>  PRT
<213>  Artificial

<220>
<223>  Li81 VL Variant

<400>  241

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Ser Pro Met
                85                  90                  95

Tyr Thr Phe Gly Gln Gly Thr Lys Leu Glu Ile Lys
            100                 105

<210>  242
<211>  119
<212>  PRT

<213>  Artificial

<220>
<223>  Li113 VH Variant

<400>  242

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser His Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  243
<211>  119
<212>  PRT
<213>  Artificial

<220>
<223>  Li113 VH Variant

<400>  243

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Phe Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  244
<211>  119
<212>  PRT
<213>  Artificial

<220>
<223>  Li113 VH Variant

<400>  244

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Leu Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  245
<211>  119
<212>  PRT

<213>  Artificial

<220>
<223>  Li113 VH Variant

<400>  245

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Met Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  246
<211>  119
<212>  PRT
<213>  Artificial

<220>
<223>  Li113 VH Variant

<400>  246

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Leu Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  247
<211>  119
<212>  PRT
<213>  Artificial

<220>
<223>  Li113 VH Variant

<400>  247

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Ser Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  248
<211>  119
<212>  PRT

<213>  Artificial

<220>
<223>  Li113 VH Variant

<400>  248

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Thr Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  249
<211>  119
<212>  PRT
<213>  Artificial

<220>
<223>  Li113 VH Variant

<400>  249

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Trp Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  250
<211>  119
<212>  PRT
<213>  Artificial

<220>
<223>  Li113 VH Variant

<400>  250

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Val Tyr Leu Asp Leu Trp Gly Arg Gly
            100                 105                 110

Thr Leu Val Thr Val Ser Ser
        115

<210>  251
<211>  119
<212>  PRT

<213>  Artificial

<220>
<223>  Li113 VH Variant

<400>  251

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp His Tyr Leu Asp Leu Trp Gly Arg Gly
            100                 105                 110

Thr Leu Val Thr Val Ser Ser
        115


<210>  252
<211>  119
<212>  PRT
<213>  Artificial

<220>
<223>  Li113 VH Variant

<400>  252

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Ser Tyr Leu Asp Leu Trp Gly Arg Gly
            100                 105                 110

Thr Leu Val Thr Val Ser Ser
        115

<210>  253
<211>  119
<212>  PRT
<213>  Artificial

<220>
<223>  Li113 VH Variant

<400>  253

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Gln Tyr Leu Asp Leu Trp Gly Arg Gly
            100                 105                 110

Thr Leu Val Thr Val Ser Ser
        115

<210>  254
<211>  119
<212>  PRT

<213>  Artificial

<220>
<223>  Li113 VH Variant

<400>  254

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Gly Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  255
<211>  119
<212>  PRT
<213>  Artificial

<220>
<223>  Li113 VH Variant

<400>  255

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Met Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  256
<211>  119
<212>  PRT
<213>  Artificial

<220>
<223>  Li113 VH Variant

<400>  256

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Asn Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  257
<211>  119
<212>  PRT

<213>  Artificial

<220>
<223>  Li113 VH Variant

<400>  257

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly His Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  258
<211>  119
<212>  PRT
<213>  Artificial

<220>
<223>  Li113 VH Variant

<400>  258

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Leu Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  259
<211>  119
<212>  PRT
<213>  Artificial

<220>
<223>  Li113 VH Variant

<400>  259

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Phe Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  260
<211>  119
<212>  PRT

<213>  Artificial

<220>
<223>  Li113 VH Variant

<400>  260

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Gly Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  261
<211>  119
<212>  PRT
<213>  Artificial

<220>
<223>  Li113 VH Variant

<400>  261

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Ile Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  262
<211>  119
<212>  PRT
<213>  Artificial

<220>
<223>  Li113 VH Variant

<400>  262

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Asp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  263
<211>  119
<212>  PRT

<213>  Artificial

<220>
<223>  Li113 VH Variant

<400>  263

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Met Tyr Leu Asp Leu Trp Gly Arg Gly
            100                 105                 110

Thr Leu Val Thr Val Ser Ser
        115


<210>  264
<211>  119
<212>  PRT
<213>  Artificial

<220>
<223>  Li113 VH Variant

<400>  264

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Leu Tyr Leu Asp Leu Trp Gly Arg Gly
            100                 105                 110

Thr Leu Val Thr Val Ser Ser
        115

<210>  265
<211>  119
<212>  PRT
<213>  Artificial

<220>
<223>  Li113 VH Variant

<400>  265

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Thr Tyr Leu Asp Leu Trp Gly Arg Gly
            100                 105                 110

Thr Leu Val Thr Val Ser Ser
        115

<210>  266
<211>  119
<212>  PRT

<213>  Artificial

<220>
<223>  Li113 VH Variant

<400>  266

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Ile Tyr Leu Asp Leu Trp Gly Arg Gly
            100                 105                 110

Thr Leu Val Thr Val Ser Ser
        115


<210>  267
<211>  119
<212>  PRT
<213>  Artificial

<220>
<223>  Li113 VH Variant

<400>  267

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Gly Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  268
<211>  119
<212>  PRT
<213>  Artificial

<220>
<223>  Li113 VH Variant

<400>  268

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Trp Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  269
<211>  119
<212>  PRT

<213>  Artificial

<220>
<223>  Li113 VH Variant

<400>  269

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Tyr Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  270
<211>  119
<212>  PRT
<213>  Artificial

<220>
<223>  Li113 VH Variant

<400>  270

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ser Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  271
<211>  119
<212>  PRT
<213>  Artificial

<220>
<223>  Li113 VH Variant

<400>  271

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Pro Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  272
<211>  119
<212>  PRT

<213>  Artificial

<220>
<223>  Li113 VH Variant

<400>  272

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Val Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115


<210>  273
<211>  119
<212>  PRT
<213>  Artificial

<220>
<223>  Li113 VH Variant

<400>  273

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Thr Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Trp Tyr Leu Asp Leu Trp Gly Arg Gly
            100                 105                 110

Thr Leu Val Thr Val Ser Ser
        115

<210>  274
<211>  119
<212>  PRT
<213>  Artificial

<220>
<223>  Li113 VH Variant

<400>  274

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Pro Met Phe Trp Val Arg Gln Ala Pro Gly Lys Gly Leu Glu Trp Val
        35                  40                  45

Ser Trp Ile Gly Pro Ser Gly Gly Ile Thr Lys Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Thr Tyr Tyr Cys
                85                  90                  95

Ala Arg Glu Gly Thr Tyr Asp Gln Tyr Leu Asp Leu Trp Gly Arg Gly
            100                 105                 110

Thr Leu Val Thr Val Ser Ser
        115

<210>  275
<211>  447
<212>  PRT

<213>  Artificial Sequence

<220>
<223>  Li113 VH Variant

<400>  275

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Lys Trp Val Arg Gln Ala Pro Gly Lys Gly Leu Glu Trp Val
        35                  40                  45

Ser Val Ile Gly Pro Ser Gly Gly Phe Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Thr Glu Gly Asp Asn Asp Ala Phe Asp Ile Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Ala Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly
        435                 440                 445

<210>  276
<211>  215
<212>  PRT
<213>  Artificial Sequence

<220>
<223>  Li113 VH Variant

<400>  276

Asp Ile Gln Met Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Met
                85                  90                  95

Tyr Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

## Description

### RELATED APPLICATIONS

This application claims the benefit of U.S. Provisional Application No. 61/711,638, filed October 9, 2012, the contents of which are incorporated herein by reference in their entirety.

### BACKGROUND OF THE INVENTION

Multiple sclerosis (MS) is an inflammatory disease of the brain and spinal cord characterized by recurrent foci of inflammation that lead to destruction of the myelin sheath. In many areas, nerve fibers are also damaged. Inflammatory activity in MS patients tends to be highest in the initial phase of disease.

Emerging data demonstrate that irreversible axonal loss occurs early in the course of MS. Transected axons fail to regenerate in the central nervous system (CNS); and therefore, early treatment aimed at suppressing MS lesion formation is of importance. As early as disease onset, axons are transected in lesions with active inflammation (Trapp et al. (1998) N Engl J Med 338: 278-285; Bjartmar et al. (2001) Curr Opin Neurol 14: 271-278; Ferguson et al. (1997) Brain 120: 393-399). The degree of demyelination is related to the degree of inflammation and the exposure of demyelinated axons to the inflammatory environment, as well as non-inflammatory mediators (Trapp et al. (1998) N Engl J Med 338: 278-285; Kornek et al. (2000) Am J Pathol 157: 267-276; Bitsch et al. (2000) Brain 123: 1174-1183). There is also destruction of oligodendrocytes with impaired remyelination in demyelinating lesions (Peterson et al. (2002) J Neuropathol Exp Neurol 61: 539-546; Chang et al. (2002) N Engl J Med 346: 165-173). The loss of oligodendrocytes leads to a reduction in the capacity to re-myelinate and may result in the loss of trophic factors that support neurons and axons (Bjartmar et al. (1999) J Neurocytol 28: 383-395).

Currently approved therapies for CNS demyelinating diseases, such as multiple sclerosis (MS), are primarily immunomodulatory, and typically do not have direct effects on CNS repair. Although some degree of axonal remyelination by oligodendrocytes takes place early during the course of MS, the ability to repair the CNS eventually fails, leading to irreversible tissue injury and an increase in disease-related disabilities. Thus, there is a need for additional therapies that enhance remyelination and neuroaxonal protection in CNS demyelinating diseases, such as MS.

### SUMMARY OF THE INVENTION

The present invention provides, at least in part, methods and compositions for enhancing one or more of: myelination, re-myelination, oligodendrocyte numbers, or neuroaxonal protection in a subject, *e.g*., a human (*e.g.,* a human MS patient), while ameliorating an inflammatory condition in the subject. In certain embodiments, the methods and compositions described herein include a reparative agent (*e.g.,* a LINGO-1 antagonist) and an immunomodulatory agent, in combination. In other embodiments, the reparative agent (*e.g.,* a LINGO-1 antagonist) can be used to treat an inflammatory condition of the optic nerve, *e.g.,* optic neuritis (*e.g.,* acute optic neuritis (AON). Thus, methods, compositions and kits described herein can be useful for treating a CNS disorder, *e.g.,* a CNS demyelinating disease.

Accordingly, in one aspect, the invention features a method of enhancing one or more of: myelination, re-myelination, oligodendrocyte numbers, or neuroaxonal protection in a subject (*e.g.,* a subject in need thereof). The method includes administering to the subject a reparative agent (*e.g.,* a LINGO-1 antagonist) and an immunomodulatory agent, in an amount sufficient to enhance one or more of: myelination, re-myelination, oligodendrocyte numbers, or neuroaxonal protection.

In a related aspect, the invention features a method of treating a CNS disorder, *e.g.,* a CNS demyelinating disease (*e.g.,* multiple sclerosis), in a subject (*e.g*., a subject in need of treatment). The method includes administering to the subject a reparative agent (*e.g.,* a LINGO-1 antagonist) and an immunomodulatory agent, in an amount sufficient to reduce one or more symptoms associated with the disorder, thereby treating the disorder. In certain embodiments, said treatment includes: reducing one or more symptoms associated with the disease; and/or reducing, retarding or preventing a relapse, or the worsening of a disability, in the subject.

In another aspect, the invention features a kit that includes a reparative agent (*e.g.,* a LINGO-1 antagonist) and an immunomodulatory agent, *e.g.,* an agent as described herein. Optionally, the kit is labeled and/or contains instructions for use in treating a CNS disorder, *e.g.,* a CNS demyelinating disease.

In yet another aspect, the invention features a packaged composition (e.g., a packaged pharmaceutical composition) that includes a reparative agent (*e.g.,* a LINGO-1 antagonist) and/or an immunomodulatory agent, *e.g.,* an agent as described herein. Optionally, the packaged composition is labeled and/or contains instructions for use of the reparative agent and the immunomodulatory agent in combination in treating a CNS disorder, *e.g.,* a CNS demyelinating disease. The LINGO-1 antagonist and/or the immunomodulatory agent can be in a form suitable for any route of administration, *e.g*., peripheral administration (*e.g*., intravenous, subcutaneous, intramuscular, intravitreal, intrathecal, or oral administration). The route of administration can be the same or different depending on the composition used. In one embodiment, the packaged pharmaceutical composition includes a LINGO-1 antagonist (*e.g*., an antibody against LINGO-1) in a form or preparation suitable for intravenous administration. In another embodiment, the packaged pharmaceutical composition includes an immunomodulatory agent (*e.g.,* an interferon) in a form or preparation suitable for intramuscular administration. One or more agents can be included in the packaged pharmaceutical composition.

In yet another aspect, the invention features a method of treating an inflammatory condition of the optic nerve, *e.g.,* optic neuritis (*e.g.,* acute optic neuritis (AON), in a subject (*e.g.,* a subject in need of treatment). The method includes administering to the subject a reparative agent (*e.g.,* a LINGO-1 antagonist), in an amount sufficient to reduce one or more symptoms associated with the condition, thereby treating the disorder. In certain embodiments, said treatment includes: reducing one or more symptoms associated with the condition or disease; and/or reducing, retarding or preventing a relapse, or the worsening of a disability, in the subject.

Additional embodiments, features or improvements of any of the foregoing methods, compositions and kits are as follows:

### CNS disorders and CNS demyelinating diseases

The CNS disorder (*e.g.,* the CNS demyelinating disease) can be any condition, disease, disorder or injury associated with one or more of: demyelination, dysmyelination, axonal injury, loss of axonal area or axial diffusivity, or loss of neuronal synapsis/connectivity, and/or dysfunction or death of an oligodendrocyte or a neuronal cell. In certain embodiments, the CNS disorder affects the nervous system by causing damage to the myelin sheath of axons. In other embodiments, the CNS disorder includes Nogo receptor-1 (NgR1-) mediated inhibition of axonal extension or neurite extension, *e.g.,* in the brain and spinal cord. In other embodiments, the CNS disorder has one or more inflammatory components. Exemplary CNS disorders include, but are not limited to, CNS demyelinating diseases, CNS injury, Amyotrophic lateral sclerosis (ALS), Huntington's disease, Alzheimer's disease, Parkinson's disease, diabetic neuropathy, stroke, idiopathic inflammatory demyelinating disease, multiple sclerosis (MS), optic neuritis (*e.g*., acute optic neuritis), neuromyelitis optica (NMO), leukodystrophies, vitamin B12 deficiency, progressive multifocal leukoencephalopathy (PML), encephalomyelitis (EPL), acute disseminated encephalomyelitis (ADEM), central pontine myelolysis (CPM), Wallerian Degeneration, adrenoleukodystrophy, Alexander's disease, Pelizaeus Merzbacher disease (PMZ), traumatic glaucoma, periventricular leukomalatia (PVL), essential tremor, white matter stroke, or transverse myelitis. A CNS demyelinating disease can be chosen from one or more of the aforesaid disorders. In one embodiment, the CNS demyelinating disease is multiple sclerosis. In other embodiments, the CNS demyelinating disease is optic neuritis, *e.g*., acute optic neuritis.

### Reparative agents

In certain embodiments, the reparative agent causes one or more of: enhances myelination or re- myelination, enhances neuroaxonal protection, increases axonal extension, increases neuronal sprouting, and/or promotes oligodendrocyte numbers (*e.g*., by increasing one or more of: survival or differentiation of oligodendrocytes).

In one embodiment, the reparative agent is an antagonist of LRR and Ig domain-containing, Nogo receptor-interacting protein ("LINGO," *e.g.,* LINGO-1). LINGO-1, previously called Sp35, is a cell surface glycoprotein that is selectively expressed in the adult CNS in neurons and oligodendrocytes, where it is believed to function as a negative regulator of oligodendrocyte differentiation, myelination, and remyelination. Thus, antagonism of LINGO-1 can enhance myelination or re-myelination of axons, *e.g*., by oligodendrocytes, and enhance neuroaxonal protection in the CNS. LINGO-1 has been described in International Applications PCT/US2006/026271, filed Jul. 7, 2006, PCT/US2004/008323, filed Mar. 17, 2004, PCT/US2005/022881, filed Jun. 24, 2005 and PCT/US2008/000316, filed Jan. 9, 2008, each of which is incorporated by reference in its entirety herein.

In one embodiment, the reparative agent, *e.g.,* the LINGO-1 antagonist, inhibits or reduces the expression or activity of LINGO-1, *e.g*., human LINGO-1.

In one embodiment, the reparative agent, *e.g.,* the LINGO-1 antagonist, inhibits or reduces the formation and/or activity of a complex (*e.g.,* a functional signaling complex) of the NgR1, p75, and LINGO-1; and/or NgR1, TAJ (TROY), and LINGO-1. In another embodiment, the reparative agent, *e.g.,* the LINGO-1 antagonist, inhibits or reduces LINGO-1 binding to NgR1.

In one embodiment, the reparative agent, *e.g.,* the antagonist of LINGO-1, is an antibody molecule. In one embodiment, the antibody molecule reduces the formation and/or activity of a complex (*e.g.,* a functional signaling complex) of the NgR1, p75, and LINGO-1; and/or NgR1, TAJ (TROY), and LINGO-1. In one embodiment, the antibody molecule binds to at least one of the components of the complex (*e.g*., at least one of NgR1, p75, and LINGO-1; and/or NgR1, TAJ (TROY), and LINGO-1), and inhibits or reduces the functional signaling.

In one embodiment, the antibody molecule binds to LINGO, *e.g*., human LINGO. In another embodiment, the antibody molecule binds to LINGO-1, *e.g*., human LINGO-1. The antibody molecule can be a monoclonal or single specificity antibody, or an antigen-binding fragment thereof (*e.g.,* an Fab, F(ab')₂, Fv, a single chain Fv fragment, a single domain antibody, a diabody (dAb), a bivalent or bispecific antibody or fragment thereof, a single domain variant thereof) that binds to LINGO-1, *e.g.,* a mammalian (*e.g.,* human LINGO-1 (or a functional variant thereof)). In one embodiment, the antibody molecule is a monoclonal antibody against LINGO-1, *e.g*., human LINGO-1. Typically, the antibody molecule is a human, a humanized, a CDR-grafted, a chimeric, a camelid, or an *in vitro* generated antibody to human LINGO-1 (or functional fragment thereof, *e.g.,* an antibody fragment as described herein). Typically, the antibody inhibits, reduces or neutralizes one or more activities of LINGO-1 (*e.g.,* one or more biological activities of LINGO-1 as described herein).

The antibody molecule can be full-length (*e.g.,* can include at least one, and typically two, complete heavy chains, and at least one, and typically two, complete light chains) or can include an antigen-binding fragment (*e.g.,* a Fab, an F(ab')₂, an Fv, a single chain Fv fragment, or a single domain antibody or fragment thereof). In yet other embodiments, the antibody molecule has a heavy chain constant region chosen from, *e.g.,* the heavy chain constant region of IgG1, IgG2, IgG3, IgG4, IgM, IgA1, IgA2, IgD, and IgE; particularly, chosen from, *e.g.,* the (*e.g*., human) heavy chain constant regions of IgG1, IgG2, IgG3, and IgG4. In another embodiment, the antibody molecule has a light chain constant region chosen from, *e.g.,* the (*e.g.,* human) light chain constant regions of kappa or lambda. The framework region or constant region of the antibody molecule can be altered, *e.g*., mutated, to modify the properties of the antibody (*e.g.*, to increase or decrease one or more of: Fc receptor binding, antibody glycosylation, the number of cysteine residues, effector cell function, and/or complement function). In one embodiment, the framework or constant region of the antibody molecule is altered, *e.g*., mutated, to decrease one or more of: Fc receptor binding, antibody glycosylation, the number of cysteine residues, effector cell function, and/or complement function. In one embodiment, the framework region of the antibody molecule is modified to reduce antibody glycosylation, effector cell and/or complement function. In one embodiment, the antibody molecule includes an aglycosyl framework.

In another embodiment, the antibody molecule binds to LINGO-1, *e.g*., human LINGO-1, and is an immunoglobulin G subclass 1 (IgG1). In certain embodiments, the antibody molecule is modified to reduce effector cell and complement function compared to wild-type IgG1. In one embodiment, the antibody molecule includes an aglycosyl (IgG1) framework.

In certain embodiments, the antibody molecule specifically binds to the same, or substantially the same, LINGO-1 epitope as the reference monoclonal antibody Li62 or Li81, described in U.S. Patent No. 8,058,406 and U.S. Patent No. 8,128,926, both of which are incorporated by reference in their entirety herein. In an embodiment, the antibody molecule comprises, consists essentially of, or consists of, an immunoglobulin heavy chain variable region (VH) wherein the CDR1, CDR2 and CDR3 regions are selected from the amino acid sequences shown in Table 3, or an amino acid sequence substantially identical thereto (*e.g.,* an amino acid sequence at least 80%, 85%, 90% or 95% identical to the amino acid sequences shown in Table 3; or at least 80%, 85%, 90, 95% or 100% identical to the VH CDR1, CDR2 and CDR3 regions of the immunoglobulin heavy chain of Li62 or Li81).

In some embodiments, the antibody molecule includes a VH that comprises, consists essentially of, or consists of, the amino acid sequence of SEQ ID NO: 4 or SEQ ID NO:8 or any one of SEQ ID NOs: 17 to 49, or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise, consist essentially of, or consist of, the amino acids of SEQ ID NOs: 6, 7, and 8, respectively, or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise, consist essentially of, or consist of, the amino acids of SEQ ID NOs: 2, 3, and 30, respectively, or an amino acid sequence substantially identical thereto (*e.g.,* an amino acid sequence at least 80%, 85%, 90% or 95% identical thereto).

In other embodiments, the antibody molecule includes an immunoglobulin light chain variable region (VL) wherein the CDR1, CDR2 and CDR3 regions are selected from the polypeptide sequences shown in Table 4, or an amino acid sequence substantially identical thereto (*e.g.,* an amino acid sequence at least 80%, 85%, 90% or 95% identical to the amino acid sequences shown in Table 4; or at least 80%, 85%, 90%, 95% or 100% identical to the VL CDR1, CDR2 and CDR3 regions of the immunoglobulin light chain of Li62 or Li81).

In one embodiment, the antibody molecule includes a VL wherein the VL CDR1, CDR2, and CDR3 comprise, consist essentially of, or consist of, the amino acids of SEQ ID NOs: 14, 15, and 16, respectively, or an amino acid sequence substantially identical thereto (*e.g.,* an amino acid sequence at least 80%, 85%, 90% or 95% identical thereto).

In one embodiment, the antibody molecule includes a VL wherein the VL CDR1, CDR2, and CDR3 comprise, consist essentially of, or consist of, the amino acids of SEQ ID NOs: 10, 11, and 12, respectively, or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise, consist essentially of, or consist of, the amino acids of SEQ ID NOs: 6, 7, and 8, respectively; and a VL wherein the VL CDR1, CDR2, and CDR3 comprise, consist essentially of, or consist of, the amino acids of SEQ ID NOs: 14, 15, and 16, respectively; or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise, consist essentially of, or consist of, the amino acids of SEQ ID NOs: 2, 3, and 30, respectively; and a VL wherein the VL CDR1, CDR2, and CDR3 comprise, consist essentially of, or consist of, the amino acids of SEQ ID NOs: 10, 11, and 12, respectively; or an amino acid sequence substantially identical thereto *(e.g.,* an amino acid sequence at least 80%, 85%, 90% or 95% identical thereto).

In other embodiments, the antibody molecule includes a VH selected from the group consisting of SEQ ID NOs: 1, 5, and 53-85, or an amino acid sequence substantially identical thereto (*e.g.,* an amino acid sequence at least 80%, 85%, 90% or 95% identical to said SEQ ID NOs: 1, 5 and 53-85).

In one embodiment, the antibody molecule includes a VH that comprises, consists essentially of, or consists of, the amino acid sequence of SEQ ID NO: 5 or an amino acid sequence substantially identical thereto (*e.g.,* an amino acid sequence at least 80%, 85%, 90% or 95% identical to said SEQ ID NO: 5).

In one embodiment, the antibody molecule includes a VH that comprises, consists essentially of, or consists of, the amino acid sequence of SEQ ID NO:66, or an amino acid sequence substantially identical thereto (*e.g*., an amino acid sequence at least 80%, 85%, 90% or 95% identical to said SEQ ID NO: 66).

In yet other embodiments, the antibody molecule includes a VL selected from the group consisting of SEQ ID NOs: 9 and 13, as shown in Table 4, or an amino acid sequence substantially identical thereto (*e.g.,* an amino acid sequence at least 80%, 85%, 90% or 95% identical to said SEQ ID NOs: 9 and 13, as shown in Table 4).

In one embodiment, the antibody molecule includes a VL that comprises, consists essentially of, or consists of, the amino acid sequence of SEQ ID NO:13, or an amino acid sequence substantially identical thereto (*e.g.,* an amino acid sequence at least 80%, 85%, 90% or 95% identical to said SEQ ID NO: 13).

In one embodiment, the antibody molecule includes a VL that comprises, consists essentially of, or consists of, the amino acid sequence of SEQ ID NO:9, or an amino acid sequence substantially identical thereto (*e.g.,* an amino acid sequence at least 80%, 85%, 90% or 95% identical to said SEQ ID NO: 9).

In one embodiment, the antibody molecule includes a VH that comprises, consists essentially of, or consists of, the amino acid sequence of SEQ ID NO:5, or an amino acid sequence substantially identical thereto (*e.g.,* an amino acid sequence at least 80%, 85%, 90% or 95% identical to said SEQ ID NO: 5); and a VL that comprises, consists essentially of, or consists of, the amino acid sequence of SEQ ID NO: 13, or an amino acid sequence substantially identical thereto (*e.g.,* an amino acid sequence at least 80%, 85%, 90% or 95% identical to said SEQ ID NO: 13).

In one embodiment, the antibody molecule includes a VH that comprises, consists essentially of, or consists of, the amino acid sequence of SEQ ID NO:66, or an amino acid sequence substantially identical thereto (*e.g.,* an amino acid sequence at least 80%, 85%, 90% or 95% identical to said SEQ ID NO: 66); and a VL that comprises, consists essentially of, or consists of, the amino acid sequence of SEQ ID NO: 9, or an amino acid sequence substantially identical thereto (*e.g.,* an amino acid sequence at least 80%, 85%, 90% or 95% identical to said SEQ ID NO: 9).

In another embodiment, the antibody molecule includes a heavy chain as shown below, that comprises, consists essentially of, or consists of, the amino acid sequence of SEQ ID NO: 275, or a sequence substantially identical thereto (*e.g.,* an amino acid sequence at least 80%, 85%, 90% or 95% identical thereto), as follows:

EVQLLESGGG LVQPGGSLRL SCAASGFTFS AYEMKWVRQA PGKGLEWVSV

IGPSGGFTFY ADSVKGRFTI SRDNSKNTLY LQMNSLRAED TAVYYCATEG
DNDAFDIWGQ GTTVTVSSAS TKGPSVFPLA PSSKSTSGGT AALGCLVKDY
FPEPVTVSWN SGALTSGVHT FPAVLQSSGL YSLSSVVTVP SSSLGTQTYI
CNVNHKPSNT KVDKKVEPKS CDKTHTCPPC PAPELLGGPS VFLFPPKPKD
TLMISRTPEV TCVVVDVSHE DPEVKFNWYV DGVEVHNAKT KPREEQYNSA
YRVVSVLTVL HQDWLNGKEY KCKVSNKALP APIEKTISKA KGQPREPQVY
TLPPSRDELT KNQVSLTCLV KGFYPSDIAV EWESNGQPEN NYKTTPPVLD
SDGSFFLYSK LTVDKSRWQQ GNVFSCSVMH EALHNHYTQK SLSLSPG (SEQ ID
NO: 275).

In other embodiments, the antibody molecule comprises, consists essentially of, or consists of, a light chain as shown below, comprising the amino acid sequence of SEQ ID NO: 276, or a sequence substantially identical thereto (*e.g.,* an amino acid sequence at least 80%, 85%, 90% or 95% identical thereto), as follows:

DIQMTQSPAT LSLSPGERAT LSCRASQSVS SYLAWYQQKP GQAPRLLIYD
ASNRATGIPA RFSGSGSGTD FTLTISSLEP EDFAVYYCQQ RSNWPMYTFG
QGTKLEIKRT VAAPSVFIFP PSDEQLKSGT ASVVCLLNNF YPREAKVQWK
VDNALQSGNS QESVTEQDSK DSTYSLSSTL TLSKADYEKH KVYACEVTHQ
GLSSPVTKSF NRGEC (SEQ ID NO: 276).

In another embodiment, the reparative agent, *e.g.,* the antagonist of LINGO-1, is a soluble LINGO molecule, *e.g.,* a LINGO-1 molecule (*e.g.,* a fragment of LINGO-1), or a soluble form of a component of the LINGO-1 complex (*e.g.,* a soluble form of NgR1, p75, or TAJ (TROY)).

A soluble form of LINGO or a complex component can be used alone or functionally linked (*e.g.,* by chemical coupling, genetic or polypeptide fusion, noncovalent association or otherwise) to a second moiety, *e.g*., an immunoglobulin Fc domain, serum albumin, pegylation, a GST, Lex-A, an MBP polypeptide sequence, or an antibody (*e.g*., a bispecific or a multispecific antibody). The fusion proteins may additionally include a linker sequence joining the first moiety, *e.g*., the soluble form of LINGO-1 or the complex component, to the second moiety. In other embodiments, additional amino acid sequences can be added to the N- or C-terminus of the fusion protein to facilitate expression, steric flexibility, detection and/or isolation or purification. For example, a soluble form of LINGO-1 or a complex component can be fused to a heavy chain constant region of the various isotypes, including: IgG1, IgG2, IgG3, IgG4, IgM, IgA1, IgA2, IgD, and IgE. Typically, the fusion protein can include the extracellular domain of LINGO or the complex component (or a sequence homologous thereto), and, *e.g.,* fused to, a human immunoglobulin Fc chain, *e.g.,* a human IgG (*e.g.,* a human IgG1 or a human IgG2, or a mutated form thereof). The Fc sequence can be mutated at one or more amino acids to reduce effector cell function, Fc receptor binding and/or complement activity.

In another embodiment, one or more reparative agents are added in combination. For example, a LINGO-1 antagonist can be added in combination with another remyelinating agent.

### Immunomodulatory agents

The methods, kits and compositions described herein can include one or more immunomodulatory agents. In certain embodiments, the immunomodulatory agent is chosen from one or more of:
an IFN-β 1 molecule;
a polymer of glutamic acid, lysine, alanine and tyrosine, *e.g.,* glatiramer (*e.g.,* Copaxone®);
an antibody or fragment thereof against alpha-4 integrin, *e.g.,* natalizumab (*e.g*., Tysabri®);
an anthracenedione molecule, *e.g.,* mitoxantrone (*e.g.,* Novantrone®);
a fingolimod, *e.g.,* FTY720 (*e.g.,* Gilenya®);
a dimethyl fumarate, *e.g.,* an oral dimethyl fumarate (*e.g.,* Tecfidera®);
an antibody to the alpha subunit of the IL-2 receptor of T cells, *e.g*., daclizumab;
an antibody against CD52, *e.g.,* alemtuzumab (*e.g.,* CAMPATH);
an inhibitor of a dihydroorotate dehydrogenase, *e.g*., leflunomide or an active metabolite thereof, *e.g.,* teriflunomide (*e.g.,* AUBAGIO);
an antibody to CD20, *e.g*., ocrelizumab;
a Sphingosine 1-phosphate (S1P) modulating agent, *e.g.,* as described in WO 2012/109108; or
a corticosteroid.

In one embodiment, the immunomodulatory agent is an IFN-β 1 molecule. The IFN-β 1 molecule can be chosen from one or more of an IFN-β1a or IFN-β 1b polypeptide, a variant, a homologue, a fragment or a pegylated variant thereof.

In one embodiment, the IFN-β 1 molecule includes an IFNβ agent chosen from an IFN-β1a molecule, an IFN-β1b molecule, or a pegylated variant of an IFN-β1a molecule or an IFN-β 1b molecule.

In one embodiment, the IFNβ1 molecule is an IFN-β1a agent (*e.g*., Avonex®, Rebif®). In another embodiment, the IFNβ1 molecule is an INF-β1b agent (*e.g.,* Betaseron®, Betaferon® or Extavia®).

In one embodiment, the immunomodulatory agent is a polymer of glutamic acid, lysine, alanine and tyrosine, *e.g.,* glatiramer (*e.g.,* Copaxone®).

In one embodiment, the immunomodulatory agent is an antibody or fragment thereof against alpha-4 integrin (*e.g.,* natalizumab (*e.g*., Tysabri®)).

In yet other embodiments, the immunomodulatory agent is an anthracenedione molecule (*e.g.,* mitoxantrone (*e.g.,* Novantrone®)).

In yet another embodiment, the immunomodulatory agent is a fingolimod (*e.g*., FTY720; *e.g*., Gilenya®).

In one embodiment, the immunomodulatory agent is a dimethyl fumarate (*e.g*., an oral dimethyl fumarate (*e.g*., BG-12)).

In other embodiments, the immunomodulatory agent is an antibody to the alpha subunit of the IL-2 receptor of T cells (*e.g*., Daclizumab).

In other embodiments, the immunomodulatory agent is an antibody to CD20, *e.g.,* ocrelizumab.

In other embodiments, the immunomodulatory agent is a corticosteroid, *e.g*., methylprednisolone (*e.g.,* high dose corticosteroid, *e.g.,* methylprednisolone).

In certain embodiments, the method further includes the use of one or more symptom management therapies, such as antidepressants, analgesics, anti-tremor agents, among others.

Any combination of the reparative agent (*e.g.,* one or more reparative agents described herein, *e.g.,* a LINGO-1 antagonist) and an immunomodulatory agent (*e.g.,* one or more immunomodulatory agents described herein) can be used in the methods, kits and compositions described herein. For example, the reparative agent can be combined with a polymer of glutamic acid, lysine, alanine and tyrosine, *e.g.*, glatiramer. In other embodiments, the reparative agent can be combined with an antibody or fragment thereof against alpha-4 integrin, *e.g*., natalizumab. In yet another embodiment, the reparative agent can be combined with an anthracenedione molecule, *e.g*., mitoxantrone. In yet another embodiment, the reparative agent can be combined with a fingolimod, *e.g*., FTY720. In yet another embodiment, the reparative agent can be combined with a dimethyl fumarate, *e.g*., an oral dimethyl fumarate. In other embodiments, the reparative agent can be combined with an antibody to the alpha subunit of the IL-2 receptor of T cells, *e.g*., daclizumab. In yet another embodiment, the reparative agent can be combined with an antibody against CD52, *e.g*., alemtuzumab. In yet another embodiment, the reparative agent can be combined with an inhibitor of a dihydroorotate dehydrogenase, *e.g*., teriflunomide. In another embodiment, the reparative agent can be combined with an antibody to CD20, *e.g*., ocrelizumab. In another embodiment, the reparative agent can be combined with a corticosteroid, *e.g*., methylprednisolone. In one embodiment, the reparative agent can be combined with a S1P modulating agent.

In other embodiment, the reparative agent is combined with two, three, four or more immunomodulatory agents, *e.g*., two, three, four or more of the immunomodulatory agents described herein. In one exemplary embodiment, a combination of a LINGO antagonist, an IFN-β 1 molecule and a corticosteroid is used. In other embodiments, a combination of a LINGO antagonist, an IFN-β 1 molecule and a polymer of glutamic acid, lysine, alanine and tyrosine, *e.g*., glatiramer, is used. In yet other embodiments, a combination of a LINGO antagonist, an IFN-β 1 molecule and an antibody or fragment thereof against alpha-4 integrin, *e.g*., natalizumab, is used.

In certain embodiment of the methods, kits and compositions described herein, the reparative agent is an antibody molecule against LINGO-1, *e.g.,* an anti-LINGO antibody as described herein, and the immunosuppressive agent is an IFN-β 1 molecule, *e.g.,* an IFN-β 1 molecule as described herein.

### Combination therapy and timing of administration

The combinations of reparative agent (*e.g.,* LINGO-1 antagonist) and the immunomodulatory agent described herein can be administered in any order, *e.g.,* concurrently or sequentially as described herein. In one embodiment, the reparative agent and the immunomodulatory agent are administered concurrently. In another embodiment, the reparative agent and the immunomodulatory agent are administered sequentially. For example, the administration of the reparative agent and the immunomodulatory agent can overlap, at least in part or completely, with each other.

In certain embodiments, initiation of the administration of the immunomodulatory agent and the reparative agent occurs at the same time. In other embodiments, the immunomodulatory agent is administered before initiating treatment with the reparative agent. In yet other embodiments, the reparative agent is administered before initiating treatment with the immunomodulatory agent. In another embodiment, the administration of the immunomodulatory agent continues after cessation of administration of the reparative agent. In other embodiments, administration of the reparative agent continues after cessation of administration of the immunomodulatory agent. In other embodiments, administration of the reparative agent continues intermittently (*e.g*., for 2 or 3 months every 3 or 6 or 12 months, or for 3-6 months every 1-2 years) while the immunomodulatory agent is given continuously.

In certain embodiments, the reparative agent is an antibody molecule against LINGO-1 and is administered intravenously, subcutaneously or intramuscularly. In one embodiment, the antibody molecule is administered intravenously. In such embodiments, the antibody molecule is administered at about 1 to 100 mg/kg (typically, at about 3 mg/kg, about 10 mg/kg, about 30 mg/kg, about 50mg/kg or about 100 mg/kg). In some embodiments, the antibody molecule is administered once every one, two, three, four or five weeks by IV infusion.

In certain embodiments, the immunomodulatory agent is an IFN-β 1 molecule is administered intravenously, subcutaneously or intramuscularly. For example, the IFN-β 1 molecule can be administered at one or more of:
(i) at 20-45 microgram (*e.g.,* 30 microgram), *e.g.,* once a week via intramuscular injection;
(ii) at 20-30 microgram (*e.g.,* 22 microgram), *e.g.,* three times a week, or at 40-50 micrograms (*e.g.,* 44 micrograms), *e.g.,* once a week, via subcutaneous injection; or
(iii) in an amount of between 10 and 50 µg intramuscularly, *e.g*., three times a week, or every five to ten days, *e.g.,* once a week; or
(iv) in an amount between 200 and 600 µg (*e.g.,* between 250 and 500 µg), *e.g.,* every other day, via subcutaneous injection. In one embodiment, the IFN-β 1 molecule is an interferon β-1b (Betaseron®/Betaferon®, or Extavia®).

In other embodiments, the reparative agent is an antibody molecule against LINGO-1 and is administered once every four weeks by IV infusion dosed at about 3 mg/kg, about 10 mg/kg, about 30 mg/kg, 50mg/kg or about 100 mg/kg; and
the immunomodulatory agent the IFN-β 1 is administered at one or more of:
(i) at 20-45 microgram (*e.g.,* 30 microgram), *e.g*., once a week via intramuscular injection;
(ii) at 20-30 microgram (*e.g.,* 22 microgram), *e.g*., three times a week, or at 40-50 micrograms (*e.g.,* 44 micrograms), *e.g.,* once a week, via subcutaneous injection; or
(iii) in an amount of between 10 and 50 µg intramuscularly, *e.g*., three times a week, or every five to ten days, *e.g.,* once a week.

### Subjects

For any of the methods, compositions and kits disclosed herein, the subject treated, is a subject (*e.g.,* a human) having, or at risk of having, a CNS disorder or a CNS demyelinating disease, *e.g*., as described herein.

In one embodiment, the subject (*e.g*., the human) has, or is at risk of having, MS. The subject with MS can be at any stage of treatment. In certain embodiments, the subject with MS is chosen from a human having one or more of: Benign MS, RRMS (*e.g*., quiescent RRMS, active RRMS), primary progressive MS (PPMS), or secondary progressive MS (SPMS), clinically isolated syndrome (CIS), or clinically defined MS (CDMS). In one embodiment, the subject is asymptomatic. In other embodiments, the subject has one or more MS-like symptoms, such as those having clinically isolated syndrome (CIS) or clinically defined MS (CDMS). In other embodiments, the subject has one or more MS relapses (*e.g*. acute optic neuritis, transverse myelitis, brainstem syndrome).

In one embodiment, the subject has a relapsing form of MS (*e.g.,* RRMS or relapsing SPMS). In one embodiment, the subject has RRMS and has one or more ongoing clinical exacerbations and/or subclinical activity, *e.g*., as shown by gadolinium (Gd) enhancement or development of new and/or enlarged T2/FLAIR lesions on magnetic resonance imaging (*e.g*., brain or spinal cord MRI). In another embodiment, the subject has SPMS and has one or more ongoing clinical exacerbations and/or subclinical activity, *e.g*., as shown by gadolinium (Gd) enhancement or development of new and/or enlarged T2/FLAIR lesions on magnetic resonance imaging (*e.g*., brain or spinal cord MRI). In one embodiment, the subject has an active form of MS, *e.g.,* an active RRMS. In other embodiments, the MS subject has at least one newly developed lesion. In other embodiment, the MS subject has at least one pre-existing lesion. In one embodiment, the subject has RRMS, and has one or more newly developed or pre-existing lesions, or a combination thereof. In other embodiments, the subject has a baseline EDSS score of 1.5 to 7.

In one embodiment, the subject is an MS patient (*e.g.,* a patient with RRMS or SPMS) prior to administration of an MS therapy (a monotherapy or a combination therapy of the agents described herein). In one embodiment, the subject is a newly diagnosed or an undiagnosed RRMS or SPMS patient or a subject with a radiologically isolated syndrome. In another embodiment, the subject is an MS patient (*e.g*., an RRMS patient) after administration of an MS therapy described herein (a monotherapy or a combination therapy of the agents described herein). In other embodiments, the subject is an MS patient after administration of the MS therapy for one, two weeks, one month, two months, three months, four months, six months, one year or more.

### Subject Monitoring

Alternatively, or in combination, with the methods disclosed herein, a method of evaluating, diagnosing, and/or monitoring the progression of, a CNS disorder or a CNS demyelinating disease is disclosed. The method includes evaluating a subject (*e.g.,* a patient, a patient group or a patient population), having the CNS disorder or CNS demyelinating disease, or at risk of developing the disorder. In one embodiment, the subject is evaluated using (i) an assessment of neurological function (*e.g*., EDSS); and/or (ii) an assessment of physical function. For example, an assessment of physical function can include an assessment of ambulatory function (*e.g*., short distance and/or longer distance ambulatory function), alone or in combination with an assessment of upper and/or lower extremity function.

In certain embodiments, the subject is evaluated by one or more of:
performing a neurological examination;
acquiring the subject's status on the Expanded Disability Status Scale (EDSS);
acquiring the subject's status on the Multiple Sclerosis Functional Composite (MSFC);
detecting the subject's lesion status, *e.g*., as assessed using an MRI;
acquiring a measure of upper and/or lower extremity function;
acquiring a measure of ambulatory function (*e.g*., short distance ambulatory function) (*e.g*., Timed Walk of 25 Feet (T25FW)); or long distance ambulatory function (*e.g*. the 6 minute walk test (6MW);
acquiring a measure of cognitive function (*e.g*., an MS-COG); or
acquiring an assessment of visual function.

In one embodiment, the measure of upper extremity function is acquired using a 9 Hole Peg Test (9HP).

In other embodiments, the measure of short distance ambulatory function is acquired using a Timed Walk of 25 Feet (T25FW).

In other embodiments, the measure of long distance ambulatory function is acquired using a 6 minute walk test (6MW).

In certain embodiments, an increase by at least 10%, 15%, 20%, 25% or higher in a measure of extremity and/or ambulatory function is indicative of disease progression, *e.g.,* a steady worsening of symptoms and/or disability, in the subject; and a decrease of at least 10%, 15%, 20%, 25% or more in a measure of extremity and/or ambulatory function as described above is indicative of an improved outcome (*e.g.,* a decrease in disease progression or an improved condition) in the subject.

In certain embodiments, the subject is evaluated using an assessment of neurological function, *e.g*., EDSS. In some embodiments, the EDSS includes an assessment of neurological function, an assessment of ambulatory function, or both. In one embodiment, an EDSS score is calculated based on a combination of one or more scores for the EDSS functional systems (FS) (e*.g.,* one, two, three, four, five, six, or all seven individual scores for the EDSS FS chosen from visual, brainstem, cerebellar, motor, sensory, bladder/bowel or cognitive systems). In other embodiments, the EDSS includes a score for ambulation. In one embodiment, the EDSS includes a determination of a subject's ambulation that includes an assessment of one or more (or all) of: Unrestricted ambulation, *e.g*., without aid or rest for a predetermined distance (*e.g*., a distance greater or equal to 500, 300, 200, or 100 meters, or less than 200 or 100 meters); unilateral assistance; bilateral assistance; essentially or fully restricted to a wheelchair; or essentially or fully restricted to a bed.

In one embodiment, the assessment of visual function is acquired by one or more of: *e.g*., low-contrast letter acuity (LCLA), Visual Function Questionnaire (VFQ), Functional Acuity Contrast Testing (FACT), VEPs (described *e.g*., in MacKay, AM (2008) Invest Ophthalmol Vis Sci. 49(1):438-41), optical coherence tomography (OCT), some of which are described in, *e.g.,* Balcer et al. (2010) Neurology 74 Suppl 3:S16-23; Bock, M. et al. (2012) Br J Ophthalmol. 96(1):62-7).

In yet other embodiments, the measure of cognitive function comprises an evaluation of a learning test, a memory test and/or an attention/processing speed test. For example, the measure of cognitive function can include an evaluation of one or more of auditory memory, verbal learning and/or remembering visual information (*e.g*., Selective Reminding Test (SRT)); tests for evaluating auditory/verbal memory (*e.g*., California Verbal Learning Test Second Edition (CVLT2)), the Rey Auditory Verbal Learning Test (RAVLT); tests for evaluating visual/spatial memory (*e.g*., Brief Visuospatial Memory Test Revised (BVMTR)); processing speed cognitive tests, *e.g*., Paced Auditory Serial Addition Test (PASAT), Symbol Digit Modalities Test (SDMT); MSNQ-information, MSNQ-subject, and/or SF-36. In one embodiment, the measure of cognitive function is performed using a composite of MS cognitive endpoint that comprises SDMT, PASAT-3 and -3, SRT-Total Learned (SRT-TL), SRT Delayed Recall (SRT-DR), and BVMTR Delayed Recall (BVMTR-DR) (*e.g.,* MS-COG as described in Cadavid et al,, 29th Congress European Committee for Treatment and Research in MS (ECTRIMS), 2-5 October 2013).

In certain embodiments, the subject's lesion status is evaluated using magnetic resonance imaging. In one embodiment, the magnetic resonance imaging comprises magnetic transfer and/or diffusion tensor imaging.

In certain embodiments, an improvement in the subject is defined by one or more of:
a. ≥1.0 point decrease in EDSS from a baseline score of ≤6.0;
b. ≥15% improvement from baseline in T25FW;
c. ≥15% improvement from baseline in 9HPT; or
d. ≥15% improvement from baseline in PASAT.

In other embodiments, the method further includes one or more of the following:
(i) identifying the subject as being in need of a therapy, *e.g.,* a therapy as described herein;
(ii) identifying the subject as having an increased or a decreased response to a therapy, *e.g.,* a therapy as described herein;
(iii) identifying the subject as being stable, as showing an improvement in function or abilities (*e.g*., as being a disease non-progressor), or showing a decline in function or abilities (*e.g*., as being a disease progressor);
(iv) diagnosing, and/or prognosing the subject.

The steps in the methods described herein (*e.g*., administration of the reparative agent and immunomodulatory agent ("administration step"), and subject monitoring and/or evaluating ("evaluating step") can be performed in any order. In one embodiment, the administration step occurs prior to the evaluating step. In another embodiment, the evaluating step occurs prior to the administration step.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting.

Other features and advantages of the invention will be apparent from the detailed description, drawings, and from the claims.

### BRIEF DESCRIPTION OF THE FIGURES

FIG. 1 depicts the region of interest (ROI) selection for the quantification of optic nerve axonal density in the mouse model of experimental autoimmune encephalomyelitis (EAE).
FIG. 2A is a line graph showing the survival curve of EAE mice treated with vehicle or anti-LINGO-1 antibody. FIG. 2B is a line graph showing the development of complete paraplegia in EAE mice treated with vehicle or anti-LINGO-1 antagonist.

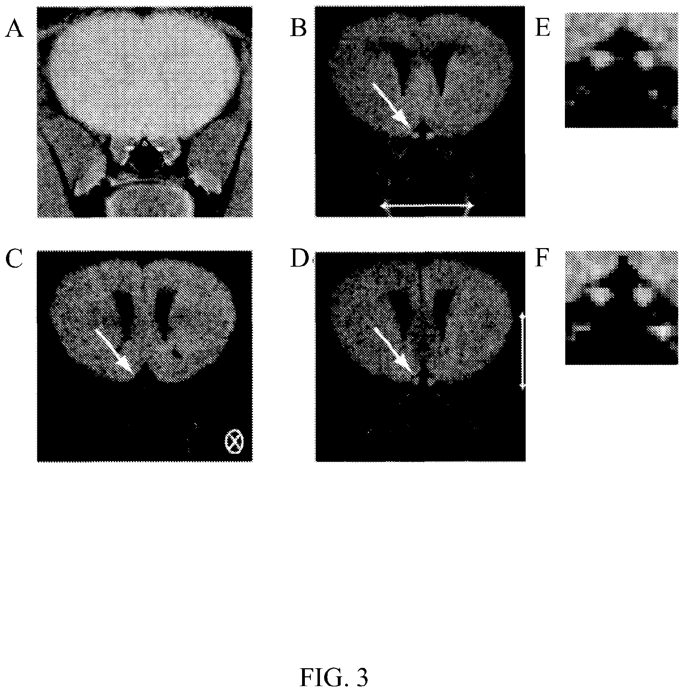

FIGS. 3A-3F contain images of the coronal optic nerve diffusion imaging in mouse EAE. Diffusion direction is indicated by the small arrows. The right optic nerve location is indicated by the large arrows. FIG. 3A is an image of a T2 weighted localizer scan. FIG. 3B is an image of a diffusion weighted image perpendicular to the optic nerve. FIG. 3C is an image of a diffusion weighted scan parallel to the optic nerve. FIG. 3D is an image of a diffusion weighted image perpendicular to the optic nerve. FIG. 3E is an enlargement of the optic nerve image of FIG. 3B. FIG. 3F is an enlargement of the optic nerve image of FIG. 3D.
FIG. 4 is a bar graph depicting the optic nerve integrity analyzed by diffusion tensor imaging (DTI) in mouse EAE.
FIG. 5 depicts the histological analysis of the optic nerve in EAE mice treated with vehicle or anti-LINGO-1 antibody or healthy mice.
FIG. 6 depicts the measurement of axonal loss in the optic nerve in EAE mice treated with vehicle or anti-LINGO-1 antibody or healthy mice. FIG. 6 includes measurements of optic nerve area (µm²), average central axon area (µm²), total central axon count, total peripheral axon count, total central axo-plasmal area (µm²), and total peripheral axo-plasmal area (µm²).
FIG. 7 depicts histological analysis of sections of optic nerve detected by anti-βIII tubulin staining and DAPI, respectively, after the following treatments: treatment group (Veh + control Antibody), methylprednisolone (MP), anti-LINGO-1 antibody, and MP + anti-LINGO-1 antibody.
FIG. 8 is a bar graph reflecting the axonal segment count/field in the treatment groups indicated. The anti-LINGO-1 monoclonal antibody treatment group (Veh + anti-LINGO-1 monoclonal antibody) showed 5-fold higher axonal numbers, suggesting that anti-LINGO-1 monoclonal antibody treatment prevented axonal loss (FIG. 8). The combination treatment group (MP + anti-LINGO-1 monoclonal antibody) showed an 8-fold increase in axonal numbers compared with the control treatment group (Veh + control Antibody.

### DETAILED DESCRIPTION OF THE INVENTION

Inflammatory demyelinating CNS diseases, such as MS, are a common cause of non-traumatic neurological disability in young adults. Currently approved therapies for MS are primarily immunomodulatory, and do not have detectable direct effects on CNS repair. For example, the current standard of care for patients with relapsing MS includes the use of immunomodulatory drugs to reduce the frequency and severity of relapses and the accumulation of relapse-related physical disability, and to provide various symptomatic treatment as needed such as for depression, bladder dysfunction, or walking impairment. Several immunomodulatory drugs are currently available for relapsing MS, including, but not limited to, different preparations of interferon β (interferon β-1a given intramuscularly [IM] [Avonex] or subcutaneously [SC] [Rebif®], interferon β-1b [Betaseron/Betaferon®/Extavia®]), glatiramer acetate (Copaxone®), natalizumab (Tysabri®), and fingolimod (Gilenya®). Short courses of corticosteroids are occasionally given with mixed success. Chemotherapeutic agents, such as mitoxantrone and cyclophosphamide, are occasionally used in cases of severe relapsing MS. Although some degree of axonal remyelination by oligodendrocytes takes place early during the course of MS, the ability to endogenously repair the CNS often fails, leading to irreversible tissue injury and an increase in disease-related disability.

Several preclinical studies have demonstrated a role for LINGO-1 antagonism in enhancing CNS remyelination and neuroaxonal protection in animal models of toxic injury (Cuprizone) (Mi et al. (2009) Ann Neurology, 65: 304-15), chemical injury (lysophosphatidylcholine [LPC]), and inflammatory demyelination (myelin oligodendrocyte glycoprotein- experimental autoimmune encephalomyelitis [MOG-EAE]) [Mi et al. (2007) Nat Med, 13: 1228-33); and of toxic (1-methyl-4-phenyl-1,2,3,6-tetrahydropyridine [MPTP]) neuronal injury (Inoue et al. (2007) Proc Natl Acad Sci, 104: 14430-5), traumatic/hypertensive optic nerve injury (Fu et al. (2008) Invest Opthalmol Vis Sci, 49: 975-85) and spinal cord injury (Ji et al. (2006) Mol Cell Neurosci, 33: 311-20; Ji et al. (2008) Mol Cell Neurosci, 39: 258-67; Lv et al. (2010) Neuroimmunomodulat, 17: 270-8). Thus, antagonizing LINGO-1 with an anti-LINGO-1 antibody can enhance remyelination and neuroaxonal protection in the CNS. An anti-LINGO-1 antibody can reach the CNS in sufficient concentrations to block LINGO-1 in both axons and oligodendroyctes after peripheral administration. This in turn, can promote remyelination via differentiation of oligodendrocyte precursor cells (OPC) normally present in the brain of MS patients.

Binding of an anti-LINGO-1 antibody to LINGO-1 in axons and neurons can also provide neuroaxonal protection via blockade of signaling by myelin debris on the Nogo66 receptor-1(NgR1)/p75/LINGO-1 receptor complex in the CNS. It has been proposed that the failure of axonal repair/neurite regeneration in MS can be due, at least in part, to signaling of myelin debris on the NgR1/p75/LINGO-1 complex and the NgR1/TROY/LINGO-1 complex in damaged axons (Mi et al. (2004) Nat Neurosci, 7: 221-8). Signaling on the NgR1 receptor complex may interfere not only with axonal regeneration (Yamashita et al. (2005) Mol Neurobiol, 32: 105-11), but also with neuronal survival following neuroaxonal injury (Mi et al. (2004) Nat Neurosci, 7: 221-8; Fu et al. (2008) Invest Opthalmol Vis Sci, 49: 975-85; Zhao et al. (2008) Cell Mol Neurobiol, 28: 727-35).

Without wishing to be bound by theory, it is believed that newly developed lesions may be easier to repair and remyelinate due, at least in part, to the greater preservation of axons and lesser interference from glial scar (Jasmin and Ohara (2002) Neuroscientists 8(3):198-203; Vick et al. (1992) J. Neurotrauma 9 Suppl 1:S93-103). However, reparative effects of LINGO-1 antagonists on pre-existing lesions can also occur. For example, the efficacy of an anti-LINGO-1 antibody treatment in pre-existing lesions is supported by (1) the finding that OPCs are found in chronically demyelinated MS lesions, (2) animal studies that show the ability of chronically demyelinated brain lesions to be remyelinated, and (3) studies showing the enhancement of remyelination by LINGO-1 blockade in established demyelinated lesions (*e.g*., in the Cuprizone model).

Thus, antagonism of LINGO-1 with an anti-LINGO-1 antibody can enhance remyelination and neuroaxonal protection in CNS demyelinating diseases such as MS and acute optic neuritis, leading to improved CNS repair with corresponding beneficial effects on neurological function and disability. Since an anti-LINGO-1 antibody does not have detectable immunomodulatory effects on the inflammatory component of MS pathogenesis, concurrent administration with an immunomodulatory agent is desirable. Therefore, combination treatments of an immunomodulatory agent, *e.g.,* IFN-β agent, *e.g.,* Avonex®; with a reparative agent, *e.g.,* anti-LINGO-1 antibody, are disclosed.

The present invention provides, at least in part, methods, composition and kits for enhancing one or more of: myelination, re-myelination, oligodendrocyte numbers, or neuroaxonal protection in a subject, *e.g.,* a human (*e.g.,* a human MS patient), while ameliorating an inflammatory condition in the subject. Such methods, compositions and kits described herein are useful for treating a CNS disorder, *e.g.,* a CNS demyelinating disease. Accordingly, methods, composition and kits include a reparative agent (*e.g.,* a LINGO-1 antagonist) and an immunomodulatory agent, in combination, as described herein.

In other embodiments, the reparative agent (*e.g.,* a LINGO-1 antagonist) can be used to treat an inflammatory condition of the optic nerve, *e.g.,* optic neuritis (*e.g.,* acute optic neuritis (AON). Thus methods and compositions comprising a reparative agent for treating an inflammatory condition of the optic nerve, *e.g.,* optic neuritis (*e.g.,* AON) are also disclosed.

The term "reparative agent" as used herein includes any agent that causes one or more of: enhances myelination, re- myelination, enhances neuroaxonal protection, increases axonal extension, increases neuronal sprouting, and/or promotes oligodendrocyte numbers (*e.g*., by increasing one or more of: survival or differentiation of oligodendrocytes), without having a substantial (*e.g*., a detectable) immunomodulatory effect. In one embodiment, the reparative agent is a LINGO-1 antagonist, *e.g*., a LINGO-1 antagonist as described herein.

Various aspects of the invention are described in further detail in the following subsections.

### Definitions

As used herein, each of the following terms has the meaning associated with it in this section.

As used herein, the articles "a" and "an" refer to one or to more than one (*e.g.,* to at least one) of the grammatical object of the article.

The term "or" is used herein to mean, and is used interchangeably with, the term "and/or", unless context clearly indicates otherwise.

The terms "proteins" and "polypeptides" are used interchangeably herein.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.

"Acquire" or "acquiring" as the terms are used herein, refer to obtaining possession of, determining, or evaluating, a desired result, *e.g.,* a value, *e.g.,* a numerical value, by "directly acquiring" or "indirectly acquiring" the result. "Directly acquiring" means performing a process (*e.g.,* performing a test, *e.g.,* a measure of upper and/or lower extremity function, and/or ambulatory function) to obtain the result, *e.g.,* the value. "Indirectly acquiring" refers to receiving the result, *e.g.,* the value, from another party or source (*e.g.,* a third party clinician or health professional that directly acquired the value).

A "CNS disorder" (*e.g.,* a "CNS demyelinating disease") can be any disease, disorder or injury associated with one or more of: demyelination, dysmyelination, axonal injury, and/or dysfunction or death of an oligodendrocyte or a neuronal cell, or loss of neuronal synapsis/connectivity. In certain embodiments, the CNS disorder affects the nervous system by causing damage to the myelin sheath of axons. In other embodiments, the CNS disorder includes Nogo receptor-1 (NgR1-) mediated inhibition of axonal extension or neurite extension, *e.g*., in the brain and spinal cord. In other embodiments, the CNS disorder has one or more inflammatory components. In one embodiment, the CNS disorder (*e.g.,* the CNS demyelinating disease) is multiple sclerosis. The CNS disorder (*e.g.,* the CNS demyelinating disease) is "treated," "inhibited" or "reduced," if at least one symptom of the disease or disorder is reduced, alleviated, terminated, slowed, or prevented.

As used herein, multiple sclerosis is "treated," "inhibited," or "reduced," if recurrence or relapse of the disease is reduced, slowed, delayed, or prevented. Exemplary clinical symptoms of multiple sclerosis that can be used to aid in determining the disease status in a subject can include *e.g*., tingling, numbness, muscle weakness, loss of balance, blurred or double vision, slurred speech, sudden onset paralysis, lack of coordination, cognitive difficulties, fatigue, heat sensitivity, spasticity, dizziness, tremors, gait abnormalities, speech/swallowing difficulties, and extent of lesions assessed by imaging techniques, *e.g*., MRI. Clinical signs of MS are routinely classified and standardized, *e.g*., using an EDSS rating system based on neurological examination and long distance ambulation. For the lower end of the scale (1-5.5) a decrease of one full step indicates an effective MS treatment (Kurtzke, Ann. Neurol. 36:573-79, 1994), while an increase of one full step will indicate the progression or worsening of the disease (*e.g*., exacerbation). For the higher end of the scale (5-7), a half a point typically indicates improvement (a reduction) or worsening (an increase).

As used herein, the "Expanded Disability Status Scale" or "EDSS" is intended to have its customary meaning in the medical practice. EDSS is a rating system that is frequently used for classifying and standardizing MS. The accepted scores range from 0 (normal) to 10 (death due to MS). Typically patients having an EDSS score of about 4-6 will have moderate disability (*e.g*., limited ability to walk), whereas patients having an EDSS score of about 7 or 8 will have severe disability (*e.g*., will require a wheelchair). More specifically, EDSS scores in the range of 1-3 refer to an MS patient who is fully ambulatory, but has some signs in one or more functional systems; EDSS scores in the range higher than 3 to 4.5 show moderate to relatively severe disability; an EDSS score of 5 to 5.5 refers to a disability impairing or precluding full daily activities; EDSS scores of 6 to 6.5 refer to an MS patient requiring intermittent to constant, or unilateral to bilateral constant assistance (cane, crutch or brace) to walk; EDSS scores of 7 to 7.5 means that the MS patient is unable to walk beyond five meters even with aid, and is essentially restricted to a wheelchair; EDSS scores of 8 to 8.5 refer to patients that are restricted to bed; and EDSS scores of 9 to 10 mean that the MS patient is confined to bed, and progressively is unable to communicate effectively or eat and swallow, until death due to MS.

As used herein, a "disease progression" includes a measure (*e.g*., one or more measures) of a worsening of one or more symptoms and/or disability in a subject. In certain embodiments, disease progression is evaluated as a steady worsening of one or more symptoms and/or disability over time, as opposed to a relapse, which is relatively short in duration. In certain embodiments, the disease progression is evaluated in a subject with a relapsing form of MS (*e.g.,* RRMS) or a progressive form of MS (*e.g.,* a subject with primary or secondary progressive multiple sclerosis (PPMS or SPMS, respectively), or a subject with progressive-relapsing MS (PRMS)).

In certain embodiments, the evaluation of disease progression includes a measure of upper extremity function (*e.g*., a 9HP assessment). Alternatively or in combination, disease progression includes a measure of lower extremity function. Alternatively or in combination, disease progression includes a measure of ambulatory function, *e.g*., short distance ambulatory function (*e.g*., T25FW). Alternatively or in combination, disease progression includes a measure of ambulatory function, *e.g*., longer distance ambulatory function (*e.g*., a 6-minute walk test). In one embodiment, the disease progression includes a measure of ambulatory function other than EDSS ambulatory function. In one embodiment, disease progression includes a measure of upper extremity function (*e.g*., a 9HP assessment) and a measure of ambulatory function, *e.g*., short distance ambulatory function (*e.g*., T25FW). In one embodiment, disease progression includes a measure of upper extremity function (*e.g*., a 9HP assessment) and a measure of lower extremity function. In one embodiment, disease progression includes a measure of upper extremity function (*e.g*., a 9HP assessment), a measure of lower extremity function, and a measure of ambulatory function, *e.g.,* short distance ambulatory function *(e.g.,* T25FW) and/or longer distance ambulatory function (*e.g.,* a timed (*e.g.,* 6-minute) walk test (*e.g.,* 6MWT)). In one embodiment, one, two or the combination of the T25FW, 6MWT and 9HP assessments can be used to acquire a disease progression value. The measure of ambulatory function (*e.g.,* short distance ambulatory function (*e.g.,* T25FW) or longer distance ambulatory function (*e.g.,* a timed (*e.g.,* 6-minute) walk test (*e.g.,* 6MWT)) and/or measure of upper extremity function (*e.g*., a 9HP assessment) can further be used in combination with the EDSS to evaluate MS, *e.g*., progressive forms of MS.

In one embodiment, a progressor is a subject who possesses a disease progression value reflecting at least one, two or all of the following criteria:
a. confirmed progression in T25FW: Time taken for 25-foot walk increased by at least 15% or 20% of the baseline walk, confirmed at a second time point at least 3, 4, 5, or 6 months apart;
b. confirmed progression in a timed (*e.g.,* 6-minute) walk test (*e.g.,* 6MWT): Time taken for walk increased by at least 15% or 20% of the baseline walk, confirmed at a second time point at least 3, 4, 5, or 6 months apart;
c. confirmed progression in 9HP: Time taken for 9-hole peg increased by at least 15% or 20% of the time taken at baseline, confirmed at a second time point at least 3, 4, 5, or 6 months apart. The progression in 9HP can occur on either hand, but will have to be confirmed on the same hand; and/or
d. confirmed progression in EDSS:
   (i) EDSS total score increase from baseline by at least 1 point, if the change in EDSS total score is determined (or primarily determined) by evaluating a change in neurological function (*e.g*., one or more changes in neurological systems); and/or
   (ii) EDSS total score increased from baseline by at least 0.5 point if the change in EDSS total score is determined (or primarily determined) by a change in ambulatory function,
if either or both of (i) or (ii) is/are confirmed on a second examination at least 3, 4, 5 or 6 months apart (typically, at least 6 months apart).

Baseline values for the aforementioned tests (*e.g*., T25FW, 6MWT, EDSS, or 9HP) can be determined using the best baseline value or the average baseline value.

"Responsiveness," to "respond" to a treatment, and other forms of this term, as used herein, refer to the reaction of a subject to treatment with a therapy as described. As an example, an MS subject responds to therapy if at least one symptom of multiple sclerosis (*e.g*., disease worsening) in the subject is reduced or retarded by about 10%, 20%, 30%, 40%, 50%, 60%, 70%, 80%, 90% or more. In another example, an MS subject responds to a therapy, if at least one symptom of multiple sclerosis in the subject is reduced by about 5%, 10%, 20%, 30%, 40%, 50% or more as determined by any appropriate measure, *e.g*., one or more of: a measure of upper or lower extremity function, a measure of ambulatory function, or an assessment of Expanded Disability Status Scale (EDSS). In another example, an MS subject responds to treatment with a therapy, if the subject has an increased time to progression. Several methods can be used to determine if a patient responds to a treatment including the assessments described herein, as set forth herein.

In certain embodiments, an improvement in the subject is defined by one or more of:
a. ≥1.0 point decrease in EDSS from a baseline score of ≤6.0;
b. ≥15% improvement from baseline in T25FW;
c. ≥15% improvement from baseline in 9HPT; or
d. ≥15% improvement from baseline in PASAT.

A "non-responder" or "progressor" refers to a subject, *e.g*., an MS patient, if in response to a therapy (*e.g.,* a therapy described herein), at least one symptom or disability of multiple sclerosis in the subject is reduced by less than about 5%, as determined by any appropriate measure, *e.g*., one or more of: a measure of upper or lower extremity function, a measure of ambulatory function, a measure of cognitive function, or an assessment of Expanded Disability Status Scale (EDSS).

The methods, compositions and kits disclosed herein encompass polypeptides and nucleic acids having the sequences specified, or sequences substantially identical or similar thereto, *e.g.,* sequences at least 85%, 90%, 95% identical or higher to the sequence specified. In the context of an amino acid sequence, the term "substantially identical" is used herein to refer to a first amino acid that contains a sufficient or minimum number of amino acid residues that are i) identical to, or ii) conservative substitutions of aligned amino acid residues in a second amino acid sequence such that the first and second amino acid sequences can have a common structural domain and/or common functional activity. For example, amino acid sequences that contain a common structural domain having at least about 85%, 90%. 91%, 92%, 93%, 94%, 95%, 96%, 97%, 98% or 99% identity to a sequence described herein are termed substantially identical.

In the context of nucleotide sequence, the term "substantially identical" is used herein to refer to a first nucleic acid sequence that contains a sufficient or minimum number of nucleotides that are identical to aligned nucleotides in a second nucleic acid sequence such that the first and second nucleotide sequences encode a polypeptide having common functional activity, or encode a common structural polypeptide domain or a common functional polypeptide activity. For example, nucleotide sequences having at least about 85%, 90%. 91%, 92%, 93%, 94%, 95%, 96%, 97%, 98% or 99% identity to a sequence described herein are termed substantially identical.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (*e.g*., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch ((1970) J. Mol. Biol. 48:444-453) algorithm which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used unless otherwise specified) are a Blossum 62 scoring matrix with a gap penalty of 12, a gap extend penalty of 4, and a frameshift gap penalty of 5.

The percent identity between two amino acid or nucleotide sequences can be determined using the algorithm of E. Meyers and W. Miller ((1989) CABIOS, 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) J. Mol. Biol. 215:403-10. BLAST nucleotide searches can be performed with the NBLAST program, score = 100, wordlength = 12 to obtain nucleotide sequences homologous to BMP-10/BMP-10 receptor nucleic acid (SEQ ID NO:1) molecules of the invention. BLAST protein searches can be performed with the XBLAST program, score = 50, wordlength = 3 to obtain amino acid sequences homologous to BMP-10/BMP-10 receptor (SEQ ID NO:1) protein molecules of the invention. To obtain gapped alignments for comparison purposes, Gapped BLAST can be utilized as described in Altschul et al., (1997) Nucleic Acids Res. 25:3389-3402. When utilizing BLAST and Gapped BLAST programs, the default parameters of the respective programs (e.g., XBLAST and NBLAST) can be used. See http://www.ncbi.nlm.nih.gov.

Also included are fragments, derivatives, analogs, or variants of the polypeptides, and any combination thereof. The terms "fragment," "variant," "derivative" and "analog" include any polypeptides which retain at least some of the properties of the corresponding native polypeptide. Fragments of polypeptides include proteolytic fragments, as well as deletion fragments. Variants of polypeptides include fragments as described above, and also polypeptides with altered amino acid sequences due to amino acid substitutions, deletions, or insertions. Variants may occur naturally or be non-naturally occurring. Non-naturally occurring variants may be produced using art-known mutagenesis techniques. Variant polypeptides may comprise conservative or non-conservative amino acid substitutions, deletions or additions.

The term "functional variant" refers polypeptides that have a substantially identical amino acid sequence to the naturally-occurring sequence, or are encoded by a substantially identical nucleotide sequence, and are capable of having one or more activities of the naturally-occurring sequence.

Derivatives of polypeptides are polypeptides which have been altered so as to exhibit additional features not found on the native polypeptide. Examples include fusion proteins.

A "conservative amino acid substitution" is one in which the amino acid residue is replaced with an amino acid residue having a similar side chain. Families of amino acid residues having similar side chains have been defined in the art. These families include amino acids with basic side chains (*e.g*., lysine, arginine, histidine), acidic side chains (*e.g.,* aspartic acid, glutamic acid), uncharged polar side chains (*e.g.,* glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.,* threonine, valine, isoleucine) and aromatic side chains (*e.g.,* tyrosine, phenylalanine, tryptophan, histidine).

Various aspects of the invention are described in further detail below. Additional definitions are set out throughout the specification.

### Reparative Agents

Methods, composition and kits described herein include a combination of a reparative agent (*e.g.,* a LINGO-1 antagonist) and an immunomodulatory agent. In one embodiment, the reparative agent is an antagonist of LRR and Ig domain-containing, Nogo receptor-interacting protein ("LINGO," *e.g.,* LINGO-1). For example, the LINGO-1 antagonist can inhibit or reduce the expression or activity of LINGO-1, *e.g.,* human LINGO-1. In one embodiment, the LINGO-1 antagonist inhibits or reduces the formation and/or activity of a complex (*e.g.,* a functional signaling complex) of the NgR1, p75, and LINGO-1; and/or NgR1, TAJ (TROY), and LINGO-1. In another embodiment, the LINGO-1 antagonist inhibits or reduces LINGO-1 binding to NgR1.

### LINGO-1 and LINGO-1 Antagonists

LINGO-1, previously called Sp35, is a cell surface glycoprotein that is selectively expressed in the adult CNS in neurons and oligodendrocytes. LINGO-1 is a member of a protein family comprising 3 other paralogs: LINGO-2 (GI: 12309630, 61% protein identity), LINGO-3 (GI: 23342615, 56% identity) and LINGO-4 (GI: 21211752, 44% identity). LINGO-1 is highly conserved evolutionarily with human and mouse orthologues sharing 99.5% identity. By Northern blot analysis, LINGO-1 was found to be highly expressed in human brain and was not detectable in non-neural tissues (Barrette et al. (2007) Mol Cell Neurosci, 34: 519-38; Carim-Todd et al. (2003) Eur Journal Neurosci, 18: 3167-82; Llorens et al. (2008) Dev Neurobiol, 68: 521-41; Mi et al. (2004) Nat Neurosci, 7: 221-8; Okafuji et al. (2005) Gene Expr Patterns, 6: 57-62; Park et al. (2006) Neurosci Lett, 404: 61-6; Shao et al. (2005) Neuron, 45: 353-9). LINGO-1 has also been described in detail in International Applications PCT/US2006/026271, filed Jul. 7, 2006, PCT/US2004/008323, filed Mar. 17, 2004, PCT/US2005/022881, filed Jun. 24, 2005 and PCT/US2008/000316, filed Jan. 9, 2008, each of which is incorporated by reference in its entirety herein.

LINGO-1 is selectively expressed in both oligodendrocyte precursor cells (OPCs) and neurons. LINGO-1 functions as a negative regulator of oligodendrocyte differentiation myelination, and remyelination; preventing myelination of axons by oligodendrocytes (Lee et al. (2007) J Neurosci, 27: 220-5; Mi et al. (2005) Nat Neurosci, 8: 745-51; Mi et al. (2008) Int Journal Biochem Cell Biol 40(10):1971-8; Mi et al. (2009) Ann Neurology, 65: 304-15). Axonal and neuronal expression of LINGO-1 increases after injury (Ji et al. (2006) Mol Cell Neurosci, 33: 311-20). LINGO-1 expression prevents myelination of axons by oligodendrocytes. Several preclinical studies have demonstrated the potential for LINGO-1 antagonism to enhance CNS remyelination and neuroaxonal protection in animal models of toxic (Cuprizone) (Mi et al. (2009) Ann Neurology, 65: 304-15), chemical injury (lysophosphatidylcholine [LPC]), and inflammatory (myelin oligodendrocyte glycoprotein- experimental autoimmune encephalomyelitis [MOG-EAE]) [Mi et al. (2007) Nat Med, 13: 1228-33) demyelination; and of toxic (1-methyl-4-phenyl-1,2,3,6-tetrahydropyridine [MPTP]) neuronal injury (Inoue et al. (2007) Proc Natl Acad Sci, 104: 14430-5), traumatic/hypertensive optic nerve injury (Fu et al. (2008) Invest Opthalmol Vis Sci, 49: 975-85) and spinal cord injury (Ji et al. (2006) Mol Cell Neurosci, 33: 311-20; Ji et al. (2008) Mol Cell Neurosci, 39: 258-67; Lv et al. (2010) Neuroimmunomodulat, 17: 270-8). Remyelination and neuroaxonal protection can be provided via blockade of signaling by myelin debris and/or sulfated proteoglycans on the NgR1 receptor complex in the CNS caused by the inhibition of LINGO-1 in axons and oligodendroyctes. This in turn may promote remylination via differentiation of oligodendrocyte precursor cells (OPCs) normally present in the brain of MS patients. Thus, antagonism of LINGO-1 can enhance myelination or re-myelination of axons, *e.g*., by oligodendrocytes, and enhance neuroaxonal protection in the CNS, and for example, in CNS demyelinating diseases such as multiple sclerosis (MS) and acute optic neuritis, leading to improved CNS repair.

LINGO-1 is also known in the art by the names LRRN6, LRRN6A, FLJ14594, LERN1, MGC17422 and UNQ201. The human, full-length wild-type LINGO-1 polypeptide contains an LRR domain consisting of 14 leucine-rich repeats (including N- and C-terminal caps), an Ig domain, a transmembrane region, and a cytoplasmic domain. The cytoplasmic domain contains a canonical tyrosine phosphorylation site. In addition, the naturally occurring LINGO-1 protein contains a signal sequence, a short basic region between the LRR-C-terminal domain (LRRCT) and Ig domain, and a transmembrane region between the Ig domain and the cytoplasmic domain. The human LINGO-1 gene (SEQ ID NO:52) contains alternative translation start codons, so that six additional amino acids, i.e., MQVSKR (SEQ ID NO:87) may or may not be present at the N-terminus of the LINGO-1 signal sequence. **Table 2** lists the LINGO-1 domains and other regions, according to amino acid residue number, based on the LINGO-1 amino acid sequence presented herein as SEQ ID NO: 51. The LINGO-1 polypeptide is characterized in more detail in PCT Publication No. WO 2004/085648, which is incorporated herein by reference in its entirety.

**TABLE 2**

| LINGO-1 Domains | | |
|---|---|---|
| Domain or Region | Beginning Residue | Ending Residue |
| Signal Sequence | 1 | 33 or 35 |
| LRRNT | 34 or 36 | 64 |
| LRR | 66 | 89 |
| LRR | 90 | 113 |
| LRR | 114 | 137 |
| LRR | 138 | 161 |
| LRR | 162 | 185 |
| LRR | 186 | 209 |
| LRR | 210 | 233 |
| LRR | 234 | 257 |
| LRR | 258 | 281 |
| LRR | 282 | 305 |
| LRR | 306 | 329 |
| LRR | 330 | 353 |
| LRRCT | 363 | 414 or 416 |
| Basic | 415 or 417 | 424 |
| Ig | 419 | 493 |
| Connecting sequence | 494 | 551 |
| Transmembrane | 552 | 576 |
| Cytoplasmic | 577 | 614 |

Tissue distribution and developmental expression of LINGO-1 has been studied in humans and rats. LINGO-1 biology has been studied in an experimental animal (rat) model. Expression of rat LINGO-1 is localized to neurons and oligodendrocytes, as determined by northern blot and immuno-histochemical staining. Rat LINGO-1 mRNA expression level is regulated developmentally, peaking shortly after birth, *i.e.,* ca. postnatal day one. In a rat spinal cord transection injury model, LINGO-1 is up-regulated at the injury site, as determined by RT-PCR. See Mi et al. Nature Neurosci. 7:221-228 (2004).

In the context of the amino acids comprising the various structural and functional domains of a LINGO-1 polypeptide, the term "about" includes the particularly recited value and values larger or smaller by several (e.g., 10, 9, 8, 7, 6, 5, 4, 3, 2, or 1) amino acids. Since the location of these domains as listed in **Table 2** have been predicted by computer graphics, one of ordinary skill would appreciate that the amino acid residues constituting the domains may vary slightly (*e.g*., by about 1 to 15 residues) depending on the criteria used to define the domain.

Full-length, wild-type LINGO-1 binds to NgR1. See PCT Publication No. WO 2004/085648. LINGO-1 is expressed in oligodendrocytes and that the LINGO-1 protein is involved in the regulation of oligodendrocyte-mediated myelination of axons. See U.S Patent Publication No. 2006/0009388 A1, which is incorporated herein by reference in its entirety.

The nucleotide sequence for the full-length LINGO-1 molecule is as follows:

ATGCTGGCGGGGGCGTGAGGAGCATGCCCAGCCCCCTCCTGGCCTGCTG
GCAGCCCATCCTCCTGCTGGTGCTGGGCTCAGTGCTGTCAGGCTCGGCCA
CGGGCTGCCCGCCCCGCTGCGAGTGCTCCGCCCAGGACCGCGCTGTGCTG
TGCCACCGCAAGCGCTTTGTGGCAGTCCCCGAGGGCATCCCCACCGAGAC
GCGCCTGCTGGACCTAGGCAAGAACCGCATCAAAACGCTCAACCAGGACG
AGTTCGCCAGCTTCCCGCACCTGGAGGAGCTGGAGCTCAACGAGAACATC
GTGAGCGCCGTGGAGCCCGGCGCCTTCAACAACCTCTTCAACCTCCGGAC
GCTGGGTCTCCGCAGCAACCGCCTGAAGCTCATCCCGCTAGGCGTCTTCA
CTGGCCTCAGCAACCTGACCAAGCTGGACATCAGCGAGAACAAGATTGTT
ATCCTGCTGGACTACATGTTTCAGGACCTGTACAACCTCAAGTCACTGGA
GGTTGGCGACAATGACCTCGTCTACATCTCTCACCGCGCCTTCAGCGGCC
TCAACAGCCTGGAGCAGCTGACGCTGGAGAAATGCAACCTGACCTCCATC
CCCACCGAGGCGCTGTCCCACCTGCACGGCCTCATCGTCCTGAGGCTCCG
GCACCTCAACATCAATGCCATCCGGGACTACTCCTTCAAGAGGCTCTACC
GACTCAAGGTCTTGGAGATCTCCCACTGGCCCTACTTGGACACCATGACA
CCCAACTGCCTCTACGGCCTCAACCTGACGTCCCTGTCCATCACACACTG
CAATCTGACCGCTGTGCCCTACCTGGCCGTCCGCCACCTAGTCTATCTCC
GCTTCCTCAACCTCTCCTACAACCCCATCAGCACCATTGAGGGCTCCATG
TTGCATGAGCTGCTCCGGCTGCAGGAGATCCAGCTGGTGGGCGGGCAGCT
GGCCGTGGTGGAGCCCTATGCCTTCCGCGGCCTCAACTACCTGCGCGTGC
TCAATGTCTCTGGCAACCAGCTGACCACACTGGAGGAATCAGTCTTCCAC
TCGGTGGGCAACCTGGAGACACTCATCCTGGACTCCAACCCGCTGGCCTG
CGACTGTCGGCTCCTGTGGGTGTTCCGGCGCCGCTGGCGGCTCAACTTCA
ACCGGCAGCAGCCCACGTGCGCCACGCCCGAGTTTGTCCAGGGCAAGGAG
TTCAAGGACTTCCCTGATGTGCTACTGCCCAACTACTTCACCTGCCGCCG
CGCCCGCATCCGGGACCGCAAGGCCCAGCAGGTGTTTGTGGACGAGGGCC

ACACGGTGCAGTTTGTGTGCCGGGCCGATGGCGACCCGCCGCCCGCCATC
CTCTGGCTCTCACCCCGAAAGCACCTGGTCTCAGCCAAGAGCAATGGGCG
GCTCACAGTCTTCCCTGATGGCACGCTGGAGGTGCGCTACGCCCAGGTAC
AGGACAACGGCACGTACCTGTGCATCGCGGCCAACGCGGGCGGCAACGAC
TCCATGCCCGCCCACCTGCATGTGCGCAGCTACTCGCCCGACTGGCCCCA
TCAGCCCAACAAGACCTTCGCTTTCATCTCCAACCAGCCGGGCGAGGGAG
AGGCCAACAGCACCCGCGCCACTGTGCCTTTCCCCTTCGACATCAAGACC
CTCATCATCGCCACCACCATGGGCTTCATCTCTTTCCTGGGCGTCGTCCT
CTTCTGCCTGGTGCTGCTGTTTCTCTGGAGCCGGGGCAAGGGCAACACAA
AGCACAACATCGAGATCGAGTATGTGCCCCGAAAGTCGGACGCAGGCATC
AGCTCCGCCGACGCGCCCCGCAAGTTCAACATGAAGATGATATGA. (SEQ ID NO: 52)

The polypeptide sequence for the full-length LINGO-1 polypeptide is as follows:

MLAGGVRSMPSPLLACWQPILLLVLGSVLSGSATGCPPRCECSAQDRAVL
CHRKRFVAVPEGIPTETRLLDLGKNRIKTLNQDEFASFPHLEELELNENI
VSAVEPGAFNNLFNLRTLGLRSNRLKLIPLGVFTGLSNLTKLDISENKIV
ILLDYMFQDLYNLKSLEVGDNDLVYISHRAFSGLNSLEQLTLEKCNLTSI
PTEALSHLHGLIVLRLRHLNINAIRDYSFKRLYRLKVLEISHWPYLDTMT
PNCLYGLNLTSLSITHCNLTAVPYLAVRHLVYLRFLNLSYNPISTIEGSM
LHELLRLQEIQLVGGQLAVVEPYAFRGLNYLRVLNVSGNQLTTLEESVFH
SVGNLETLILDSNPLACDCRLLWVFRRRWRLNFNRQQPTCATPEFVQGKE
FKDFPDVLLPNYFTCRRARIRDRKAQQVFVDEGHTVQFVCRADGDPPPAI
LWLSPRKHLVSAKSNGRLTVFPDGTLEVRYAQVQDNGTYLCIAANAGGND
SMPAHLHVRSYSPDWPHQPNKTFAFISNQPGEGEANSTRATVPFPFDIKT
LIIATTMGFISFLGVVLFCLVLLFLWSRGKGNTKHNIEIEYVPRKSDAGI
SSADAPRKFNMKMI. (SEQ ID NO: 51)

### Anti-LINGO-1 Antibody Molecules

In certain embodiments the antibody molecule binds to LINGO, e.g., human LINGO. In another embodiment, the antibody molecule binds to LINGO-1, e.g., human LINGO-1. In one embodiment, the antibody molecule is isolated, purified or recombinant. By an "isolated" polypeptide or a fragment, variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for purposed of the invention, as are native or recombinant polypeptides which have been separated, fractionated, or partially or substantially purified by any suitable technique.

As used herein, the term "antibody molecule" refers to a protein comprising at least one immunoglobulin variable domain sequence. The term antibody molecule includes, for example, full-length antibodies, mature antibodies, fragments, *e.g*., antigen-binding fragments of an antibody, derivatives, analogs, or variants of the antibodies disclosed herein, and any combination thereof.

The terms "fragment," "variant," "derivative" and "analog" when referring to LINGO-1 antibody molecules or antibody polypeptides include any polypeptides which retain at least some of the antigen-binding properties of the corresponding native antibody or polypeptide. Fragments of polypeptides include proteolytic fragments, as well as deletion fragments, in addition to specific antibody fragments discussed elsewhere herein. Variants of LINGO-1 antibody and antibody polypeptides include fragments as described above, and also polypeptides with altered amino acid sequences due to amino acid substitutions, deletions, or insertions. Variants may occur naturally or be non-naturally occurring. Non-naturally occurring variants may be produced using art-known mutagenesis techniques. Variant polypeptides may comprise conservative or non-conservative amino acid substitutions, deletions or additions. Derivatives of LINGO-1 antibody molecules and antibody polypeptides are polypeptides which have been altered so as to exhibit additional features not found on the native polypeptide. Examples include fusion proteins.

As used herein a "derivative" of a LINGO-1 antibody molecule or antibody polypeptide refers to a subject polypeptide having one or more residues chemically derivatized by reaction of a functional side group. Also included as "derivatives" are those peptides which contain one or more naturally occurring amino acid derivatives of the twenty standard amino acids. For example, 4-hydroxyproline may be substituted for proline; 5-hydroxylysine may be substituted for lysine; 3-methylhistidine may be substituted for histidine; homoserine may be substituted for serine; and ornithine may be substituted for lysine.

For example, an antibody molecule can include a heavy (H) chain variable domain sequence (abbreviated herein as VH), and a light (L) chain variable domain sequence (abbreviated herein as VL). In another example, an antibody molecule includes two heavy (H) chain variable domain sequences and two light (L) chain variable domain sequence, thereby forming two antigen binding sites, such as Fab, Fab', F(ab')₂, Fc, Fd, Fd', Fv, single chain antibodies (scFv for example), single variable domain antibodies, diabodies (Dab) (bivalent and bispecific), and chimeric (*e.g*., humanized) antibodies, which may be produced by the modification of whole antibodies or those synthesized de novo using recombinant DNA technologies. These functional antibody fragments retain the ability to selectively bind with their respective antigen or receptor. Antibodies and antibody fragments can be from any class of antibodies including, but not limited to, IgG, IgA, IgM, IgD, and IgE, and from any subclass (*e.g.,* IgG1, IgG2, IgG3, and IgG4) of antibodies. The antibody molecules can be monoclonal or polyclonal. The antibody can also be a human, humanized, CDR-grafted, or *in vitro* generated antibody. The antibody can have a heavy chain constant region chosen from, *e.g.,* IgG1, IgG2, IgG3, or IgG4. The antibody can also have a light chain chosen from, *e.g.,* kappa or lambda.

Examples of antigen-binding fragments include: (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a diabody (dAb) fragment, which consists of a VH domain; (vi) a camelid or camelized variable domain; (vii) a single chain Fv (scFv), see *e.g.,* Bird et al. (1988) Science 242:423-426; and Huston et al. (1988) Proc. Natl. Acad. Sci. USA 85:5879-5883); (viii) a single domain antibody. These antibody fragments are obtained using conventional techniques known to those with skill in the art, and the fragments are screened for utility in the same manner as are intact antibodies.

Antibody molecules can also be single domain antibodies. Single domain antibodies can include antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, fish, shark, goat, rabbit, and bovine. In one aspect of the invention, a single domain antibody can be derived from a variable region of the immunoglobulin found in fish, such as, for example, that which is derived from the immunoglobulin isotype known as Novel Antigen Receptor (NAR) found in the serum of shark. Methods of producing single domain antibodies derivied from a variable region of NAR ("IgNARs") are described in WO 03/014161 and Streltsov (2005) Protein Sci. 14:2901-2909. According to another aspect of the invention, a single domain antibody is a naturally occurring single domain antibody known as heavy chain antibody devoid of light chains. Such single domain antibodies are disclosed in WO 9404678, for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in Camelidae species, for example in camel, llama, dromedary, alpaca and guanaco. Other species besides Camelidae may produce heavy chain antibodies naturally devoid of light chain; such VHHs are within the scope of the invention.

The VH and VL regions can be subdivided into regions of hypervariability, termed "complementarity determining regions" (CDR), interspersed with regions that are more conserved, termed "framework regions" (FR). The extent of the framework region and CDRs has been precisely defined by a number of methods (see, Kabat, E. A., et al. (1991) Sequences of Proteins of Immunological Interest, Fifth Edition, U.S. Department of Health and Human Services, NIH Publication No. 91-3242; Chothia, C. et al. (1987) J. Mol. Biol. 196:901-917; and the AbM definition used by Oxford Molecular's AbM antibody modelling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Generally, unless specifically indicated, the following definitions are used: AbM definition of CDR1 of the heavy chain variable domain and Kabat definitions for the other CDRs. In addition, embodiments of the invention described with respect to Kabat or AbM CDRs may also be implemented using Chothia hypervariable loops. Each VH and VL typically includes three CDRs and four FRs, arranged from amino-terminus to carboxy-terminus in the following order: FR1, CDR1, FR2, CDR2, FR3, CDR3, FR4.

As used herein, an "immunoglobulin variable domain sequence" refers to an amino acid sequence which can form the structure of an immunoglobulin variable domain. For example, the sequence may include all or part of the amino acid sequence of a naturally-occurring variable domain. For example, the sequence may or may not include one, two, or more N- or C-terminal amino acids, or may include other alterations that are compatible with formation of the protein structure.

The term "antigen-binding site" refers to the part of an antibody molecule that comprises determinants that form an interface that binds to LINGO-1, or an epitope thereof. With respect to proteins (or protein mimetics), the antigen-binding site typically includes one or more loops (of at least four amino acids or amino acid mimics) that form an interface that binds to LINGO-1. Typically, the antigen-binding site of an antibody molecule includes at least one or two CDRs, or more typically at least three, four, five or six CDRs.

The terms "monoclonal antibody" or "monoclonal antibody composition" as used herein refer to a preparation of antibody molecules of single molecular composition. A monoclonal antibody composition displays a single binding specificity and affinity for a particular epitope. A monoclonal antibody can be made by hybridoma technology or by methods that do not use hybridoma technology (e.g., recombinant methods).

An "effectively human" protein is a protein that does not evoke a neutralizing antibody response, e.g., the human anti-murine antibody (HAMA) response. HAMA can be problematic in a number of circumstances, e.g., if the antibody molecule is administered repeatedly, e.g., in treatment of a chronic or recurrent disease condition. A HAMA response can make repeated antibody administration potentially ineffective because of an increased antibody clearance from the serum (*see, e.g.,* Saleh et al., Cancer Immunol. Immunother., 32:180-190 (1990)) and also because of potential allergic reactions (*see, e.g.,* LoBuglio et al., Hybridoma, 5:5117-5123 (1986)).

In certain embodiments, the antibody molecule can be a monoclonal or single specificity antibody, or an antigen-binding fragment thereof (*e.g.,* an Fab, F(ab')₂, Fv, a single chain Fv fragment, a single domain antibody, a diabody (dAb), a bivalent or bispecific antibody or fragment thereof, a single domain variant thereof) that binds to LINGO-1, *e.g.,* a mammalian (*e.g.,* human LINGO-1 (or a functional variant thereof)). In one embodiment, the antibody molecule is a monoclonal antibody against LINGO-1, *e.g*., human LINGO-1. Typically, the antibody molecule is a human, humanized, a CDR-grafted, chimeric, camelid, or *in vitro* generated antibody to human LINGO-1 (or functional fragment thereof, *e.g.,* an antibody fragment as described herein). Typically, the antibody inhibits, reduces or neutralizes one or more activities of LINGO-1 *(e.g.,* one or more biological activities of LINGO-1 as described herein).

In certain embodiments, the antibody molecule specifically binds to the same, or substantially the same, LINGO-1 epitope as the reference monoclonal antibody Li62 or Li81, described in U.S. Patent No. 8,058,406, incorporated by reference in its entirety herein. Exemplary anti-LINGO-1 antibody molecules are described in U.S. Patent No. 8,058,406.

In one embodiment, antibody molecule includes at least the antigen-binding domains of Li62, Li81. As used herein, the term "antigen binding domain" includes a site that specifically binds an epitope on an antigen (e.g., an epitope of LINGO-1). The antigen binding domain of an antibody typically includes at least a portion of an immunoglobulin heavy chain variable region and at least a portion of an immunoglobulin light chain variable region. The binding site formed by these variable regions determines the specificity of the antibody.

In other embodiments, the anti- LINGO-1 antibody molecule competitively inhibits Li62 or Li81 from binding to LINGO-1.

In certain embodiments, the anti- LINGO-1 antibody molecule specifically or preferentially binds to a particular LINGO-1 polypeptide fragment or domain. Such LINGO-1 polypeptide fragments include, but are not limited to, a LINGO-1 polypeptide comprising, consisting essentially of, or consisting of amino acids 34 to 532; 34 to 417; 34 to 425; 34 to 493; 66 to 532; 66 to 417; 66 to 426; 66 to 493; 66 to 532; 417 to 532; 417 to 425 (the LINGO-1 basic region); 417 to 493; 417 to 532; 419 to 493 (the LINGO-11 g region); or 425 to 532 of SEQ ID NO:51; or a LINGO-1 variant polypeptide at least 70%, 75%, 80%, 85%, 90%, or 95% identical to amino acids 34 to 532; 34 to 417; 34 to 425; 34 to 493; 66 to 532; 66 to 417; 66 to 426; 66 to 493; 66 to 532; 417 to 532; 417 to 425 (the LINGO-1 basic region); 417 to 493; 417 to 532; 419 to 493 (the LINGO-11 g region); or 425 to 532 of SEQ ID NO:51.

In certain embodiments, the anti- LINGO-1 antibody molecule specifically or preferentially binds to a LINGO-1 peptide fragment comprising, consisting essentially of, or consisting of one or more leucine-rich-repeats (LRR) of LINGO-1. Such fragments, include, for example, fragments comprising, consisting essentially of, or consisting of amino acids 66 to 89; 66 to 113; 66 to 137; 90 to 113; 114 to 137; 138 to 161; 162 to 185; 186 to 209; 210 to 233; 234 to 257; 258 to 281; 282 to 305; 306 to 329; or 330 to 353 of SEQ ID NO:51. Corresponding fragments of a variant LINGO-1 polypeptide at least 70%, 75%, 80%, 85%, 90%, or 95% identical to amino acids 66 to 89; 66 to 113; 90 to 113; 114 to 137; 138 to 161; 162 to 185; 186 to 209; 210 to 233; 234 to 257; 258 to 281; 282 to 305; 306 to 329; or 330 to 353 of SEQ ID NO:51 are also contemplated.

In certain embodiments, the anti- LINGO-1 antibody molecule specifically or preferentially binds to a fragment comprising, consisting essentially of, or consisting of one or more cysteine rich regions flanking the LRR of LINGO-1. Such fragments, include, for example, a fragment comprising, consisting essentially of, or consisting of amino acids 34 to 64 of SEQ ID NO:51 (the N-terminal LRR flanking region (LRRNT)), or a fragment comprising, consisting essentially of, or consisting of amino acids 363 to 416 of SEQ ID NO:51 (the C-terminal LRR flanking region (LRRCT)), amino acids Corresponding fragments of a variant LINGO-1 polypeptide at least 70%, 75%, 80%, 85%, 90%, or 95% identical to amino acids 34 to 64 and 363 to 416 of SEQ ID NO:51 are also contemplated.

In certain embodiments, the anti- LINGO-1 antibody molecule specifically or preferentially binds to a fragment comprising, consisting essentially of, or consisting of amino acids 41 to 525 of SEQ ID NO:51; 40 to 526 of SEQ ID NO:51; 39 to 527 of SEQ ID NO:51; 38 to 528 of SEQ ID NO:51; 37 to 529 of SEQ ID NO:51; 36 to 530 of SEQ ID NO:51; 35 to 531 of SEQ ID NO:51; 34 to 531 of SEQ ID NO:51; 46 to 520 of SEQ ID NO:51; 45 to 521 of SEQ ID NO:51; 44 to 522 of SEQ ID NO:51; 43 to 523 of SEQ ID NO:51; and 42 to 524 of SEQ ID NO:51.

In certain embodiments, the anti- LINGO-1 antibody molecule specifically or preferentially binds to a fragment comprising, consisting essentially of, or consisting of amino acids 1 to 33 of SEQ ID NO:51; 1 to 35 of SEQ ID NO:51; 34 to 64 of SEQ ID NO:51; 36 to 64 of SEQ ID NO:51; 66 to 89 of SEQ ID NO:51; 90 to 113 of SEQ ID NO:51; 114 to 137 of SEQ ID NO:51; 138 to 161 of SEQ ID NO:51; 162 to 185 of SEQ ID NO:51; 186 to 209 of SEQ ID NO:51; 210 to 233 of SEQ ID NO:51; 234 to 257 of SEQ ID NO:51; 258 to 281 of SEQ ID NO:51; 282 to 305 of SEQ ID NO:51; 306 to 329 of SEQ ID NO:51; 330 to 353 of SEQ ID NO:51; 363 to 416 of SEQ ID NO:51; 417 to 424 of SEQ ID NO:51; 419 to 493 of SEQ ID NO:51; and 494 to 551 of SEQ ID NO:51.

In certain embodiments, the anti- LINGO-1 antibody molecule specifically or preferentially binds to a fragment comprising, consisting essentially of, or consisting of amino acids 1 to 33 of SEQ ID NO:51; 1 to 35 of SEQ ID NO:51; 1 to 64 of SEQ ID NO:51; 1 to 89 of SEQ ID NO:51; 1 to 113 of SEQ ID NO:51; 1 to 137 of SEQ ID NO:51; 1 to 161 of SEQ ID NO:51; 1 to 185 of SEQ ID NO:51; 1 to 209 of SEQ ID NO:51; 1 to 233 of SEQ ID NO:51; 1 to 257 of SEQ ID NO:51; 1 to 281 of SEQ ID NO:51; 1 to 305 of SEQ ID NO:51; 1 to 329 of SEQ ID NO:51; 1 to 353 of SEQ ID NO:51; 1 to 416 of SEQ ID NO:51; 1 to 424 of SEQ ID NO:51; 1 to 493 of SEQ ID NO:51; 1 to 551 of SEQ ID NO:51; 1 to 531 of SEQ ID NO:51 and 1 to 532 of SEQ ID NO:51.

In certain embodiments, the anti- LINGO-1 antibody molecule specifically or preferentially binds to a fragment comprising, consisting essentially of, or consisting of amino acids 34 to 64 of SEQ ID NO:51; 34 to 89 of SEQ ID NO:51; 34 to 113 of SEQ ID NO:51; 34 to 137 of SEQ ID NO:51; 34 to 161 of SEQ ID NO:51; 34 to 185 of SEQ ID NO:51; 34 to 209 of SEQ ID NO:51; 34 to 233 of SEQ ID NO:51; 34 to 257 of SEQ ID NO:51; 34 to 281 of SEQ ID NO:51; 34 to 305 of SEQ ID NO:51; 34 to 329 of SEQ ID NO:51; 34 to 353 of SEQ ID NO:51; 34 to 416 of SEQ ID NO:51; 34 to 424 of SEQ ID NO:51; 34 to 493 of SEQ ID NO:51; and 34 to 551 of SEQ ID NO:51.

In certain embodiments, the anti- LINGO-1 antibody molecule specifically or preferentially binds to a fragment comprising, consisting essentially of, or consisting of amino acids 34 to 530 of SEQ ID NO:51; 34 to 531 of SEQ ID NO:51; 34 to 532 of SEQ ID NO:51; 34 to 533 of SEQ ID NO:51; 34 to 534 of SEQ ID NO:51; 34 to 535 of SEQ ID NO:51; 34 to 536 of SEQ ID NO:51; 34 to 537 of SEQ ID NO:51; 34 to 538 of SEQ ID NO:51; 34 to 539 of SEQ ID NO:51; 30 to 532 of SEQ ID NO:51; 31 to 532 of SEQ ID NO:51; 32 to 532 of SEQ ID NO:51; 33 to 532 of SEQ ID NO:51; 34 to 532 of SEQ ID NO:51; 35 to 532 of SEQ ID NO:51; 36 to 532 of SEQ ID NO:51; 30 to 531 of SEQ ID NO:51; 31 to 531 of SEQ ID NO:51; 32 to 531 of SEQ ID NO:51; 33 to 531 of SEQ ID NO:51; 34 to 531 of SEQ ID NO:51; 35 to 531 of SEQ ID NO:51; and 36 to 531 of SEQ ID NO:51.

In certain embodiments, the anti- LINGO-1 antibody molecule specifically or preferentially binds to a fragment comprising, consisting essentially of, or consisting of amino acids 36 to 64 of SEQ ID NO:51; 36 to 89 of SEQ ID NO:51; 36 to 113 of SEQ ID NO:51; 36 to 137 of SEQ ID NO:51; 36 to 161 of SEQ ID NO:51; 36 to 185 of SEQ ID NO:51; 36 to 209 of SEQ ID NO:51; 36 to 233 of SEQ ID NO:51; 36 to 257 of SEQ ID NO:51; 36 to 281 of SEQ ID NO:51; 36 to 305 of SEQ ID NO:51; 36 to 329 of SEQ ID NO:51; 36 to 353 of SEQ ID NO:51; 36 to 416 of SEQ ID NO:51; 36 to 424 of SEQ ID NO:51; 36 to 493 of SEQ ID NO:51; and 36 to 551 of SEQ ID NO:51.

In certain embodiments, the anti- LINGO-1 antibody molecule specifically or preferentially binds to a fragments comprising, consisting essentially of, or consisting of amino acids 36 to 530 of SEQ ID NO:51; 36 to 531 of SEQ ID NO:51; 36 to 532 of SEQ ID NO:51; 36 to 533 of SEQ ID NO:51; 36 to 534 of SEQ ID NO:51; 36 to 535 of SEQ ID NO:51; 36 to 536 of SEQ ID NO:51; 36 to 537 of SEQ ID NO:51; 36 to 538 of SEQ ID NO:51; and 36 to 539 of SEQ ID NO:51.

In certain embodiments, the anti- LINGO-1 antibody molecule specifically or preferentially binds to a fragment comprising, consisting essentially of, or consisting of amino acids 417 to 493 of SEQ ID NO:51; 417 to 494 of SEQ ID NO:51; 417 to 495 of SEQ ID NO:51; 417 to 496 of SEQ ID NO:51; 417 to 497 of SEQ ID NO:51; 417 to 498 of SEQ ID NO:51; 417 to 499 of SEQ ID NO:51; 417 to 500 of SEQ ID NO:51; 417 to 492 of SEQ ID NO:51; 417 to 491 of SEQ ID NO:51; 412 to 493 of SEQ ID NO:51; 413 to 493 of SEQ ID NO:51; 414 to 493 of SEQ ID NO:51; 415 to 493 of SEQ ID NO:51; 416 to 493 of SEQ ID NO:51; 411 to 493 of SEQ ID NO:51; 410 to 493 of SEQ ID NO:51; 410 to 494 of SEQ ID NO:51; 411 to 494 of SEQ ID NO:51; 412 to 494 of SEQ ID NO:51; 413 to 494 of SEQ ID NO:51; 414 to 494 of SEQ ID NO:51; 415 to 494 of SEQ ID NO:51; 416 to 494 of SEQ ID NO:51; 417 to 494 of SEQ ID NO:51; and 418 to 494 of SEQ ID NO:51.

In certain embodiments, the anti- LINGO-1 antibody molecule specifically or preferentially binds to a LINGO-1 polypeptide comprising, consisting essentially of, or consisting of peptides of the Ig domain of LINGO-1 or fragments, variants, or derivatives of such polypeptides. Specifically, polypeptides comprising, consisting essentially of, or consisting of the following polypeptide sequences: ITX₁X₂X₃ (SEQ ID NO:88), ACX₁X₂X₃ (SEQ ID NO:89), VCX₁X₂X₃ (SEQ ID NO:90) and SPX₁X₂X₃ (SEQ ID NO:91) where X₁ is lysine, arginine, histidine, glutamine, or asparagine, X₂ is lysine, arginine, histidine, glutamine, or asparagine and X₃ is lysine, arginine, histidine, glutamine, or asparagine. For example, LINGO-1 peptide fragments to which certain antibody molecules can bind include, those fragments comprising, consisting essentially of, or consisting of the following polypeptide sequences: SPRKH (SEQ ID NO:92), SPRKK (SEQ ID NO:93), SPRKR (SEQ ID NO:94), SPKKH (SEQ ID NO:95), SPHKH (SEQ ID NO:96), SPRRH (SEQ ID NO:97), SPRHH (SEQ ID NO:98), SPRRR (SEQ ID NO:99), SPHHH (SEQ ID NO:100) SPKKK (SEQ ID NO:101), LSPRKH (SEQ ID NO:102), LSPRKK (SEQ ID NO:103), LSPRKR (SEQ ID NO:104), LSPKKH (SEQ ID NO:105), LSPHKH (SEQ ID NO:106), LSPRRH (SEQ ID NO:107), LSPRHH (SEQ ID NO:108), LSPRRR (SEQ ID NO:109), LSPHHH (SEQ ID NO:110) LSPKKK (SEQ ID NO:111), WLSPRKH (SEQ ID NO:112), WLSPRKK (SEQ ID NO:113), WLSPRKR (SEQ ID NO:114), WLSPKKH (SEQ ID NO:115), WLSPHKH (SEQ ID NO:116), WLSPRRH (SEQ ID NO:117), WLSPRHH (SEQ ID NO:118), WLSPRRR (SEQ ID NO:119), WLSPHHH (SEQ ID NO:120) WLSPKKK (SEQ ID NO:121). These LINGO-1 polypeptides include the basic "RKH loop" (Arginine-Lysine-Histidine amino acids 456-458) in the Ig domain of LINGO-1. Additional LINGO-1 peptides which include a basic tripeptide are ITPKRR (SEQ ID NO:122), ACHHK (SEQ ID NO:123) and VCHHK (SEQ ID NO:124).

In certain embodiments, the anti- LINGO-1 antibody molecule specifically or preferentially binds to a LINGO-1 polypeptide comprising, consisting essentially of, or consisting of peptides of the Ig domain of LINGO-1 or fragments, variants, or derivatives of such polypeptides. Specifically, peptides comprising, consisting essentially of, or consisting of the following polypeptide sequences: X₄X₅RKH (SEQ ID NO:125), X₄X₅RRR (SEQ ID NO:126), X₄X₅KKK (SEQ ID NO:127), X₄X₅HHH (SEQ ID NO:128), X₄X₅RKK (SEQ ID NO:129), X₄X₅RKR (SEQ ID NO:130), X₄X₅KKH (SEQ ID NO:131), X₄X₅HKH (SEQ ID NO:132), X₄X₅RRH (SEQ ID NO:133) and X₄X₅RHH (SEQ ID NO:134) where X₄ is any amino acid and X₅ is any amino acid.

In certain embodiments, the anti- LINGO-1 antibody molecule specifically or preferentially binds to a LINGO-1 polypeptide comprising, consisting essentially of, or consisting of peptides of the Ig domain of LINGO-1 or fragments, variants, or derivatives of such polypeptides. Specifically, polypeptides comprising, consisting essentially of, or consisting of the following polypeptide sequences: ITX₆X₇X₈ (SEQ ID NO:135), ACX₆X₇X₈ (SEQ ID NO:136), VCX₆X₇X₈ (SEQ ID NO:137) and SPX₆X₇X₈ (SEQ ID NO:where X₆ is lysine, arginine, histidine, glutamine, or asparagine, X₇ is any amino acid and X₈ is lysine, arginine, histidine, glutamine, or asparagine. For example, a polypeptide comprising, consisting essentially of, or consisting of the following polypeptide sequence: SPRLH (SEQ ID NO:139).

In certain embodiments, the anti- LINGO-1 antibody molecule specifically or preferentially binds to a LINGO-1 polypeptide comprising, consisting essentially of, or consisting of peptides which contain amino acids 452-458 in the Ig domain of LINGO-1, or derivatives thereof, wherein amino acid 452 is a tryptophan or phenylalanine residue.

In certain embodiments, the anti-LINGO-1 antibody molecule specifically or preferentially binds to a LINGO-1 polypeptide comprising, consisting essentially of, or consisting of peptides of the basic domain of LINGO-1. Specifically, peptides comprising, consisting essentially of, or consisting of the following polypeptide sequences: RRARIRDRK (SEQ ID NO:140), KKVKVKEKR (SEQ ID NO:141), RRLRLRDRK (SEQ ID NO:142), RRGRGRDRK (SEQ ID NO:143) and RRIRARDRK (SEQ ID NO:144).

Additional exemplary soluble LINGO-1 polypeptides and methods and materials for obtaining these molecules for producing antibodies or antibody fragments of the present invention may be found, e.g., in International Patent Application No. PCT/US2004/008323, incorporated herein by reference in its entirety.

Methods of making antibodies are known in the art and described herein. Once antibodies to various fragments of, or to the full-length LINGO-1 without the signal sequence, have been produced, determining which amino acids, or epitope, of LINGO-1 to which the antibody or antigen binding fragment binds can be determined by epitope mapping protocols as described herein as well as methods known in the art (e.g. double antibody-sandwich ELISA as described in "Chapter 11--Immunology," Current Protocols in Molecular Biology, Ed. Ausubel et al., v.2, John Wiley & Sons, Inc. (1996)). Additional epitope mapping protocols may be found in Morris, G. Epitope Mapping Protocols, New Jersey: Humana Press (1996), which are both incorporated herein by reference in their entireties. Epitope mapping can also be performed by commercially available means (i.e. ProtoPROBE, Inc. (Milwaukee, Wis.)).

Additionally, antibodies produced which bind to any portion of LINGO-1 can then be screened for their ability to act as an antagonist of LINGO-1 and thus promote neurite outgrowth, neuronal and oligodendrocyte survival, proliferation and differentiation as well as promote myelination. Antibodies can be screened for oligodendrocyte/neuronal survival for example by using the methods described herein such as in Examples 11 or 12 or as described in PCT/US2008/000316, filed Jan. 9, 2008, and PCT/US2006/026271, filed Jul. 7, 2006, which are incorporated herein by reference in their entireties. Additionally, antibodies can be screened for example by their ability to promote myelination by using the methods described herein such as in Examples 2, 6, 9, 10, 11 or 13 or as described in PCT/US2008/000316 and/or PCT/US2006/026271. Finally, antibodies can be screened for their ability to promote oligodendrocyte proliferation and differentiation, as well as neurite outgrowth for example by using the methods described herein such as in Examples 4 or 5 or as described in PCT/US2008/000316 and/or PCT/US2006/026271. Other antagonist functions of antibodies of the present invention can be tested using other assays as described in the Examples of US 8,058,406, incorporated by reference herein.

In certain embodiments, the anti-LINGO-1 antibody molecule specifically or preferentially binds to at least one epitope of LINGO-1, where the epitope comprises, consists essentially of, or consists of at least about four to five amino acids of SEQ ID NO:5, at least seven, at least nine, or between at least about 15 to about 30 amino acids of SEQ ID NO:5. The amino acids of a given epitope of SEQ ID NO:51 as described may be, but need not be contiguous or linear. In certain embodiments, the at least one epitope of LINGO-1 comprises, consists essentially of, or consists of a non-linear epitope formed by the extracellular domain of LINGO-1 as expressed on the surface of a cell or as a soluble fragment, *e.g*., fused to an IgG Fc region. Thus, in certain embodiments the at least one epitope of LINGO-1 comprises, consists essentially of, or consists of at least 4, at least 5, at least 6, at least 7, at least 8, at least 9, at least 10, at least 15, at least 20, at least 25, between about 15 to about 30, or at least 10, 15, 20, 25, 30, 35, 40, 45, 50, 55, 60, 65, 70, 75, 80, 85, 90, 95, or 100 contiguous or non-contiguous amino acids of SEQ ID NO:51, where the non-contiguous amino acids form an epitope through protein folding.

In other embodiments, the anti-LINGO-1 antibody molecule specifically or preferentially binds to at least one epitope of LINGO-1, where the epitope comprises, consists essentially of, or consists of, in addition to one, two, three, four, five, six or more contiguous or non-contiguous amino acids of SEQ ID NO:51 as described above, and an additional moiety which modifies the protein, e.g., a carbohydrate moiety may be included such that the LINGO-1 antibody binds with higher affinity to modified target protein than it does to an unmodified version of the protein. Alternatively, the LINGO-1 antibody does not bind the unmodified version of the target protein at all.

In certain embodiments, the anti-LINGO-1 antibody molecule specifically or preferentially binds to a LINGO-1 polypeptide or fragment thereof, or a LINGO-1 variant polypeptide, with an affinity characterized by a dissociation constant (K_{D}) which is less than the K_{D} for said reference monoclonal antibody.

In certain embodiments, the anti-LINGO-1 antibody molecule specifically or preferentially binds to at least one epitope of LINGO-1 or fragment or variant described above, *i.e*., binds to such an epitope more readily than it would bind to an unrelated, or random epitope; binds preferentially to at least one epitope of LINGO-1 or fragment or variant described above, *i.e*., binds to such an epitope more readily than it would bind to a related, similar, homologous, or analogous epitope; competitively inhibits binding of a reference antibody which itself binds specifically or preferentially to a certain epitope of LINGO-1 or fragment or variant described above; or binds to at least one epitope of LINGO-1 or fragment or variant described above with an affinity characterized by a dissociation constant K_{D} of less than about 5 x 10⁻² M, about 10⁻² M, about 5 x 10⁻³ M, about 10⁻³ M, about 5 x 10⁻⁴ M, about 10⁻⁴ M, about 5 x 10⁻⁵ M, about 10⁻⁵ M, about 5 x 10⁻⁶ M, about 10⁻⁶ M, about 5 x 10⁻⁷ M, about 10⁻⁷ M, about 5 x 10⁻⁸ M, about 10⁻⁸ M, about 5 x 10⁻⁹ M, about 10⁻⁹ M, about 5 x 10⁻¹⁰ M, about 10⁻¹⁰¹ M, about 5 x 10⁻¹¹ M, about 10⁻¹¹ M, about 5 x 10⁻¹² M, about 10⁻¹² M, about 5 x 10⁻¹³ M, about 10⁻¹³ M, about 5 x 10⁻¹⁴ M, about 10⁻¹⁴ M, about 5 x 10⁻¹⁵ M, about 10⁻¹⁵ M. In a particular aspect, the antibody or fragment thereof preferentially binds to a human LINGO-1 polypeptide or fragment thereof, relative to a murine LINGO-1 polypeptide or fragment thereof.

In other embodiments, the anti-LINGO-1 antibody molecule binds LINGO-1 polypeptides or fragments or variants thereof with an off rate (k(off)) of less than or equal to 5 x 10⁻² sec⁻¹, 10⁻² sec⁻¹, 5 x 10⁻³ sec⁻¹ or 10⁻³ sec⁻¹. Alternatively, an antibody, or antigen-binding fragment, variant, or derivative thereof of the invention binds LINGO-1 polypeptides or fragments or variants thereof with an off rate (k(off)) of less than or equal to 5 x 10⁻⁴ sec¹, 10⁻⁴ sec¹, 5 x 10⁻⁵ sec¹, or 10⁻⁵ sec¹, 5 x 10⁻⁶ sec¹, 10⁻⁶ sec¹, 5 x 10⁻⁷ sec¹ or 10⁻⁷ sec¹.

In other embodiments, the anti-LINGO-1 antibody molecule binds LINGO-1 polypeptides or fragments or variants thereof with an on rate (k(on)) of greater than or equal to 10³ M⁻¹ sec⁻¹, 5 x 10³ M⁻¹ sec⁻¹, 10⁴ M⁻¹ sec⁻¹, 5 x 10⁴ M⁻¹ sec⁻¹. Alternatively, the antibody molecule binds LINGO-1 polypeptides or fragments or variants thereof with an on rate (k(on)) greater than or equal to 10⁵ M⁻¹ sec⁻¹, 5 x 10⁵ M⁻¹ sec⁻¹, 10⁶ M⁻¹ sec⁻¹, 5 x 10⁶ M⁻¹ sec⁻¹, or 10⁷ M⁻¹ sec⁻¹, 5 x 10⁷ M⁻¹ sec⁻¹.

In other embodiments, the LINGO-1 antibody molecule is an antagonist of LINGO-1 activity. In certain embodiments, for example, binding of an antagonist LINGO-1 antibody to LINGO-1, as expressed on neurons, blocks myelin-associated neurite outgrowth inhibition or neuronal cell death. In other embodiments, binding of the LINGO-1 antibody to LINGO-1, as expressed on oligodendrocytes, blocks inhibition of oligodendrocyte growth or differentiation, or blocks demyelination or dysmyelination of CNS neurons.

Modified forms of LINGO-1 antibody molecules can be made from whole precursor or parent antibodies using techniques known in the art. Exemplary techniques are discussed in more detail herein.

In certain embodiments, the antibody molecule can be recombinantly produced, e.g., produced by phage display or by combinatorial methods. Phage display and combinatorial methods for generating anti- LINGO-1 antibodies are known in the art (as described in, *e.g.,* Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) BiolTechnology 9:1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) JMol Biol 226:889-896; Clackson et al. (1991) Nature 352:624-628; Gram et al. (1992) PNAS 89:3576-3580; Garrad et al. (1991) BiolTechnology 9:1373-1377; Hoogenboom et al. (1991) Nuc Acid Res 19:4133-4137; and Barbas et al. (1991) PNAS 88:7978-7982, the contents of all of which are incorporated by reference herein).

In one embodiment, the anti- LINGO-1 antibody is a fully human antibody (*e.g.,* an antibody made in a mouse which has been genetically engineered to produce an antibody from a human immunoglobulin sequence), or a non-human antibody, e.g., a rodent (mouse or rat), goat, primate (*e.g*., monkey), camel antibody. The non-human antibody can be a rodent (mouse or rat antibody). Method of producing rodent antibodies are known in the art.

Human monoclonal antibodies can be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, *e.g.,* Wood et al. International Application WO 91/00906, Kucherlapati et al. PCT publication WO 91/10741; Lonberg et al. International Application WO 92/03918; Kay et al. International Application 92/03917; Lonberg, N. et al. 1994 Nature 368:856-859; Green, L.L. et al. 1994 Nature Genet. 7:13-21; Morrison, S.L. et al. 1994 Proc. Natl. Acad. Sci. USA 81:6851-6855; Bruggeman et al. 1993 Year Immunol 7:33-40; Tuaillon et al. 1993 PNAS 90:3720-3724; Bruggeman et al. 1991 Eur J Immunol 21:1323-1326).

An anti- LINGO-1 antibody can be one in which the variable region, or a portion thereof, e.g., the CDRs, are generated in a non-human organism, *e.g*., a rat or mouse. Chimeric, CDR-grafted, and humanized antibodies are within the invention. Antibodies generated in a non-human organism, *e.g*., a rat or mouse, and then modified, *e.g*., in the variable framework or constant region, to decrease antigenicity in a human are within the invention.

Chimeric antibodies can be produced by recombinant DNA techniques known in the art. For example, a gene encoding the Fc constant region of a murine (or other species) monoclonal antibody molecule is digested with restriction enzymes to remove the region encoding the murine Fc, and the equivalent portion of a gene encoding a human Fc constant region is substituted (see Robinson et al., International Patent Publication PCT/US86/02269; Akira, et al., European Patent Application 184,187; Taniguchi, M., European Patent Application 171,496; Morrison et al., European Patent Application 173,494; Neuberger et al., International Application WO 86/01533; Cabilly et al. U.S. Patent No. 4,816,567; Cabilly et al., European Patent Application 125,023; Better et al. (1988 Science 240:1041-1043); Liu et al. (1987) PNAS 84:3439-3443; Liu et al., 1987, J. Immunol. 139:3521-3526; Sun et al. (1987) PNAS 84:214-218; Nishimura et al., 1987, Canc. Res. 47:999-1005; Wood et al. (1985) Nature 314:446-449; and Shaw et al., 1988, J. Natl Cancer Inst. 80:1553-1559).

A humanized or CDR-grafted antibody will have at least one or two but generally all three recipient CDRs (of heavy and or light immuoglobulin chains) replaced with a donor CDR. The antibody may be replaced with at least a portion of a non-human CDR or only some of the CDRs may be replaced with non-human CDRs. It is only necessary to replace the number of CDRs required for binding of the humanized antibody to LINGO-1 or a fragment thereof.

An antibody can be humanized by methods known in the art. Humanized antibodies can be generated by replacing sequences of the Fv variable region which are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. US 5,585,089, US 5,693,761 and US 5,693,762, the contents of all of which are hereby incorporated by reference. Humanized or CDR-grafted antibodies can be produced by CDR-grafting or CDR substitution, wherein one, two, or all CDRs of an immunoglobulin chain can be replaced. See e.g., U.S. Patent 5,225,539; Jones et al. 1986 Nature 321:552-525; Verhoeyan et al. 1988 Science 239:1534; Beidler et al. 1988 J. Immunol. 141:4053-4060; Winter US 5,225,539, the contents of all of which are hereby expressly incorporated by reference. Winter describes a CDR-grafting method which may be used to prepare the humanized antibodies of the present invention (UK Patent Application GB 2188638A, filed on March 26, 1987; Winter US 5,225,539), the contents of which is expressly incorporated by reference.

Also within the scope of the invention are humanized antibodies in which specific amino acids have been substituted, deleted or added. Humanized antibodies can have amino acid substitutions in the framework region, such as to improve binding to the antigen. For example, a humanized antibody will have framework residues identical to the donor framework residue or to another amino acid other than the recipient framework residue. To generate such antibodies, a selected, small number of acceptor framework residues of the humanized immunoglobulin chain can be replaced by the corresponding donor amino acids. Preferred locations of the substitutions include amino acid residues adjacent to the CDR, or which are capable of interacting with a CDR (*see e.g.,* US 5,585,089). Criteria for selecting amino acids from the donor are described in US 5,585,089, e.g., columns 12-16 of US 5,585,089, the e.g., columns 12-16 of US 5,585,089, the contents of which are hereby incorporated by reference. Other techniques for humanizing antibodies are described in Padlan et al. EP 519596 A1, published on December 23, 1992.

The anti- LINGO-1 antibody can be a single chain antibody. A single-chain antibody (scFV) may be engineered (see, for example, Colcher, D. et al. (1999) Ann N Y Acad Sci 880:263-80; and Reiter, Y. (1996) Clin Cancer Res 2:245-52). The single chain antibody can be dimerized or multimerized to generate multivalent antibodies having specificities for different epitopes of the same target LINGO-1 protein.

In yet other embodiments, the antibody molecule has a heavy chain constant region chosen from, *e.g.,* the heavy chain constant regions of IgG1, IgG2, IgG3, IgG4, IgM, IgA1, IgA2, IgD, and IgE; particularly, chosen from, *e.g.,* the *(e.g.,* human) heavy chain constant regions of IgG1, IgG2, IgG3, and IgG4. In another embodiment, the antibody molecule has a light chain constant region chosen from, *e.g.,* the *(e.g.,* human) light chain constant regions of kappa or lambda. The constant region can be altered, *e.g*., mutated, to modify the properties of the antibody (*e.g*., to increase or decrease one or more of: Fc receptor binding, antibody glycosylation, the number of cysteine residues, effector cell function, and/or complement function). In one embodiment the antibody has: effector function; and can fix complement. In other embodiments the antibody does not; recruit effector cells; or fix complement. In another embodiment, the antibody has reduced or no ability to bind an Fc receptor. For example, it is a isotype or subtype, fragment or other mutant, which does not support binding to an Fc receptor, *e.g.,* it has a mutagenized or deleted Fc receptor binding region.

LINGO-1 antibody molecules can comprise a constant region which mediates one or more effector functions. For example, binding of the C1 component of complement to an antibody constant region may activate the complement system. Activation of complement is important in the opsonisation and lysis of cell pathogens. The activation of complement also stimulates the inflammatory response and may also be involved in autoimmune hypersensitivity. Further, antibodies bind to receptors on various cells via the Fc region, with a Fc receptor binding site on the antibody Fc region binding to a Fc receptor (FcR) on a cell. There are a number of Fc receptors which are specific for different classes of antibody, including IgG (gamma receptors), IgE (epsilon receptors), IgA (alpha receptors) and IgM (mu receptors). Binding of antibody to Fc receptors on cell surfaces triggers a number of important and diverse biological responses including engulfment and destruction of antibody-coated particles, clearance of immune complexes, lysis of antibody-coated target cells by killer cells (also referred to herein as antibody-dependent cell-mediated cytotoxicity, or ADCC), release of inflammatory mediators, placental transfer and control of immunoglobulin production.

In certain embodiments, the anti-LINGO-1 antibody molecule, in which at least a fraction of one or more of the constant region domains has been deleted or otherwise altered so as to provide desired biochemical characteristics such as reduced effector functions, the ability to non-covalently dimerize, increased ability to localize at the site of a tumor, reduced serum half-life, or increased serum half-life when compared with a whole, unaltered antibody of approximately the same immunogenicity. For example, certain antibodies for use in the diagnostic and treatment methods described herein are domain deleted antibodies which comprise a polypeptide chain similar to an immunoglobulin heavy chain, but which lack at least a portion of one or more heavy chain domains. For instance, in certain antibodies, one entire domain of the constant region of the modified antibody will be deleted, for example, all or part of the CH2 domain will be deleted.

In certain LINGO-1 antibody molecules, the Fc portion may be mutated to decrease effector function using techniques known in the art. For example, the deletion or inactivation (through point mutations or other means) of a constant region domain may reduce Fc receptor binding of the circulating modified antibody thereby increasing tumor localization. In other cases it may be that constant region modifications consistent with the instant invention moderate complement binding and thus reduce the serum half life and nonspecific association of a conjugated cytotoxin. Yet other modifications of the constant region may be used to modify disulfide linkages or oligosaccharide moieties that allow for enhanced localization due to increased antigen specificity or antibody flexibility. The resulting physiological profile, bioavailability and other biochemical effects of the modifications, such as tumor localization, biodistribution and serum half-life, may easily be measured and quantified using well know immunological techniques without undue experimentation.

### Exemplary anti-LINGO-1 Antibody Molecules

In certain embodiments, the anti-LINGO-1 antibody molecules comprise, consist essentially of, or consist of an immunoglobulin heavy chain variable region (VH), where at least one of the CDRs of the heavy chain variable region, or at least two the CDRs of the heavy chain variable region are at least 80%, 85%, 90% or 95% identical to reference heavy chain CDR1, CDR2, or CDR3 amino acid sequences of Li62 or Li81 or variants thereof as described in **Table 3.** Alternatively, the CDR1, CDR2, and CDR3 regions of the VH are at least 80%, 85%, 90% or 95% identical to reference heavy chain CDR1, CDR2, and CDR3 amino acid sequences of Li62 or Li81 or variants thereof as described in **Table 3.** Thus, according to this embodiment a heavy chain variable region of the invention has CDR1, CDR2, or CDR3 polypeptide sequences related to the polypeptide sequences shown in **Table 3.** In certain embodiment, the anti-LINGO-1 antibody molecules comprise, consist essentially of, or consist of the VH polypeptide or a fragment thereof as described in Table 3, or an amino acid sequence at least 80%, 85%, 90% or 95% identical thereto.

**Table 3: LINGO-1 Antibody VH Sequences**

| Antibody | VH SEQUENCE | VH CDR1 | VH CDR2 | VH CDR3 |
|---|---|---|---|---|
| Li62 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGHNDWYFDL WGRGTLVTVSS (SEQ ID NO:1) | IYPMF (SEQ ID NO:2) | WIGPSG GITKYA DSVKG (SEQ ID NO:3) | EGHND WYFDL (SEQ ID NO:4) |
| Li62 variant B06 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGYYDWYFDQ WGRGTLVTVSS (SEQ ID NO:53) | IYPMF (SEQ ID NO:2) | WIGPSG GITKYA DSVKG (SEQ ID NO:3) | EGYYD WYFDQ (SEQ ID NO:17) |
| Li62 variant B12 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGQYDWYFDV WGRGTLVTVSS (SEQ ID NO:54) | IYPMF (SEQ ID NO:2) | WIGPSG GITKYA DSVKG (SEQ ID NO:3) | EGQYD WYFDV (SEQ ID NO:18) |
| Li62 variant F06 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGDYDWYFDL WGRGTLVTVSS (SEQ ID NO:55) | IYPMF (SEQ ID NO:2) | WIGPSG GITKYA DSVKG (SEQ ID NO:3) | EGDYD WYFDL (SEQ ID NO:19) |
| Li62 variant B01 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGQYDWYFEL WGRGTLVTVSS (SEQ ID NO:56) | IYPMF (SEQ ID NO:2) | WIGPSG GITKYA DSVKG (SEQ ID NO:3) | EGQYD WYFEL (SEQ ID NO:20) |
| Li62 variant D09 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREADIDWFFDL WGRGTLVTVSS (SEQ ID NO:57) | IYPMF (SEQ ID NO:2) | WIGPSG GITKYA DSVKG (SEQ ID NO:3) | EADID WFFDL (SEQ ID NO:21) |
| Li62 variant D12 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGHYDWYFDL WGRGTLVTVSS (SEQ ID NO:58) | IYPMF (SEQ ID NO:2) | WIGPSG GITKYA DSVKG (SEQ ID NO:3) | EGHYD WYFDL (SEQ ID NO:22) |
| Li62 variant F01 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGRYDWYFDP WGRGTLVTVSS (SEQ ID NO:59) | IYPMF (SEQ ID NO:2) | WIGPSG GITKYA DSVKG (SEQ ID NO:3) | EGRYD WYFDP (SEQ ID NO:23) |
| Li62 variant F02 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGDYDWYFGL WGRGTLVTVSS (SEQ ID NO:60) | IYPMF (SEQ ID NO:2) | WIGPSG GITKYA DSVKG (SEQ ID NO:3) | EGDYD WYFGL (SEQ ID NO:24) |
| Li62 variant F06 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGRYDWYFDL WGRGTLVTVSS (SEQ ID NO:61) | IYPMF (SEQ ID NO:2) | WIGPSG GITKYA DSVKG (SEQ ID NO:3) | EGRYD WYFDL (SEQ ID NO:25) |
| Li62 variant F10 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCARESHIDRYFDLW GRGTLVTVSS (SEQ ID NO:62) | IYPMF (SEQ ID NO:2) | WIGPSG GITKYA DSVKG (SEQ ID NO:3) | ESHIDR YFDL (SEQ ID NO:26) |
| Li62 variant G08 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGQYDWYFDV WGRGTLVTVSS (SEQ ID NO:63) | IYPMF (SEQ ID NO:2) | WIGPSG GITKYA DSVKG (SEQ ID NO:3) | EGQYD WYFDV (SEQ ID NO:27) |
| Li62 variant H08 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGHYNGYFDL WGRGTLVTVSS (SEQ ID NO:64) | IYPMF (SEQ ID NO:2) | WIGPSG GITKYA DSVKG (SEQ ID NO:3) | EGHYN GYFDL (SEQ ID NO:28) |
| Li62 variant C10 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGYYDWYFDL WGRGTLVTVSS (SEQ ID NO:65) | IYPMF (SEQ ID NO:2) | WIGPSG GITKYA DSVKG (SEQ ID NO:3) | EGYYD WYFDL (SEQ ID NO:29) |
| Li62 variant C02 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGTYDWYLDL WGRGTLVTVSS (SEQ ID NO:66) | IYPMF (SEQ ID NO:2) | WIGPSG GITKYA DSVKG (SEQ ID NO:3) | EGTYD WYLDL (SEQ ID NO:30) |
| Li62 variant D05 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGYYDWYFEL WGRGTLVTVSS (SEQ ID NO:67) | IYPMF (SEQ ID NO:2) | WIGPSG GITKYA DSVKG (SEQ ID NO:3) | EGYYD WYFEL (SEQ ID NO:31) |
| Li62 variant F02 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGLIDWFFDQ WGRGTLVTVSS (SEQ ID NO:68) | IYPMF (SEQ ID NO:2) | WIGPSG GITKYA DSVKG (SEQ ID NO:3) | EGLID WFFDQ (SEQ ID NO:32) |
| Li62 variant C10 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGQFDWYFDL WGRGTLVTVSS (SEQ ID NO:69) | IYPMF (SEQ ID NO:2) | WIGPSG GITKYA DSVKG (SEQ ID NO:3) | EGQFD WYFDL (SEQ ID NO:33) |
| Li62 variant H08 | EVQLLESGGGLVQPGGSLRLSCAASGFTFSIYPMFWV RQAPGKGLEWVSWIGPSGGITKYADSVKGRFTISRD NSKNTLYLQMNSLRAEDTATYYCAREGTYDWYFDL WGRGTLVTVSS (SEQ ID NO:70) | IYPMF (SEQ ID NO:2) | WIGPSG GITKYA DSVKG (SEQ ID NO:3) | EGTYD WYFDL (SEQ ID NO:34) |
| Li81 |  (SEQ ID NO:5) | AYEM K (SEQ ID NO:6) | VIGPSG GFTFYA DSVKG (SEQ ID NO: 7) | EGDND AFDI (SEQ ID NO:8) |
| Li81 variant F09 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGENDAFDVW GQGTTVTVSS (SEQ ID NO:71) | AYEM K (SEQ ID NO:6) | VIGPSG GFTFYA DSVKG (SEQ ID NO: 7) | EGEND AFDV (SEQ ID NO:35) |
| Li81 variant G02 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDNDAYDT WGQGTTVTVSS (SEQ ID NO:72) | AYEM K (SEQ ID NO:6) | VIGPSG GFTFYA DSVKG (SEQ ID NO: 7) | EGDND AYDT (SEQ ID NO:36) |
| Li81 variant H03 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGTNDAFDIW GQGTTVTVSS (SEQ ID NO:73) | AYEM K (SEQ ID NO:6) | VIGPSG GFTFYA DSVKG (SEQ ID NO: 7) | EGTND AFDI (SEQ ID NO:37) |
| Li81 variant A12 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDNDAFDSW GQGTTVTVSS (SEQ ID NO:74) | AYEM K (SEQ ID NO:6) | VIGPSG GFTFYA DSVKG (SEQ ID NO: 7) | EGDND AFDS (SEQ ID NO:38) |
| Li81 variant C02 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDNDAFDTW GQGTTVTVSS (SEQ ID NO:75) | AYEM K (SEQ ID NO:6) | VIGPSG GFTFYA DSVKG (SEQ ID NO: 7) | EGDND AFDT(S EQ ID NO:39) |
| Li81 variant C11 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDNDAYDR WGQGTTVTVSS (SEQ ID NO:76) | AYEM K (SEQ ID NO:6) | VIGPSG GFTFYA DSVKG (SEQ ID NO: 7) | EGDND AYDR (SEQ ID NO:40) |
| Li81 variant D11 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDNDVFDSW GQGTTVTVSS (SEQ ID NO:77) | AYEM K (SEQ ID NO:6) | VIGPSG GFTFYA DSVKG (SEQ ID NO: 7) | EGDND VFDS (SEQ ID NO:41) |
| Li81 variant E05 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDDDVFDM WGQGTTVTVSS (SEQ ID NO:78) | AYEM K (SEQ ID NO:6) | VIGPSG GFTFYA DSVKG (SEQ ID NO: 7) | EGDDD VFDM (SEQ ID NO:42) |
| Li81 variant H04 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGYNDAFDFW GQGTTVTVSS (SEQ ID NO:79) | AYEM K (SEQ ID NO:6) | VIGPSG GFTFYA DSVKG (SEQ ID NO: 7) | EGYND AFDF (SEQ ID NO:43) |
| Li81 variant B04 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDDDAYDM WGQGTTVTVSS (SEQ ID NO:80) | AYEM K (SEQ ID NO:6) | VIGPSG GFTFYA DSVKG (SEQ ID NO: 7) | EGDDD AYDM (SEQ ID NO:44) |
| Li81 variant A02 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEQDYDTYDLW GQGTTVTVSS (SEQ ID NO:81) | AYEM K (SEQ ID NO:6) | VIGPSG GFTFYA DSVKG (SEQ ID NO: 7) | EQDYD TYDL (SEQ ID NO:45) |
| Li81 variant B12 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGDDDAFDTW GQGTTVTVSS (SEQ ID NO:82) | AYEM K (SEQ ID NO:6) | VIGPSG GFTFYA DSVKG (SEQ ID NO: 7) | EGDDD AFDT (SEQ ID NO:46) |
| Li81 variant H06 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEADDDAFDIW GQGTTVTVSS (SEQ ID NO:83) | AYEM K (SEQ ID NO:6) | VIGPSG GFTFYA DSVKG (SEQ ID NO: 7) | EADDD AFDI (SEQ ID NO:47) |
| Li81 variant H08 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGENDAFDM WGQGTTVTVSS (SEQ ID NO:84) | AYEM K (SEQ ID NO:6) | VIGPSG GFTFYA DSVKG (SEQ ID NO: 7) | EGEND AFDM (SEQ ID NO:48) |
| Li81 variant E07 | EVQLLESGGGLVQPGGSLRLSCAASGFTFSAYEMKW VRQAPGKGLEWVSVIGPSGGFTFYADSVKGRFTISRD NSKNTLYLQMNSLRAEDTAVYYCATEGEYDTYDIW GQGTTVTVSS (SEQ ID NO:85) | AYEM K (SEQ ID NO:6) | VIGPSG GFTFYA DSVKG (SEQ ID NO: 7) | EGEYD TYDI (SEQ ID NO:49) |

In another embodiment, the anti-LINGO-1 antibody molecule includes a polypeptide comprising, consisting essentially of, or consisting of an immunoglobulin heavy chain variable region (VH), wherein at least the CDR3 region is at least 80%, 85%, 90% or 95% identical to a reference CDR3 sequence selected from the group consisting of SEQ ID NOs: 4, 8 and 17-49. In further embodiments, the CDR3 region is identical to a reference CDR3 sequence selected from the group consisting of SEQ ID NOs: 4, 8 and 17-49. In still further embodiments, the anti-LINGO-1 antibody molecule includes a polypeptide comprising, consisting essentially of, or consisting of an immunoglobulin heavy chain variable region (VH), wherein, the CDR1 and CDR2 regions are at least 80%, 85%, 90%, 95% or 100% identical to the CDR1 and CDR2 amino acid sequences of SEQ ID NOs: 2 and 3, respectively, and the CDR3 region is at least 80%, 85%, 90%, 95% or 100% identical to a CDR3 amino acid sequence selected from the group consisting of SEQ ID NOs: 4 and 17-34. In other embodiments, the anti-LINGO-1 antibody molecule includes a polypeptide comprising, consisting essentially of, or consisting of an immunoglobulin heavy chain variable region (VH), wherein the CDR1 and CDR2 regions are at least 80%, 85%, 90%, 95% or 100% identical to the CDR1 and CDR2 amino acid sequences of SEQ ID NOs: 6 and 7, respectively, and the CDR3 region is at least 80%, 85%, 90%, 95% or 100% identical to a CDR3 amino acid sequence selected from the group consisting of SEQ ID NOs: 8 and 35-49.

In another embodiment, the anti-LINGO-1 antibody molecule includes a polypeptide comprising, consisting essentially of, or consisting of an immunoglobulin heavy chain variable region (VH) in which the CDR1, CDR2, and CDR3 regions have polypeptide sequences which are identical to the CDR1, CDR2, and CDR3 groups shown in **Table 3.** In certain embodiments, the anti-LINGO-1 antibody molecule includes the VH polypeptide specifically or preferentially binds to LINGO-1.

In a further embodiment, the anti-LINGO-1 antibody molecule includes a polypeptide comprising, consisting essentially of, or consisting of a VH polypeptide at least 80%, 85%, 90% 95% or 100% identical to a reference VH polypeptide sequence selected from SEQ ID NOs: 1, 5 and 53-85. In one particular embodiment, the VH polypeptide comprises a CDR3 amino acid sequence selected from the group consisting of SEQ ID NOs: 4, 8 and 17-49.

In certain embodiments, the anti-LINGO-1 antibody molecule includes a polypeptide comprising, consisting essentially of, or consisting of a VH polypeptide selected from the group consisting of SEQ ID NOs: 1, 5 and 53-85. In certain embodiments, an antibody or antigen-binding fragment comprising the VH polypeptide specifically or preferentially binds to LINGO-1.

In another aspect, the anti-LINGO-1 antibody molecule includes a VH comprising the amino acids of SEQ ID NO: 1 or SEQ ID NO: 5. In certain embodiments, an antibody or antigen-binding fragment comprising the VH that specifically or preferentially binds to LINGO-1. In certain embodiments, an antibody or antigen-binding fragment thereof comprising, consisting essentially of, or consisting of a VH that specifically or preferentially binds to the same epitope as Li62, Li81 or a variant thereof as described in **Table 3** or will competitively inhibit such a monoclonal antibody from binding to LINGO-1.

In certain embodiments, the anti-LINGO-1 antibody molecule includes a polypeptide, comprising, consisting essentially of, or consisting of an immunoglobulin heavy chain which is identical to the polypeptide of SEQ ID NO:146 except for a replacement of one or more of the following amino acids: W50, P53,157 and/or W104. In some embodiments, W50 is replaced with an H, F, L, M, G, I, or D residue. In some embodiments, P53 is replaced with an L, S, T, W, or G residue. In some embodiments, 157 is replaced with a G, M, N, H, L, F, W, Y, S, P, V or T residue. In some embodiments, W104 is replaced with a V, H, S, Q, M, L, T, or I residue.

In certain embodiments, the anti-LINGO-1 antibody molecule includes a polypeptide, comprising, consisting essentially of, or consisting of an immunoglobulin heavy chain variable region which is identical to the polypeptide of SEQ ID NO:5 except for a replacement of amino acid P53. In some embodiments, P53 is replaced with an L, S, T, W, or G residue.

In certain embodiments, the anti-LINGO-1 antibody molecule includes a polypeptide, comprising, consisting essentially of, or consisting of an immunoglobulin heavy chain variable region which is identical to the polypeptide of SEQ ID NO: 1 except for a replacement of one or more of the following amino acids: W50, P53, 157 and/or W104. In some embodiments, W50 is replaced with an H, F, L, M, G, I, or D residue. In some embodiments, P53 is replaced with an L, S, T, W, or G residue. In some embodiments, 157 is replaced with a G, M, N, H, L, F, W, Y, S, P, V or T residue. In some embodiments, W104 is replaced with a V, H, S, Q, M, L, T, or I residue.

In certain embodiments, the anti-LINGO-1 antibody molecule includes a polypeptide, comprising, consisting essentially of, or consisting of an immunoglobulin heavy chain variable region which is identical to the polypeptide of SEQ ID NO:66 except for a replacement of one or more of the following amino acids: W50, P53, 157 and/or W104. In some embodiments, W50 is replaced with an H, F, L, M, G, I, or D residue. In some embodiments, P53 is replaced with an L, S, T, W, or G residue. In some embodiments, 157 is replaced with a G, M, N, H, L, F, W, Y, S, P, V or T residue. In some embodiments, W104 is replaced with a V, H, S, Q, M, L, T, or I residue.

In certain embodiments, the anti-LINGO-1 antibody molecule includes one or more of the VH polypeptides described above specifically or preferentially binds to the same epitope as Li62, Li81 or a variant thereof as described in **Table 3,** or can competitively inhibit such an antibody from binding to LINGO-1.

In another embodiment, the anti-LINGO-1 antibody molecule includes a polypeptide comprising, consisting essentially of, or consisting of an immunoglobulin light chain variable region (VL), where at least one of the CDRs of the light chain variable region or at least two of the CDRs of the light chain variable region are at least 80%, 85%, 90% or 95% identical to reference heavy chain CDR1, CDR2, or CDR3 amino acid sequences from monoclonal LINGO-1 antibodies disclosed herein. Alternatively, the CDR1, CDR2 and CDR3 regions of the VL are at least 80%, 85%, 90% or 95% identical to reference light chain CDR1, CDR2, and CDR3 amino acid sequences from monoclonal LINGO-1 antibodies disclosed herein. Thus, according to this embodiment a light chain variable region of the antibody molecule has CDR1, CDR2, and CDR3 polypeptide sequences related to the polypeptides shown in **Table 4**. In certain embodiments, the anti-LINGO-1 antibody molecule comprising the VL polypeptide specifically or preferentially binds to LINGO-1.

**Table 4: LINGO-1 Antibody VL Sequences**

| Antibody | VL SEQUENCE | VL CDR1 | VL CDR2 | VL CDR3 |
|---|---|---|---|---|
| Li62 | DIQMTQSPSFLSASVGDSVAITCRASQDISRYLAWYQQ RPGKAPKLLIYDASNLQTGVPSRFSGSGSGTDFTFTITS LQPEDFGTYYCQQYDTLHPSFGPGTTVDIK (SEQ ID NO: 9) | RASQD ISRYL A (SEQ ID NO:10) | DASNL QT (SEQ ID NO:11) | QQYDT LHPS (SEQ ID NO:12) |
| Li81 | DIQMTQSPATLSLSPGERATLSCRASQSVSSYLAWYQ QKPGQAPRLLIYDASNRATGIPARFSGSGSGTDFTLTIS SLEPEDFAVYYCQQRSNWPMYTFGQGTKLEIK (SEQ ID NO:13) | RASQS VSSYL A (SEQ ID NO:14) | DASNR AT (SEQ ID NO: 15) | QQRSN WPMY T (SEQ ID NO: 16) |

In another embodiment, the anti-LINGO-1 antibody molecule includes a polypeptide comprising, consisting essentially of, or consisting of an immunoglobulin light chain variable region (VL) in which the CDR1, CDR2, and CDR3 regions have polypeptide sequences which are identical to the CDR1, CDR2, and CDR3 groups shown in Table 4. In certain embodiments, an antibody or antigen-binding fragment comprising the VL polypeptide specifically or preferentially binds to LINGO-1.

In a further embodiment, the anti-LINGO-1 antibody molecule includes a polypeptide comprising, consisting essentially of, or consisting of a VL polypeptide at least 80%, 85%, 90% or 95% identical to a reference VL polypeptide sequence selected from SEQ ID NO: 9 or SEQ ID NO: 13, shown in **Table 4.** In certain embodiments, the anti-LINGO-1 antibody molecule includes comprising the VL polypeptide specifically or preferentially binds to LINGO-1. In another aspect, the anti-LINGO-1 antibody molecule includes a polypeptide comprising, consisting essentially of, or consisting of a VL polypeptide selected from SEQ ID NO: 9 or SEQ ID NO: 13, shown in **Table 4.** In certain embodiments, the anti-LINGO-1 antibody molecule comprising the VL polypeptide specifically or preferentially binds to LINGO-1.

In certain embodiments, the anti-LINGO-1 antibody molecule includes a polypeptide consisting essentially of, or consisting of an immunoglobulin light chain which is identical to the polypeptide of SEQ ID NO: 145 except for a replacement of amino acid W94. In some embodiments, W94 is replaced with an A, D, L, N, G, Q, V, or S residue.

In certain embodiments, the anti-LINGO-1 antibody molecule includes a polypeptide, comprising, consisting essentially of, or consisting of an immunoglobulin light chain variable region which is identical to the polypeptide of SEQ ID NO:5 except for a replacement of amino acid W94. In some embodiments, W94 is replaced with an A, D, L, N, G, Q, V, or S residue.

In certain embodiments, the anti-LINGO-1 antibody molecule includes a polypeptide comprising, consisting essentially of, one or more of the VL polypeptides described above specifically or preferentially binds to the same epitope as Li62 or Li81, or will competitively inhibit such a monoclonal antibody from binding to LINGO-1.

In other embodiments, the anti-LINGO-1 antibody molecule comprises, consists essentially of or consists of a VH polypeptide, as shown in **Table 3,** and a VL polypeptide, as shown in **Table 4,** selected from the group consisting of: i) SEQ ID NO: 1 or SEQ ID NOs: 53-70 and SEQ ID NO: 9; and iii) SEQ ID NO: 5 or SEQ ID NOs: 71-85 and SEQ ID NO: 13.

In some embodiments, the anti-LINGO-1 antibody molecule comprises, consists essentially of or consists of an antibody heavy chain as shown below in SEQ ID NO: 86, or an amino acid sequence at least 80%, 85%, 90% or 95% identical thereto.

EVQLLESGGGLVQPGGSLRLSCAASGFTFSAYEMKWVRQAPGKGLEWVSV
IGPSGGFTFYADISVKGRFTISRDNSKNTLYLQMNSLRAEDTAVYYCATE
GDNDAFDIWGQGTTVTVSSASTKGPISVFPLAPSSKSTSGGTAALGCLVK
DYFPEPVTVSWNSGALTSGVHTFPAVLQSSGLYSLSSVVTIVPSSSLGTQ
TYICNVNHKPSNTKVDKKVEPKSCDKTHTCPPCPAPELLGGPSVFLFPPK
PKDTLIMISRTPEVTCVVVDVSHEDPEVKFNWYVDGVEVHNAKTKPREEQ
YNSTYRVVSVLTVLHQDIWLNGKEYKCKVSNKALPAPIEKTISKAKGQPR
EPQVYTLPPSRDELTKNQVSLTCLVKGFYPSIDIAVEWESNGQPENNYKT
TPPVLDSDGSFFLYSKLTVDKSRWQQGNVFSCSVMHEALHNHYTIQKSLS LSPG (SEQ
ID NO:86)

In other embodiments, the anti-LINGO-1 antibody molecule comprises, consists essentially of or consists of an aglycosylated version of an antibody heavy chain. For example, an aglycosylated version of Li81 is described in PCT/US2008/000316, filed Jan. 9, 2008 and US 8,128,926, which are incorporated herein by reference in its entirety. An aglycosylated version of the Li81 antibody was created by changing a single amino acid (T to A) in the Li81 heavy chain sequence. The sequence of an aglycosylated version of Li81 heavy chain (SEQ ID NO:50) is shown below. The single amino acid change is marked in bold and underlined:

EVQLLESGGGLVQPGGSLRLSCAASGFTFSAYEMKWVRQAPGKGLEWVSV
IGPSGGFTFYADISVKGRFTISRDNSKNTLYLQMNSLRAEDTAVYYCATE
GDNDAFDIWGQGTTVTVSSASTKGPISVFPLAPSSKSTSGGTAALGCLVK
DYFPEPVTVSWNSGALTSGVHTFPAVLQSSGLYSLSSVVTIVPSSSLGTQ
TYICNVNHKPSNTKVDKKVEPKSCDKTHTCPPCPAPELLGGPSVFLFPPK
PKDTLIMISRTPEVTCVVVDVSHEDPEVKFNWYVDGVEVHNAKTKPREEQ
YNSAYRVVSVLTVLHQDIWLNGKEYKCKVSNKALPAPIEKTISKAKGQPR
EPQVYTLPPSRDELTKNQVSLTCLVKGFYPSIDIAVEWESNGQPENNYKT
TPPVLDSDGSFFLYSKLTVDKSRWQQGNVFSCSVMHEALHNHYTIQKSLS LSPG. (SEQ
ID NO: 50)

The anti-LINGO-1 antibody molecule includes a heavy chain at least 80%, 85%, 90% or 95% identical to a reference polypeptide comprising the amino acids of SEQ ID NO:50 or 86. In certain embodiments, an antibody or antigen-binding fragment comprising the heavy chain specifically or preferentially binds to LINGO-1.

In one embodiment, the anti-LINGO-1 antibody molecule is a fully human anti-LINGO-1 monoclonal antibody engineered into an aglycosyl immunoglobulin G subclass 1 (IgG1) framework to reduce effector function (also referred to herein as anti-LINGO-1 Antibody 1. Histological and functional evaluations of LINGO-1 knock-out mice have been performed, and *in vivo* pharmacological activity of anti-LINGO-1 Antibody 1 has been demonstrated in several animal models of demyelination. Anti-LINGO-1 Antibody 1 has been characterized *in vitro* and *in vivo* based on the evaluation of binding characteristics, biological activity, and pharmacological activity. The results of these studies indicate that anti-LINGO-1 Antibody 1 has the following characteristics described in **Table 1.**

**Table 1. Characteristics of Anti-LINGO-1 Antibody 1**

| |
|---|
| Binds to LINGO-1 with similar high apparent affinity across human, monkey, rat and mouse. |
| Is selective for LINGO-1 and does not bind the other LINGO family members, LINGO-2, LINGO-3, or LINGO-4. |
| Promotes differentiation of primary rat, monkey, and human oligodendrocytes *in vitro.* |
| Promotes axonal myelination in an *in vitro* rat dorsal root ganglion/OPC co-culture bioassay. |
| Has reduced Fc (γ) and complement effector functions compared to wild-type IgG1. |
| Is efficacious in animal models using biochemical and functional readouts. Remyelination activity has been demonstrated in the rat LPC model following systemic administration from 1 to 100 mg/kg. |
| Functional recovery in the rat MOG-EAE model has been demonstrated following weekly systemic administration of 3 and 10 mg/kg. |
| Is efficacious in animal models when given in the presence of interferon β. |
| Is efficacious in animal models when given in the presence of high dose corticosteroids. |

In one embodiment, the antibody molecule includes a VH wherein the VH CDR1, CDR2, and CDR3 comprise the amino acids of SEQ ID NOs: 6, 7, and 8, respectively, or an amino acid sequence substantially identical thereto *(*e.g., an amino acid sequence at least 80%, 85%, 90% or 95% identical thereto).

In one embodiment, the antibody molecule includes a VH wherein the VH CDR1, CDR2, and CDR3 comprise the amino acids of SEQ ID NOs: 2, 3, and 30, respectively, or an amino acid sequence substantially identical thereto (*e.g.,* an amino acid sequence at least 80%, 85%, 90% or 95% identical thereto).

In another embodiment, the antibody molecule includes a VL wherein the VL CDR1, CDR2, and CDR3 comprise the amino acids of SEQ ID NOs: 14, 15, and 16, respectively, or an amino acid sequence substantially identical thereto (*e.g.,* an amino acid sequence at least 80%, 85%, 90% or 95% identical thereto).

In another embodiment, the antibody molecule includes a VH wherein the VL CDR1, CDR2, and CDR3 comprise the amino acids of SEQ ID NOs: 10, 11, and 12, respectively, or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise the amino acids of SEQ ID NOs: 6, 7, and 8, respectively; and a VL wherein the VL CDR1, CDR2, and CDR3 comprise the amino acids of SEQ ID NOs: 14, 15, and 16, respectively; or an amino acid sequence substantially identical thereto (*e.g.,* an amino acid sequence at least 80%, 85%, 90% or 95% identical thereto).

In one embodiment, the antibody molecule includes a VH wherein the VH CDR1, CDR2, and CDR3 comprise the amino acids of SEQ ID NOs: 2, 3, and 30, respectively; and a VL wherein the VL CDR1, CDR2, and CDR3 comprise the amino acids of SEQ ID NOs: 10, 11, and 12, respectively; or an amino acid sequence substantially identical thereto (*e.g.,* an amino acid sequence at least 80%, 85%, 90% or 95% identical thereto).

In one embodiment, the antibody molecule includes a VH that includes the amino acid sequence of SEQ ID NO: 5, or an amino acid sequence substantially identical thereto (*e.g.,* an amino acid sequence at least 80%, 85%, 90% or 95% identical to said SEQ ID NO: 5).

In one embodiment, the antibody molecule includes a VH that includes the amino acid sequence of SEQ ID NO:66, or an amino acid sequence substantially identical thereto (*e.g.,* an amino acid sequence at least 80%, 85%, 90% or 95% identical to said SEQ ID NO: 66).

In one embodiment, the antibody molecule includes a VL that includes the amino acid sequence of SEQ ID NO:13, or an amino acid sequence substantially identical thereto (*e.g.,* an amino acid sequence at least 80%, 85%, 90% or 95% identical to said SEQ ID NO: 13).

In one embodiment, the antibody molecule includes a VL that includes the amino acid sequence of SEQ ID NO:9, or an amino acid sequence substantially identical thereto (*e.g.,* an amino acid sequence at least 80%, 85%, 90% or 95% identical to said SEQ ID NO: 9).

In one embodiment, the antibody molecule includes a VH that includes the amino acid sequence of SEQ ID NO:5, or an amino acid sequence substantially identical thereto (*e.g.,* an amino acid sequence at least 80%, 85%, 90% or 95% identical to said SEQ ID NO: 5); and a VL that includes the amino acid sequence of SEQ ID NO: 13, or an amino acid sequence substantially identical thereto (*e.g.,* an amino acid sequence at least 80%, 85%, 90% or 95% identical to said SEQ ID NO: 13).

In one embodiment, the antibody molecule includes a VH that includes the amino acid sequence of SEQ ID NO:66, or an amino acid sequence substantially identical thereto (*e.g.,* an amino acid sequence at least 80%, 85%, 90% or 95% identical to said SEQ ID NO: 66); and a VL that includes the amino acid sequence of SEQ ID NO: 9, or an amino acid sequence substantially identical thereto (*e.g.,* an amino acid sequence at least 80%, 85%, 90% or 95% identical to said SEQ ID NO: 9).

In another embodiment, the antibody molecule includes a heavy chain as shown below, comprising the amino acid sequence of SEQ ID NO: 275, or a sequence substantially identical thereto (*e.g.,* an amino acid sequence at least 80%, 85%, 90% or 95% identical thereto), as follows:

EVQLLESGGG LVQPGGSLRL SCAASGFTFS AYEMKWVRQA PGKGLEWVSV
IGPSGGFTFY ADSVKGRFTI SRDNSKNTLY LQMNSLRAED TAVYYCATEG
DNDAFDIWGQ GTTVTVSSAS TKGPSVFPLA PSSKSTSGGT AALGCLVKDY
FPEPVTVSWN SGALTSGVHT FPAVLQSSGL YSLSSVVTVP SSSLGTQTYI
CNVNHKPSNT KVDKKVEPKS CDKTHTCPPC PAPELLGGPS VFLFPPKPKD
TLMISRTPEV TCVVVDVSHE DPEVKFNWYV DGVEVHNAKT KPREEQYNSA
YRVVSVLTVL HQDWLNGKEY KCKVSNKALP APIEKTISKA KGQPREPQVY

TLPPSRDELT KNQVSLTCLV KGFYPSDIAV EWESNGQPEN NYKTTPPVLD
SDGSFFLYSK LTVDKSRWQQ GNVFSCSVMH EALHNHYTQK SLSLSPG (SEQ ID
NO: 275).

In other embodiments, the antibody molecule includes a light chain as shown below comprising the amino acid sequence of SEQ ID NO: 276, or a sequence substantially identical thereto (*e.g.,* an amino acid sequence at least 80%, 85%, 90% or 95% identical thereto), as follows:

DIQMTQSPAT LSLSPGERAT LSCRASQSVS SYLAWYQQKP GQAPRLLIYD
ASNRATGIPA RFSGSGSGTD FTLTISSLEP EDFAVYYCQQ RSNWPMYTFG
QGTKLEIKRT VAAPSVFIFP PSDEQLKSGT ASVVCLLNNF YPREAKVQWK
VDNALQSGNS QESVTEQDSK DSTYSLSSTL TLSKADYEKH KVYACEVTHQ
GLSSPVTKSF NRGEC (SEQ ID NO: 276).

Any of the polypeptides described above may further include additional polypeptides, e.g., a signal peptide to direct secretion of the encoded polypeptide, antibody constant regions as described herein, or other heterologous polypeptides as described herein. Additionally, polypeptides of the invention include polypeptide fragments as described elsewhere. Additionally polypeptides of the invention include fusion polypeptide, Fab fragments, and other derivatives, as described herein.

Also, as described in more detail elsewhere herein, the present invention includes compositions comprising the polypeptides described above.

It will also be understood by one of ordinary skill in the art that LINGO-1 antibody polypeptides as disclosed herein may be modified such that they vary in amino acid sequence from the naturally occurring binding polypeptide from which they were derived. For example, a polypeptide or amino acid sequence derived from a designated protein may be similar, e.g., have a certain percent identity to the starting sequence, e.g., it may be 60%, 70%, 75%, 80%, 85%, 90%, or 95% identical to the starting sequence.

Furthermore, nucleotide or amino acid substitutions, deletions, or insertions leading to conservative substitutions or changes at "non-essential" amino acid regions may be made. For example, a polypeptide or amino acid sequence derived from a designated protein may be identical to the starting sequence except for one or more individual amino acid substitutions, insertions, or deletions, e.g., one, two, three, four, five, six, seven, eight, nine, ten, fifteen, twenty or more individual amino acid substitutions, insertions, or deletions. In certain embodiments, a polypeptide or amino acid sequence derived from a designated protein has one to five, one to ten, one to fifteen, or one to twenty individual amino acid substitutions, insertions, or deletions relative to the starting sequence.

### Soluble LINGO Antagonists and Fusion Proteins

In another embodiment, the reparative agent, *e.g.,* the antagonist of LINGO-1, is a soluble LINGO molecule, *e.g.,* a LINGO-1 molecule (*e.g.,* a fragment of LINGO-1), or a soluble form of a component of the LINGO-1 complex (*e.g.,* a soluble form of NgR1, p75, or TAJ (TROY)).

In certain embodiments, a soluble LINGO molecule or a LINGO-1 antibody molecule comprises an amino acid sequence or one or more moieties not normally associated with an antibody. Exemplary modifications are described in more detail below. For example, a single-chain fv antibody fragment of the invention may comprise a flexible linker sequence, or may be modified to add a functional moiety (*e.g.,* PEG, a drug, a toxin, or a label).

An antibody molecule, a soluble form of LINGO-1, or a complex component, as described herein, can be used alone or functionally linked (*e.g.,* by chemical coupling, genetic or polypeptide fusion, non-covalent association or otherwise) to a second moiety, a heterologous moiety, *e.g.,* a heterologous polypeptide. The term "heterologous" as applied to a polynucleotide or a polypeptide, means that a portion with which it is not naturally linked in nature. For example, the polynucleotide or polypeptide is derived from a distinct entity from that of the rest of the entity to which it is being compared. For instance, as used herein, a "heterologous polypeptide" to be fused to a LINGO-1 antibody molecule is derived from a non-immunoglobulin polypeptide of the same species, or an immunoglobulin or non-immunoglobulin polypeptide of a different species.

Exemplary heterologous moieties include, but are not limited to, an immunoglobulin Fc domain, serum albumin, pegylation, a GST, Lex-A and an MBP polypeptide sequence. The fusion proteins may additionally include a linker sequence joining the first moiety, *e.g.*, the antibody molecule, the soluble form of LINGO-1 or the complex component, to the second moiety. In other embodiments, additional amino acid sequences can be added to the N- or C-terminus of the fusion protein to facilitate expression, steric flexibility, detection and/or isolation or purification. For example, a soluble form of LINGO-1 or a complex component can be fused to a heavy chain constant region of the various isotypes, including: IgG1, IgG2, IgG3, IgG4, IgM, IgA1, IgA2, IgD, and IgE.

It shall be understood that the antibody molecules and soluble or fusion proteins described herein can be functionally linked (*e.g.,* by chemical coupling, genetic fusion, non-covalent association or otherwise) to one or more other molecular entities, such as an antibody (*e.g.,* a bispecific or a multispecific antibody), among others.

In one embodiment, the fusion protein includes the extracellular domain of LINGO or the complex component (or a sequence homologous thereto), and, *e.g.,* fused to, a human immunoglobulin Fc chain, *e.g.,* human IgG (*e.g.,* human IgG1 or human IgG2, or a mutated form thereof). The Fc sequence can be mutated at one or more amino acids to reduce effector cell function, Fc receptor binding and/or complement activity.

In certain embodiments, an anti-LINGO-1 antibody molecule can comprise, consist essentially of, or consist of, a fusion protein. Fusion proteins in this context are chimeric molecules which comprise, for example, an immunoglobulin antigen-binding domain with at least one target binding site, and at least one heterologous portion. The amino acid sequences can normally exist in separate proteins that are brought together in the fusion polypeptide or they may normally exist in the same protein, but are placed in a new arrangement in the fusion polypeptide. Fusion proteins can be created, for example, by chemical synthesis, or by creating and translating a polynucleotide in which the peptide regions are encoded in the desired relationship.

### Nucleic Acid Molecule/Recombinant Expression

Nucleic acid molecules, host cells and vectors that include a nucleotide sequence encoding any of the polypeptides, *e.g*., reparative agents and immunomodulators, described herein, are also encompassed by the invention.

In one exemplary embodiment, an isolated polynucleotide comprising, consisting essentially of, or consisting of a nucleic acid encoding an immunoglobulin heavy chain variable region (VH) of an anti-LINGO-1 antibody molecule, where at least one of the CDRs of the heavy chain variable region or at least two of the CDRs of the heavy chain variable region are at least 80%, 85%, 90% or 95% identical to reference heavy chain CDR1, CDR2, or CDR3 amino acid sequences of Li62 or Li81 or variants thereof as described in **Table 3** is provided. Alternatively, the CDR1, CDR2, and CDR3 regions of the VH are at least 80%, 85%, 90% or 95% identical to reference heavy chain CDR1, CDR2, and CDR3 amino acid sequences of Li62 or Li81 or variants thereof as described in **Table 3.** Thus, according to this embodiment, a heavy chain variable region of the invention has CDR1, CDR2, or CDR3 polypeptide sequences related to the polypeptide sequences shown in **Table 3.**

In another exemplary embodiment, an isolated polynucleotide comprising, consisting essentially of, or consisting of a nucleic acid encoding an immunoglobulin light chain variable region (VL) of an anti-LINGO-1 antibody molecule, where at least one of the CDRs of the light chain variable region or at least two of the CDRs of the light chain variable region are at least 80%, 85%, 90% or 95% identical to reference light chain CDR1, CDR2, or CDR3 amino acid sequences from monoclonal LINGO-1 antibodies disclosed herein is provided. Alternatively, the CDR1, CDR2, and CDR3 regions of the VL are at least 80%, 85%, 90% or 95% identical to reference light chain CDR1, CDR2, and CDR3 amino acid sequences from monoclonal LINGO-1 antibodies disclosed herein. Thus, according to one embodiment, a light chain variable region of the invention has CDR1, CDR2, or CDR3 polypeptide sequences related to the polypeptide sequences shown in **Table 4.**

Any of the polynucleotides described above may further include additional nucleic acids, encoding, *e.g.,* a signal peptide to direct secretion of the encoded polypeptide, antibody constant regions as described herein, or other heterologous polypeptides as described herein.

Compositions comprising the polynucleotides comprising one or more of the polynucleotides described above are also disclosed. In one embodiment, the compositions comprising a first polynucleotide and second polynucleotide wherein said first polynucleotide encodes a VH polypeptide as described herein and wherein said second polynucleotide encodes a VL polypeptide as described herein.

Also disclosed are fragments of the polynucleotides of the invention, as described elsewhere. Additionally polynucleotides which encode fusion polynucleotides, Fab fragments, and other derivatives, as described herein, are also contemplated by the invention.

The polynucleotides can be produced or manufactured by any method known in the art. For example, if the nucleotide sequence of the antibody is known, a polynucleotide encoding the antibody may be assembled from chemically synthesized oligonucleotides (*e.g.,* as described in Kutmeier et al., BioTechniques 17:242 (1994)), which, briefly, involves the synthesis of overlapping oligonucleotides containing portions of the sequence encoding the antibody, annealing and ligating of those oligonucleotides, and then amplification of the ligated oligonucleotides by PCR.

Recombinant expression of a polypeptide described herein, *e.g.,* an antibody molecule that binds to LINGO-1, requires construction of an expression vector containing the polynucleotide that encodes the polypeptide, *e.g*., the antibody molecule. Once a polynucleotide encoding the antibody molecule or a heavy or light chain of an antibody, or portion thereof (preferably containing the heavy or light chain variable domain), has been obtained, the vector for the production of the antibody molecule may be produced by recombinant DNA technology using techniques known in the art. Thus, methods for preparing a protein by expressing a polynucleotide containing a polypeptide encoding nucleotide sequence are described herein and in US 8,058,406, the contents of which are incorporated by reference in their entirety.

Methods known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. Replicable vectors comprising a nucleotide sequence encoding a polypeptide described herein (*e.g.,* an anti-LINGO-1 antibody molecule, or a heavy or light chain thereof, or a heavy or light chain variable domain) operably linked to a promoter. Such vectors may include the nucleotide sequence encoding the constant region of the antibody molecule (see, e.g., PCT Publication WO 86/05807; PCT Publication WO 89/01036; and U.S. Pat. No. 5,122,464) and the variable domain of the antibody may be cloned into such a vector for expression of the entire heavy or light chain.

The host cell may be co-transfected with two expression vectors, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain (Proudfoot, Nature 322:52 (1986); Kohler, Proc. Natl. Acad. Sci. USA 77:2197 (1980)). The coding sequences for the heavy and light chains may comprise cDNA or genomic DNA.

The term "vector" or "expression vector" is used herein to mean vectors used as a vehicle for introducing into and expressing a desired gene in a host cell. As known to those skilled in the art, such vectors can easily be selected from the group consisting of plasmids, phages, viruses and retroviruses. In general, vectors compatible with the instant invention will comprise a selection marker, appropriate restriction sites to facilitate cloning of the desired gene and the ability to enter and/or replicate in eukaryotic or prokaryotic cells.

For the purposes of this invention, numerous expression vector systems may be employed. For example, one class of vector utilizes DNA elements which are derived from animal viruses such as bovine papilloma virus, polyoma virus, adenovirus, vaccinia virus, baculovirus, retroviruses (RSV, MMTV or MOMLV) or SV40 virus. Others involve the use of polycistronic systems with internal ribosome binding sites. Additionally, cells which have integrated the DNA into their chromosomes may be selected by introducing one or more markers which allow selection of transfected host cells. The marker may provide for prototrophy to an auxotrophic host, biocide resistance (e.g., antibiotics) or resistance to heavy metals such as copper. The selectable marker gene can either be directly linked to the DNA sequences to be expressed, or introduced into the same cell by co-transformation. Additional elements may also be needed for optimal synthesis of mRNA. These elements may include signal sequences, splice signals, as well as transcriptional promoters, enhancers, and termination signals.

In one embodiment, the cloned variable region genes are inserted into an expression vector along with the heavy and light chain constant region genes (preferably human) synthetic as discussed above. In one embodiment, this is effected using a proprietary expression vector of Biogen IDEC, Inc., referred to as NEOSPLA (U.S. Pat. No. 6,159,730). This vector contains the cytomegalovirus promoter/enhancer, the mouse beta globin major promoter, the SV40 origin of replication, the bovine growth hormone polyadenylation sequence, neomycin phosphotransferase exon 1 and exon 2, the dihydrofolate reductase gene and leader sequence. This vector has been found to result in very high level expression of antibodies upon incorporation of variable and constant region genes, transfection in CHO cells, followed by selection in G418 containing medium and methotrexate amplification. Of course, any expression vector which is capable of eliciting expression in eukaryotic cells may be used in the present invention. Examples of suitable vectors include, but are not limited to plasmids pcDNA3, pHCMV/Zeo, pCR3.1, pEF1/His, pIND/GS, pRc/HCMV2, pSV40/Zeo2, pTRACER-HCMV, pUB6/V5-His, pVAX1, and pZeoSV2 (available from Invitrogen, San Diego, Calif.), and plasmid pCI (available from Promega, Madison, Wis.). In general, screening large numbers of transformed cells for those which express suitably high levels if immunoglobulin heavy and light chains is routine experimentation which can be carried out, for example, by robotic systems. Vector systems are also taught in U.S. Pat. Nos. 5,736,137 and 5,658,570, each of which is incorporated by reference in its entirety herein. This system provides for high expression levels, *e.g.,* >30 pg/cell/day. Other exemplary vector systems are disclosed, *e.g*., in U.S. Pat. No. 6,413,777.

In other embodiments, the LINGO-1 antibody molecules can be expressed using polycistronic constructs such as those disclosed in United States Patent Application Publication No. 2003-0157641 A1, filed Nov. 18, 2002 and incorporated herein in its entirety. In these novel expression systems, multiple gene products of interest such as heavy and light chains of antibodies may be produced from a single polycistronic construct. These systems advantageously use an internal ribosome entry site (IRES) to provide relatively high levels of binding polypeptides in eukaryotic host cells. Compatible IRES sequences are disclosed in U.S. Pat. No. 6,193,980 which is also incorporated herein. Those skilled in the art will appreciate that such expression systems can be used to effectively produce the full range of polypeptides disclosed in the instant application.

More generally, once the vector or DNA sequence encoding a monomeric subunit of the polypeptide, *e.g*., the anti-LINGO-1 antibody molecule, has been prepared, the expression vector may be introduced into an appropriate host cell. Introduction of the plasmid into the host cell can be accomplished by various techniques well known to those of skill in the art. These include, but are not limited to, transfection (including electrophoresis and electroporation), protoplast fusion, calcium phosphate precipitation, cell fusion with enveloped DNA, microinjection, and infection with intact virus. *See,* Ridgway, A. A. G. "Mammalian Expression Vectors" Vectors, Rodriguez and Denhardt, Eds., Butterworths, Boston, Mass., Chapter 24.2, pp. 470-472 (1988). Typically, plasmid introduction into the host is via electroporation. The host cells harboring the expression construct are grown under conditions appropriate to the production of the light chains and heavy chains, and assayed for heavy and/or light chain protein synthesis. Exemplary assay techniques include enzyme-linked immunosorbent assay (ELISA), radioimmunoassay (RIA), or fluorescence-activated cell sorter analysis (FACS), immunohistochemistry and the like.

The expression vector is transferred to a host cell by conventional techniques and the transfected cells are then cultured by conventional techniques to produce a polypeptide for use in the methods described herein. Thus, the invention includes host cells containing a polynucleotide encoding an antibody of the invention, or a heavy or light chain thereof, operably linked to a heterologous promoter. In preferred embodiments for the expression of double-chained antibodies, vectors encoding both the heavy and light chains may be co-expressed in the host cell for expression of the entire immunoglobulin molecule, as detailed in US 8,058,406, the contents of which are incorporated by reference herein in its entirety.

As used herein, "host cells" refers to cells which harbor vectors constructed using recombinant DNA techniques and encoding at least one heterologous gene. In descriptions of processes for isolation of antibodies from recombinant hosts, the terms "cell" and "cell culture" are used interchangeably to denote the source of antibody unless it is clearly specified otherwise. In other words, recovery of polypeptide from the "cells" may mean either from spun down whole cells, or from the cell culture containing both the medium and the suspended cells.

A variety of host-expression vector systems may be utilized to express polypeptides, *e.g*., antibody molecules, for use in the methods described herein. These include, but are not limited to, microorganisms such as bacteria (*e.g., E. coli, B. subtilis*) transformed with recombinant bacteriophage DNA, plasmid DNA or cosmid DNA expression vectors containing polypeptide coding sequences; yeast (*e.g., Saccharomyces, Pichia*) transformed with recombinant yeast expression vectors containing antibody coding sequences; insect cell systems infected with recombinant virus expression vectors (e.g., baculovirus) containing polypeptide coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BLK, 293, 3T3 cells) harboring recombinant expression constructs containing promoters derived from the genome of mammalian cells (*e.g.,* metallothionein promoter) or from mammalian viruses (*e.g*., the adenovirus late promoter; the vaccinia virus 7.5K promoter). Typically, bacterial cells such as *Escherichia coli,* and more typically, eukaryotic cells, especially for the expression of whole recombinant antibody molecule, are used for the expression of a recombinant polypeptide or antibody molecule. For example, mammalian cells such as Chinese hamster ovary cells (CHO), in conjunction with a vector such as the major intermediate early gene promoter element from human cytomegalovirus is an effective expression system for polypeptides antibodies (Foecking et al., Gene 45:101 (1986); Cockett et al., Bio/Technology 8:2 (1990)).

The host cell line used for protein expression is often of mammalian origin; those skilled in the art are credited with ability to preferentially determine particular host cell lines which are best suited for the desired gene product to be expressed therein. Exemplary host cell lines include, but are not limited to, CHO (Chinese Hamster Ovary), DG44 and DUXB11 (Chinese Hamster Ovary lines, DHFR minus), HELA (human cervical carcinoma), CVI (monkey kidney line), COS (a derivative of CVI with SV40 T antigen), VERY, BHK (baby hamster kidney), MDCK, 293, W138, R1610 (Chinese hamster fibroblast) BALBC/3T3 (mouse fibroblast), HAK (hamster kidney line), SP2/O (mouse myeloma), P3.times.63-Ag3.653 (mouse myeloma), BFA-1c1BPT (bovine endothelial cells), RAJI (human lymphocyte) and 293 (human kidney). Host cell lines are typically available from commercial services, the American Tissue Culture Collection or from published literature.

In addition, a host cell strain may be chosen which modulates the expression of the inserted sequences, or modifies and processes the gene product in the specific fashion desired. Such modifications (*e.g.,* glycosylation) and processing (*e.g.,* cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (e.g., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which stably express the antibody molecule.

CHO cells are particularly preferred. In certain embodiment, the antibody molecules are expressed in CHO cells stably transfected with expression vectors containing the IgG₁-agly heavy light chain structural genes specific to the human LINGO-1 protein. A native human kappa light chain signal peptide and a human heavy chain signal peptide, which are post-translationally removed by endoplasmic reticulum-associated signal peptidase, can be used to direct secretion of the anti-LINGO-1 antibody molecule. The antibody molecule can be purified from the media and formulated as a liquid. The antibody molecule can consists of 2 heavy and 2 light chains connected by inter-chain disulfide bonds. In one embodiment, the mass of the intact antibody molecule is approximately 144.4 kDa.

A number of selection systems may be used, including but not limited to the herpes simplex virus thymidine kinase (Wigler et al., Cell 11:223 (1977)), hypoxanthine-guanine phosphoribosyltransferase (Szybalska & Szybalski, Proc. Natl. Acad. Sci. USA 48:202 (1992)), and adenine phosphoribosyltransferase (Lowy et al., Cell 22:817 1980) genes can be employed in tk-, hgprt- or aprt-cells, respectively. Also, antimetabolite resistance can be used as the basis of selection for the following genes: dhfr, which confers resistance to methotrexate (Wigler et al., Natl. Acad. Sci. USA 77:357 (1980); O'Hare et al., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 Clinical Pharmacy 12:488-505; Wu and Wu, Biotherapy 3:87-95 (1991); Tolstoshev, Ann. Rev. Pharmacol. Toxicol. 32:573-596 (1993); Mulligan, Science 260:926-932 (1993); and Morgan and Anderson, Ann. Rev. Biochem. 62:191-217 (1993); TIB TECH 11(5):155-215 (May, 1993); and hygro, which confers resistance to hygromycin (Santerre et al., Gene 30:147 (1984). Methods commonly known in the art of recombinant DNA technology which can be used are described in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Prolocols in Human Genetics, John Wiley & Sons, NY (1994); Colberre-Garapin et al., J. Mol. Biol. 150:1 (1981), which are incorporated by reference herein in their entireties.

Additional methods and host systems expression, production and/or purification of the polypeptides, *e.g*., antibodies, are disclosed in US 8,058,406, the contents of which are incorporated by reference herein in its entirety.

### Nucleic Acid Antagonists

In certain embodiments, the LINGO-1 antagonist inhibits the expression of nucleic acid encoding a LINGO-1. Examples of such LINGO-1 antagonists include nucleic acid molecules, for example, antisense molecules, ribozymes, RNAi double stranded molecules, triple helix molecules, microRNA molecules that hybridize to a nucleic acid encoding a LINGO-1, or a transcription regulatory region, and block or reduce mRNA expression of LINGO-1.

An "antisense" nucleic acid can include a nucleotide sequence which is complementary to a "sense" nucleic acid encoding a protein, *e.g*., complementary to the coding strand of a doublestranded cDNA molecule or complementary to an mRNA sequence. The antisense nucleic acid can be complementary to an entire LINGO-1 coding strand, or to only a portion thereof. In another embodiment, the antisense nucleic acid molecule is antisense to a "noncoding region" of the coding strand of a nucleotide sequence encoding LINGO-1 (e.g., the 5' and 3' untranslated regions). Anti-sense agents can include, for example, from about 8 to about 80 nucleobases (i.e. from about 8 to about 80 nucleotides), e.g., about 8 to about 50 nucleobases, or about 12 to about 30 nucleobases. Anti-sense compounds include ribozymes, external guide sequence (EGS) oligonucleotides (oligozymes), and other short catalytic RNAs or catalytic oligonucleotides which hybridize to the target nucleic acid and modulate its expression. Anti-sense compounds can include a stretch of at least eight consecutive nucleobases that are complementary to a sequence in the target gene. An oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, *i.e.,* under physiological conditions in the case of *in vivo* assays or therapeutic treatment or, in the case of *in vitro* assays, under conditions in which the assays are conducted. Exemplary antisense compounds include DNA or RNA sequences that specifically hybridize to the target nucleic acid, *e.g.,* the mRNA encoding LINGO-1. The complementary region can extend for between about 8 to about 80 nucleobases. The compounds can include one or more modified nucleobases, which are known in the art. Descriptions of nucleic acid agents are available. *See, e.g.,* U.S. Patent Nos. 4,987,071;. 5,116,742; and 5,093,246; Woolf et al. (1992) Proc Natl Acad Sci USA*;* Antisense RNA and DNA, D.A. Melton, Ed., Cold Spring Harbor Laboratory, Cold Spring Harbor, N.Y. (1988); 89:7305-9; Haselhoff and Gerlach (1988) Nature 334:585-59; Helene, C. (1991) Anticancer Drug Des. 6:569-84; Helene (1992) Ann. N.Y. Acad. Sci. 660:27-36; and Maher (1992) Bioassays 14:807-15.

siRNAs are small double stranded RNAs (dsRNAs) that optionally include overhangs. For example, the duplex region of an siRNA is about 18 to 25 nucleotides in length, *e.g.,* about 19, 20, 21, 22, 23, or 24 nucleotides in length. Typically, the siRNA sequences are exactly complementary to the target mRNA. dsRNAs and siRNAs in particular can be used to silence gene expression in mammalian cells (e.g., human cells). siRNAs also include short hairpin RNAs (shRNAs) with 29-base-pair stems and 2-nucleotide 3' overhangs. See, e.g., Clemens et al. (2000) Proc. Natl. Acad. Sci. USA 97:6499-6503; Billy et al. (2001) Proc. Natl. Sci. USA 98:14428-14433; Elbashir et al. (2001) Nature. 411:494-8; Yang et al. (2002) Proc. Natl. Acad. Sci. USA 99:9942-9947; Siolas et al. (2005), Nat. Biotechnol. 23(2):227-31; 20040086884; U.S. 20030166282; 20030143204; 20040038278; and 20030224432.

In still another embodiment, the nucleic acid molecule is a ribozyme. A ribozyme having specificity for a LINGO-1-encoding nucleic acid can include one or more sequences complementary to the nucleotide sequence of a LINGO-1 mRNA, and a sequence having known catalytic sequence responsible for mRNA cleavage (*see* U.S. Pat. No. 5,093,246 or Haselhoff and Gerlach (1988) Nature 334:585-591; Cech et al. U.S. Patent No. 4,987,071; and Cech et al. U.S. Patent No. 5,116,742; Bartel, D. and Szostak, J.W. (1993) Science 261:1411-1418).

In one embodiment, the nucleic acid molecule is a microRNA molecule. A microRNA having specificity for a LINGO-1-encoding nucleic acid can include one or more sequences complementary to the nucleotide sequence of a LINGO-1 mRNA, which can result in gene silencing via translational repression or target degradation (*see* Bartel DP (2009) Cell 136 (2): 215-33; Kusenda B, et al. (2006) Biomed Pap Med Fac Univ Palacky Olomouc Czech Repub 150 (2): 205-15).

LINGO-1 gene expression can be inhibited by targeting nucleotide sequences complementary to the regulatory region of the LINGO-1 (e.g., the LINGO-1 promoter and/or enhancers) to form triple helical structures that prevent transcription of the LINGO-1 gene in target cells. See generally, Helene, C. (1991) Anticancer Drug Des. 6:569-84; Helene, C. (1992) Ann. N.Y. Acad. Sci. 660:27-36; and Maher, L.J. (1992) Bioassays 14:807-15. The potential sequences that can be targeted for triple helix formation can be increased by creating a so-called "switchback" nucleic acid molecule.

A LINGO-1 nucleic acid molecule can be modified at the base moiety, sugar moiety or phosphate backbone to improve, *e.g*., the stability, hybridization, or solubility of the molecule. For non-limiting examples of synthetic oligonucleotides with modifications see Toulmé (2001) Nature Biotech. 19:17 and Faria et al. (2001) Nature Biotech. 19:40-44; Hyrup B. et al. (1996) Bioorganic & Medicinal Chemistry 4: 5-23).

### Immunomodulatory Agents

Several immunomodulatory agents are presently used to modify the course of multiple sclerosis in patients. Such agents include, but are not limited to, an IFN-β 1 molecule; a polymer of glutamic acid, lysine, alanine and tyrosine, *e.g*., glatiramer; an antibody or fragment thereof against alpha-4 integrin, *e.g*., natalizumab; an anthracenedione molecule, *e.g.,* mitoxantrone; a fingolimod, *e.g.,* FTY720; a dimethyl fumarate, *e.g*., an oral dimethyl fumarate; an antibody to the alpha subunit of the IL-2 receptor of T cells, *e.g.,* daclizumab; an antibody against CD52, *e.g.,* alemtuzumab; an inhibitor of a dihydroorotate dehydrogenase, *e.g.,* teriflunomide (*e.g.,* ; an antibody to CD20, *e.g*., ocrelizumab; and a corticosteroid. The reparative agents disclosed herein can be used in combination with any of these agents.

Exemplary immunomodulatory agents are described in more detail as follows.

### IFNβ agents (Beta interferons)

One known therapy for MS includes treatment with interferon beta. Interferons (IFNs) are natural proteins produced by the cells of the immune systems of most animals in response to challenges by foreign agents such as viruses, bacteria, parasites and tumor cells. Interferons belong to the large class of glycoproteins known as cytokines. Interferon beta has 165 amino acids. Interferons alpha and beta are produced by many cell types, including T-cells and B-cells, macrophages, fibroblasts, endothelial cells, osteoblasts and others, and stimulate both macrophages and NK cells. Interferon gamma is involved in the regulation of immune and inflammatory responses. It is produced by activated T-cells and Th1 cells.

Several different types of interferon are now approved for use in humans. Interferon alpha (including forms interferon alpha-2a, interferon alpha-2b, and interferon alfacon-1) was approved by the United States Food and Drug Administration (FDA) as a treatment for Hepatitis C. There are two currently FDA-approved types of interferon beta. Interferon beta la (Avonex®) is identical to interferon beta found naturally in humans, and interferon beta 1b (Betaseron®) differs in certain ways from interferon beta la found naturally in humans, including that it contains a serine residue in place of a cysteine residue at position 17. Other uses of interferon beta have included treatment of AIDS, cutaneous T-cell lymphoma, Acute Hepatitis C (non-A, non-B), Kaposi's sarcoma, malignant melanoma, hairy cell leukemia, and metastatic renal cell carcinoma.

IPNβ agents can be administered to the subject by any method known in the art, including systemically (*e.g*., orally, parenterally, subcutaneously, intravenously, rectally, intramuscularly, intravitreally, intraperitoneally, intranasally, transdermally, or by inhalation or intracavitary installation). Typically, the IFNβ agents are administered subcutaneously, or intramuscularly.

IFNβ agents can be used to treat those subjects determined to be "responders" using the methods described herein. In one embodiment, the IFNβ agents are used as a monotherapy (*i.e.,* as a single "disease modifying therapy") although the treatment regimen can further comprise the use of "symptom management therapies" such as antidepressants, analgesics, anti-tremor agents, etc. In one embodiment, the IFNβ agent is an IFNβ-1A agent (*e.g*., Avonex®, Rebif®). In another embodiment, the INFβ agent is an INFβ-1B agent (*e.g*., Betaseron®, Betaferon®, Extavia®).

Avonex®, an Interferon β-1a, is indicated for the treatment of patients with relapsing forms of MS that are determined to be responders using the methods described herein to slow the accumulation of physical disability and decrease the frequency of clinical exacerbations. Avonex® (Interferon beta-la) is a 166 amino acid glycoprotein with a predicted molecular weight of approximately 22,500 daltons. It is produced by recombinant DNA technology using genetically engineered Chinese Hamster Ovary cells into which the human interferon beta gene has been introduced. The amino acid sequence of Avonex® is identical to that of natural human interferon beta. The recommended dosage of Avonex® (Interferon beta-la) is 30 mcg injected intramuscularly once a week. Avonex® is commercially available as a 30 mcg lyophilized powder vial or as a 30 mcg prefilled syringe.

Interferon beta 1a (Avonex®) is identical to interferon beta found naturally in humans (AVONEX®, *i.e.,* Interferon beta Ia (SwissProt Accession No. P01574 and gi:50593016). The sequence of interferon beta is:

MTNKCLLQIALLLCFSTTALSMSYNLLGFLQRSSNFQCQKLLWQLNGRLEYCLKDRM
NFDIPEEIKQLQQFQKEDAALTIYEMLQNIFAIFRQDSSSTGWNETIVENLLANVYHQIN
HLKTVLEEKLEKEDFTRGKLMSSLHLKRYYGRILHYLKAKEYSHCAWTIVRVEILRNF
YFINRLTGYLRN (SEQ ID NO: 275).

Methods for making Avonex® are known in the art.

Treatment of responders identified using the methods described herein further contemplates that compositions (*e.g*., IFN beta 1 a molecules) having biological activity that is substantially similar to that of AVONEX® will permit successful treatment similar to treatment with AVONEX® when administered in a similar manner. Such other compositions include, e.g., other interferons and fragments, analogues, homologues, derivatives, and natural variants thereof with substantially similar biological activity. In one embodiment, the INFβ agent is modified to increase one or more pharmacokinetic properties. For example, the INFβ agent can be a modified form of interferon 1a to include a pegylated moiety. PEGylated forms of interferon beta 1a are described in, *e.g.,* Baker, D.P. et al. (2006) Bioconjug Chem 17(1):179-88; Arduini, RM et al. (2004) Protein Expr Purif 34(2):229-42; Pepinsky, RB et al. (2001) J. Pharmacol. Exp. Ther. 297(3):1059-66; Baker, D.P. et al. (2010) J Interferon Cytokine Res 30(10):777-85 (all of which are incorporated herein by reference in their entirety, and describe a human interferon beta la modified at its N-terminal alpha amino acid to include a PEG moiety, *e.g.,* a 20 kDa mPEG-O-2-methylpropionaldehyde moiety). Pegylated forms of IFN beta la can be administered by, *e.g.,* injectable routes of administration (*e.g.,* subcutaneously).

Rebif® is also an Interferon β-1a agent, while Betaseron®, Betaferon®, and Extavia® are Interferon β-1b agents. Both Rebif® and Betaseron® are formulated for administration by subcutaneous injection.

Dosages of IFNβ agents to administer can be determined by one of skill in the art, and include clinically acceptable amounts to administer based on the specific interferon-beta agent used. For example, AVONEX® is typically administered at 30 microgram once a week via intramuscular injection. Other forms of interferon beta 1a, specifically REBIF®, is administered, for example, at 22 microgram three times a week or 44 micrograms once a week, via subcutaneous injection. Interferon beta- 1A can be administered, *e.g*., intramuscularly, in an amount of between 10 and 50 µg. For example, AVONEX® can be administered every five to ten days, *e.g.,* once a week, while Rebif® can be administered three times a week.

### Anti-VLA4 antibody (e.g., Natalizumab (Tysabri®))

Anti-VLA4 antibodies (*e.g*., Natalizumab) inhibit the migration of leukocytes from the blood to the central nervous system. These antibodies bind to VLA-4 (also called α4β1) on the surface of activated T-cells and other mononuclear leukocytes. They can disrupt adhesion between the T-cell and endothelial cells, and thus prevent migration of mononuclear leukocytes across the endothelium and into the parenchyma. As a result, the levels of pro-inflammatory cytokines can also be reduced. Natalizumab can decrease the number of brain lesions and clinical relapses and accumulation of disability in patients with relapse remitting multiple sclerosis and relapsing secondary-progressive multiple sclerosis.

Natalizumab and related VLA-4 binding antibodies are described, *e.g*., in U.S. Pat. No. 5,840,299. Monoclonal antibodies 21.6 and HP1/2 are exemplary murine monoclonal antibodies that bind VLA-4. Natalizumab is a humanized version of murine monoclonal antibody 21.6 (see, *e.g.,* U.S. Pat. No. 5,840,299). A humanized version of HP 1/2 has also been described (see, *e.g.,* U.S. Pat. No. 6,602,503). Several additional VLA-4 binding monoclonal antibodies, such as HP2/1, HP2/4, L25 and P4C2, are described, *e.g.,* in U.S. Pat. No. 6,602,503; Sanchez-Madrid et al, (1986) Eur. J. Immunol 16:1343-1349; Hemler et al, (1987) J Biol. Chem. 2:11478-11485; Issekutz et al. (1991) J Immunol 147: 109 (TA-2 mab); Pulido et al. (1991) J Biol. Chem. 266: 10241-10245; and U.S. Pat. No. 5,888,507). The contents of the aforesaid publications (including the antibody compositions, dosages, methods of administration and production) are incorporated herein by reference in their entirety.

### Dimethyl Fumarate (Tecfidera®)

Dimethyl fumarate, DMF, (Tecfidera®) is a fumaric acid ester. DMF is thought to decrease leukocyte passage through the blood brain barrier and exert neuroprotective effects by the activation of antioxidative pathways, specifically through activation of the Nrf-2 pathway (Lee et al. (2008) Int MS Journal 15: 12-18). Research also suggests that BG-12® has the potential to reduce the activity and impact of inflammatory cells on the CNS and induce direct cytoprotective responses in CNS cells. These effects may enhance the CNS cells' ability to mitigate the toxic inflammatory and oxidative stress that plays a role in MS pathophysiology.

### Glatiramer acetate (Copaxone®)

Copaxone® (glatiramer acetate) consists of the acetate salts of synthetic polypeptides, specifically the four naturally occurring amino acids: L-glutamic acid, L-alanine, L-tyrosine, and L-lysine (Bornstein et al. (1987) N Engl J Med. 317: 408-414). Copaxone® exhibits structural similarity to myelin basic protein and is thought to function as an immune modulator by shifting the T helper cell type 1 response towards a T helper cell type 2 response (Duda et al. (2000) J Clin Invest 105: 967-976; Nicholas et al. (2011) Drug Design, Development, and Therapy 5: 255-274).

### Mitoxantrone (Novantrone®, an anthracenedione molecule)

Mitoxantrone is an anthracenedione molecule (1,4-dihydroxy-5,8-bis[2-(2-hydroxyethylamino) ethylamino]-anthracene-9,10-dione) and a type II topoisomerase inhibitor that disrupts DNA synthesis and repair of cells. It is used to treat cancers and MS. Mitoxantrone is used to treat several forms of advancing MS, including secondary progressive MS, progressive relapsing MS, and advanced relapsing-remitting MS.

For example, mitoxantrone is effective in slowing the progression of secondary progressive MS and extending the time between relapses in relapsing-remitting MS and progressive relapsing MS (Fox E (2006) Clin Ther 28 (4): 461-74).

### Fingolimod (Gilenya®; sphingosine 1-phosphate receptor modulator)

Fingolimod is an immunomodulating drug, approved for treating MS. It has reduced the rate of relapses in relapsing-remitting multiple sclerosis by over half, but may have serious adverse effects. Fingolimod is a sphingosine 1-phosphate receptor modulator, which sequesters lymphocytes in lymph nodes, preventing them from moving to the central nervous system for autoimmune responses in MS.

### Antibodies to the alpha subunit of the IL-2 receptor of T cells (daclizumab; Zenapax®)

An antibody to the alpha subunit of the IL-2 receptor of T cells, *e.g*., daclizumab, can be used in the methods and compositions disclosed herein. Daclizumab is a therapeutic humanized monoclonal antibody to the alpha subunit of the IL-2 receptor of T cells. Daclizumab was effective in reducing lesions and improving clinical scores in patients with multiple sclerosis not controlled with interferon (Rose JW et al. (2007). Neurology 69 (8): 785-789).

### Antibody against CD52, e.g., alemtuzumab

Antibodies against CD52, *e.g*., alemtuzumab (currently under further development as Lemtrada®), bind to CD52, which is a protein present on the surface of mature lymphocytes, but not on stem cells. Phase III studies reported positive results comparing alemtuzumab with Rebif® (high-dose subcutaneous interferon beta-la) in the treatment of patients with relapsing-remitting MS (RRMS). Alemtuzumab has been approved in Europe.

### Antibody to CD20, e.g., ocrelizumab

Antibodies against CD20, *e.g*., ocrelizumab, rituximab, ofatumumab, target mature B lymphocytes. Phase 2 clinical studies of rituximab and ocrelizumab in relapse remitting MS have demonstrated a statistically significant reduction in disease activity measured by brain lesions (*e.g*., measured by MRI scans) and relapse rate compared to placebo.

### Inhibitors of dihydroorotate dehydrogenase, e.g., teriflunomide

Inhibitors of dihydroorotate dehydrogenase, *e.g*., teriflunomide, inhibit pyrimidine synthesis. Teriflunomide (also known as A77 1726 or) is an active metabolite of leflunomide. Teriflunomide inhibits rapidly dividing cells, including activated T cells, which are thought to drive the disease process in MS. Teriflunomide was investigated in clinical trials as a medication for treating MS. (Vollmer EMS News (May 28, 2009)).

### Steroids

Steroids, *e.g*., corticosteroid, and ACTH agents can be used to treat acute relapses in relapsing-remitting MS or secondary progressive MS. Such agents include, but are not limited to, Depo-Medrol®, Solu-Medrol®, Deltasone®, Delta-Cortef®, Medrol®, Decadron®, and Acthar®.

One or more of the aforesaid immunomodulatory agents can be used in combination with the reparative agents disclosed herein, as described in more detail below and exemplified by the combination of IFN-b and anti-LINGO-1 Antibody Therapy.

### Therapeutic Methods

Reparative agents, such as LINGO-1 antagonists, can relieve NgR1-mediated inhibition of axonal regeneration and dendritic arborization that normally takes place in CNS neurons. This is beneficial in situations where axonal repair or neurite sprouting is needed in the brain or spinal cord following CNS injury. Spinal cord injury, including partial or complete crush or severance, exemplifies a situation in which axonal repair is needed, but is normally inhibited through operation of the NgR1 pathway. Examples of diseases or disorders in which axonal extension and/or neurite sprouting in the brain can be beneficial include, but are not limited to, stroke, multiple sclerosis (MS), and other neurodegenerative diseases or disorders such as multiple sclerosis, progressive multifocal leukoencephalopathy (PML), encephalomyelitis (EPL), acute disseminated encephalomyelitis (ADEM), central pontine myelolysis (CPM), neuromyelitis optics (NMO), adrenoleukodystrophy, Alexander's disease, Pelizaeus Merzbacher disease (PMZ), periventricular leukomalatia (PVL), Globoid cell Leucodystrophy (Krabbe's disease) and Wallerian Degeneration, optic neuritis (*e.g*., acute optic neuritis), transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, neuropathy, acute ischemic optic neuropathy, vitamin B12 deficiency, isolated vitamin E deficiency syndrome, AR, Bassen-Kornzweig syndrome, Marchiafava-Bignami syndrome, metachromatic leukodystrophy, trigeminal neuralgia, Bell's palsy, spinal cord injury, traumatic glaucoma, essential tremor, osmotic hyponatremia, and all neurological diseases related to neuronal cell death.

LINGO-1 is expressed in oligodendrocytes, and contributes to oligodendrocyte biology. Soluble derivatives of LINGO-1, polynucleotides (e.g. RNAi), as well as certain antibodies which specifically bind to LINGO-1 can act as antagonists to LINGO-1 function in oligodendrocytes, promoting proliferation, differentiation and survival of oligodendrocytes and promoting myelination of neurons *in vitro* and *in vivo.* This can be beneficial for treating or preventing disorders or conditions involving demyelination and dysmyelination. Examples of diseases or disorders in which oligodendrocyte proliferation, differentiation and survival, and/or myelination or remyelination would be beneficial include multiple sclerosis (MS), progressive multifocal leukoencephalopathy (PML), encephalomyelitis (EPL), acute disseminated encephalomyelitis (ADEM), central pontine myelolysis (CPM), adrenoleukodystrophy, neuromyelitis optics (NMO), Alexander's disease, Pelizaeus Merzbacher disease (PMZ), Globoid cell Leucodystrophy (Krabbe's disease), Wallerian Degeneration, optic neuritis (*e.g*., acute optic neuritis), periventricular leukomalatia (PVL), transverse myelitis, amylotrophic lateral sclerosis (ALS), Huntington's disease, Alzheimer's disease, Parkinson's disease, spinal cord injury, traumatic brain injury, post radiation injury, neurologic complications of chemotherapy, stroke, acute ischemic optic neuropathy, vitamin B 12 deficiency, isolated vitamin E deficiency syndrome, AR, Bassen-Kornzweig syndrome, Marchiafava-Bignami syndrome, metachromatic leukodystrophy, trigeminal neuralgia, traumatic glaucoma, osmotic hyponatremia and Bell's palsy.

Accordingly, methods for treating spinal cord injury, diseases or disorders associated with inhibition of neuronal growth in the CNS, diseases or disorders associated with inhibition of oligodendrocyte growth or differentiation, and diseases involving demyelination or dysmyelination of CNS neurons in a subject suffering from such injury or disease or predisposed to contract such disease, are disclosed. The method includes administering to the subject an effective amount of a reparative agent, *e.g.,* a LINGO-1 antagonist, alone or in combination with an immunomodulatory agent.

In one embodiment, the reparative agent, alone or in combination, reduces one or more symptoms of an inflammatory condition of the optic nerve (*e.g.,* optic neuritis, *e.g.,* acute optic neuritis (AON)). AON is an inflammatory disease of the optic nerve that often occurs in multiple sclerosis. AON is caused by inflammatory injury to the optic nerve and presents with visual loss due to edema, inflammation, and damage to the myelin sheath covering the optic nerve and axons. There is significant loss of the retinal nerve fiber layer and retinalganglion cell layer as a result of AON. Current treatment of AON is limited to intravenous treatment with high dose corticosteroids which fasten the resolution of edema, but do not promote central nervous system (CNS) remyelination or provide neuroaxonal protection from CNS inflammatory demyelination. Thus the reparative agents disclosed herein can be used, alone or in combination, to treat such inflammation of the optic nerve.

"Treat," "treatment," and other forms of this word refer to the administration of a combination therapy, alone or in combination with one or more symptom management agents, to a subject, *e.g*., an MS patient, to impede disease progression, to induce remission, to extend the expected survival time of the subject and or reduce the need for medical interventions (*e.g*., hospitalizations). In those subjects, treatment can include, but is not limited to, inhibiting or reducing one or more symptoms such as numbness, tingling, muscle weakness; reducing relapse rate or severity, reducing size or number of sclerotic lesions; inhibiting or retarding the development of new lesions; prolonging survival, or prolonging progression-free survival, and/or enhanced quality of life.

As used herein, unless otherwise specified, the terms "prevent," "preventing" and "prevention" contemplate an action that occurs before a subject begins to suffer from a relapse and/or which inhibits or reduces the severity of the disease.

As used herein, and unless otherwise specified, the terms "manage," "managing" and "management" encompass preventing the progression of disease symptoms in a subject who has already suffered from the disease, and/or lengthening the time that the subject who has suffered from the disease remains in remission. The terms encompass modulating the threshold, development and/or duration of the disease, or changing the way that a patient responds to the disease.

As used herein, and unless otherwise specified, a "therapeutically effective amount" of a compound is an amount sufficient to provide a therapeutic benefit in the treatment or management of the disease, or to delay or minimize one or more symptoms associated with the disease. A therapeutically effective amount of a compound means an amount of therapeutic agent, alone or in combination with other therapeutic agents, which provides a therapeutic benefit in the treatment or management of the disease. The term "therapeutically effective amount" can encompass an amount that improves overall therapy, reduces or avoids symptoms or causes of the disease, or enhances the therapeutic efficacy of another therapeutic agent.

As used herein, and unless otherwise specified, a "prophylactically effective amount" of a compound is an amount sufficient to prevent relapse of the disease, or one or more symptoms associated with the disease, or prevent its recurrence. A prophylactically effective amount of a compound means an amount of the compound, alone or in combination with other therapeutic agents, which provides a prophylactic benefit in the prevention of disease relapse. The term "prophylactically effective amount" can encompass an amount that improves overall prophylaxis or enhances the prophylactic efficacy of another prophylactic agent.

As used herein, the term "patient" or "subject" typically refers to a human (*i.e*., a male or female of any age group, *e.g.,* a pediatric patient (*e.g.,* infant, child, adolescent) or adult patient (*e.g*., young adult, middle-aged adult or senior adult) or other mammal, such as a primate (*e.g*., cynomolgus monkey, rhesus monkey); commercially relevant mammals such as cattle, pigs, horses, sheep, goats, cats, and/or dogs;, that will be or has been the object of treatment, observation, and/or experiment. When the term is used in conjunction with administration of a compound or drug, then the patient has been the object of treatment, observation, and/or administration of the compound or drug.

### Treatment of MS

Multiple sclerosis (MS) is a central nervous system disease that is characterized by inflammation and loss of axons and myelin sheaths.

Subjects having MS can be identified by clinical criteria establishing a diagnosis of clinically definite MS as defined by Poser et al. (1983) Ann. Neurol. 13:227. Briefly, an individual with clinically definite MS has had two attacks and clinical evidence of either two lesions or clinical evidence of one lesion and paraclinical evidence of another, separate lesion. Definite MS may also be diagnosed by evidence of two attacks and oligoclonal bands of IgG in cerebrospinal fluid or by combination of an attack, clinical evidence of two lesions and oligoclonal band of IgG in cerebrospinal fluid. The McDonald criteria can also be used to diagnose MS. (McDonald et al. (2001) Recommended diagnostic criteria for Multiple sclerosis: guidelines from the International Panel on the Diagnosis of Multiple Sclerosis, Ann Neurol 50:121-127); Polman, CH et al. (2005 Dec). Diagnostic criteria for multiple sclerosis: 2005 revisions to the "McDonald Criteria" Annals of Neurology 58 (6): 840-6; Polman, C.H. et al. (2011) Ann. Neurol. 69(2):292-302). The McDonald criteria include the use of MRI evidence of CNS impairment over time to be used in diagnosis of MS, in the absence of multiple clinical attacks. Further updates to the McDonald criteria (Polman et al, Annals of Neurology 2011) allow the diagnosis of MS at the time of first CNS demyelinating episode based on the finding of characteristic MRI lesions. Effective treatment of multiple sclerosis may be evaluated in several different ways. The following parameters can be used to gauge effectiveness of treatment. Two exemplary criteria include: EDSS (extended disability status scale), and appearance of exacerbations on MRI (magnetic resonance imaging).

Exacerbations are defined as the appearance of a new symptom that is attributable to MS and accompanied by an appropriate new neurologic abnormality. In addition, the exacerbation must last at least 24 hours and be preceded by stability or improvement for at least 30 days. Briefly, patients are given a standard neurological examination by clinicians. Exacerbations are mild, moderate, or severe according to changes in a Neurological Rating Scale like, for example, the Scripps Neurological Rating Scale (Sipe et al. (1984) Neurology 34:1368); the EDSS; or by patient reported outcomes (*e.g.,* MSWS-12). An annual exacerbation rate and proportion of exacerbation-free patients are determined.

Therapy can be deemed to be effective using a clinical measure if there is a statistically significant difference in the rate or proportion of exacerbation-free or relapse-free patients between the treated group and the placebo group for either of these measurements. In addition, time to first exacerbation and exacerbation duration and severity may also be measured. A measure of effectiveness as therapy in this regard is a statistically significant difference in the time to first exacerbation or duration and severity in the treated group compared to control group. An exacerbation-free or relapse-free period of greater than one year, 18 months, 20, or 24 months is particularly noteworthy. Clinical measurements include the relapse rate in one and two-year intervals, and a change in EDSS, including time to worsening from baseline of 1.0 unit on the EDSS that persists for three or six months. On a Kaplan-Meier curve, a delay in sustained progression of disability shows efficacy. Other criteria include a change in area and volume of T2 images on MRI, and the number and volume of lesions determined by gadolinium enhanced images.

MRI can be used to measure active lesions using gadolinium-DTPA-enhanced imaging (McDonald et al., Ann. Neurol. 36:14, 1994) or the location and extent of lesions using T2-weighted techniques. Briefly, baseline MRIs are obtained. The same imaging plane and patient position are used for each subsequent study. Positioning and imaging sequences can be chosen to maximize lesion detection and facilitate lesion tracing. The same positioning and imaging sequences can be used on subsequent studies. The presence, location and extent of MS lesions can be determined by radiologists. Areas of lesions can be outlined and summed slice by slice for total lesion area. Three analyses may be done: evidence of new lesions, rate of appearance of active lesions, and percentage change in lesion area (Paty et al., (1993) Neurology 43:665). Improvement due to therapy can be established by a statistically significant improvement in an individual patient compared to baseline or in a treated group versus a placebo group.

Exemplary symptoms associated with multiple sclerosis, which can be treated with the methods described herein or managed using symptom management therapies, include: optic neuritis, decreased visual acuity, diplopia, nystagmus, ocular dysmetria, internuclear ophthalmoplegia, movement and sound phosphenes, afferent pupillary defect, paresis, monoparesis, paraparesis, hemiparesis, quadraparesis, plegia, paraplegia, hemiplegia, tetraplegia, quadraplegia, spasticity, dysarthria, muscle atrophy, spasms, cramps, hypotonia, clonus, myoclonus, myokymia, restless leg syndrome, footdrop, dysfunctional reflexes, paraesthesia, anaesthesia, neuralgia, neuropathic and neurogenic pain, L'hermitte's, proprioceptive dysfunction, trigeminal neuralgia, ataxia, intention tremor, dysmetria, vestibular ataxia, vertigo, speech ataxia, dystonia, dysdiadochokinesia, frequent micturation, bladder spasticity, flaccid bladder, detrusor-sphincter dyssynergia, erectile dysfunction, anorgasmy, frigidity, constipation, fecal urgency, fecal incontinence, depression, cognitive dysfunction, dementia, mood swings, emotional lability, euphoria, bipolar syndrome, anxiety, aphasia, dysphasia, fatigue, Uhthoffs symptom, gastroesophageal reflux, and sleeping disorders.

Each case of MS displays one of several patterns of presentation and subsequent course. Most commonly, MS first manifests itself as a series of attacks followed by complete or partial remissions as symptoms mysteriously lessen, only to return later after a period of stability. This is called relapsing-remitting MS (RRMS). Primary-progressive MS (PPMS) is characterized by a gradual clinical decline with no distinct remissions, although there may be temporary plateaus or minor relief from symptoms. Secondary-progressive MS (SPMS) begins with a relapsing-remitting course followed by a later progressive course independently of relapses. Rarely, patients may have a progressive-relapsing (PRMS) course in which the disease takes a progressive path punctuated by intermittent acute attacks. PPMS, SPMS, and PRMS are sometimes lumped together and called chronic progressive MS.

A few patients experience malignant MS, defined as a swift and relentless decline resulting in significant disability or even death shortly after disease onset. This decline may be arrested or decelerated by determining the likelihood of the patient to respond to a therapy early in the therapeutic regime and switching the patient to an agent that they have the highest likelihood of responding to.

### Combination Therapy

The invention discloses combined administration of an immunomodulatory agent, *e.g.,* an IFN-β agent, *e.g.,* Avonex®; and a reparative agent, *e.g.,* an anti-LINGO-1 antibody, for treatment of a demyelinating disorder, *e.g.,* MS.

The agents, *e.g*., pharmaceutical compositions including the agents, can be administered concurrently with, prior to, or subsequent to, one or more other additional therapies or therapeutic agents. In general, each agent can be administered at a dose and/or on a time schedule determined for that agent. In will further be appreciated that the additional therapeutic agent utilized in this combination can be administered together in a single composition or administered separately in different compositions. The particular combination to employ in a regimen will take into account compatibility of the pharmaceutical composition with the additional therapeutically active agent and/or the desired therapeutic effect to be achieved. In general, it is expected that additional therapeutic agents utilized in combination be utilized at levels that do not exceed the levels at which they are utilized individually. In some embodiments, the levels utilized in combination will be lower than those utilized individually.

Treatment of a subject with a disease with a reparative agent can be combined with one or more immunomodulatory agents. Exemplary immunomodulatory agents are described herein and include, but are not limited to, an IFN-β 1 molecule; a polymer of glutamic acid, lysine, alanine and tyrosine, *e.g*., glatiramer; an antibody or fragment thereof against alpha-4 integrin, *e.g.,* natalizumab; an anthracenedione molecule, *e.g.,* mitoxantrone; a fingolimod, *e.g.,* FTY720; a dimethyl fumarate, *e.g.,* an oral dimethyl fumarate; an antibody to the alpha subunit of the IL-2 receptor of T cells, *e.g.,* daclizumab; an antibody against CD52, *e.g*., alemtuzumab; an inhibitor of a dihydroorotate dehydrogenase, *e.g*., teriflunomide; a corticosteroid; and an anti-CD20 antibody, *e.g*., ocrelizumab.

In one embodiment, a combination of Avonex® and anti-LINGO-1 antibody therapy is administered. In certain embodiments, an anti-LINGO-1 antibody can be administered once about every 4 weeks (plus or minus about 5 days) by intravenous (IV) infusion in addition to once weekly Avonex® intramuscular (IM) injections. Anti-LINGO-1 antibody treatment doses can include: IV infusions of: 3mg/kg; or 10mg/kg; or 30mg/kg; or 50mg/kg or 100 mg/kg; concurrent with once-weekly Avonex® IM injections.

In one embodiment, 3 mg/kg IV infusion once every 4 weeks of an anti-LINGO-1 antibody was selected. This regimen is expected to yield a mean average serum concentration similar to rat serum EC50 in the spinal cord lysolecithin model (adjusted for -0.1% brain penetration). Additional dosing regimens, 10 mg/kg and 30 mg/kg can also be administered. These 2 dosing regimens are expected to yield mean average serum concentrations approximately 1.2-fold and 3.7-fold higher than the rat serum EC90 (adjusted for ∼0.1% brain penetration), respectively.

In certain embodiments, the immunomodulatory agent is an IFN-β 1 molecule and is administered intravenously, subcutaneously or intramuscularly. For example, the IFN-β 1 molecule can be administered at one or more of:
(i) at 20-45 microgram (*e.g.,* 30 microgram), *e.g.,* once a week via intramuscular injection;
(ii) at 20-30 microgram (*e.g.,* 22 microgram), *e.g.,* three times a week, or at 40-50 micrograms (*e.g.,* 44 micrograms), *e.g.,* three times a week, via subcutaneous injection; or
(iii) in an amount of between 10 and 50 µg intramuscularly, *e.g*., three times a week, or every five to ten days, *e.g.,* once a week.

In one embodiment, Avonex® is administered at 30 microgram once a week via intramuscular injection. Following titration when applicable, Avonex® can be administered by IM injection following dosage and administration schedules known in the art.

In one embodiment, the IFN-β agent, *e.g*., Avonex®, is administered by an injection device, *e.g.,* an autoinjection device or pen.

In one embodiment, the anti-LINGO-1 antibody molecule is supplied as a liquid drug product containing 50 mg/mL BIIB033 (also referred to herein as an antibody molecule having a VH that includes the amino acid sequence of SEQ ID NO: 275 and a VL that includes the amino acid sequence of SEQ ID NO: 276), 10 mM sodium citrate, 160 mM L-arginine hydrochloride (pH 6.5), and 0.03% (weight per volume) polysorbate 80. The anti-LINGO-1 antibody molecule can be administered by IV infusion following saline dilution.

In one embodiment, the immunomodulatory agent is Avonex®, which is is formulated as a sterile clear liquid for IM injection. Each 0.5 mL of Avonex in a prefilled glass syringe contains 30 mcg of interferon β-1a. Other ingredients include sodium acetate trihydrate, glacial acetic acid, arginine hydrochloride, and polysorbate 20 in Water for Injection at a pH of approximately 4.8. The immunomodulatory agent, *e.g.,* Avonex®, can be administered by any suitable means, *e.g*., a pen or other device.

### Symptom management

Treatment of a subject with a combination therapy described herein can be combined with one or more of the following therapies often used in symptom management of subjects having MS: Tegretol® (carbamazepine), Epitol® (carbamazepine), Atretol® (carbamazepine), Carbatrol® (carbamazepine), Neurontin® (gabapentin), Topamax® (topiramate), Zonegran® (zonisamide), Dilantin® (phenytoin), Norpramin® (desipramine), Elavil® (amitriptyline), Tofranil® (imipramine), Imavate® (imipramine), Janimine® (imipramine), Sinequan® (doxepine), Adapin® (doxepine), Triadapin® (doxepine), Zonalon® (doxepine), Vivactil® (protriptyline), Marinol® (synthetic cannabinoids), Trental® (pentoxifylline), Neurofen® (ibuprofen), aspirin, acetaminophen, Atarax® (hydroxyzine), Prozac® (fluoxetine), Zoloft® (sertraline), Lustral® (sertraline), Effexor XR® (venlafaxine), Celexa® (citalopram), Paxil®, Seroxat®, Desyrel® (trazodone), Trialodine® (trazodone), Pamelor® (nortriptyline), Aventyl® (imipramine), Prothiaden® (dothiepin), Gamanil® (lofepramine), Parnate® (tranylcypromine), Manerix® (moclobemide), Aurorix® (moclobemide), Wellbutrin SR® (bupropion), Amfebutamone@ (bupropion), Serzone® (nefazodone), Remeron® (mirtazapine), Ambien® (zolpidem), Xanax® (alprazolam), Restoril® (temazepam), Valium® (diazepam), BuSpar® (buspirone), Symmetrel® (amantadine), Cylert® (pemoline), Provigil® (modafinil), Ditropan XL® (oxybutynin), DDAVP® (desmopressin, vasopressin), Detrol® (tolterodine), Urecholine® (bethane), Dibenzyline® (phenoxybenzamine), Hytrin® (terazosin), Pro-Banthine® (propantheline), Urispas® (hyoscyamine), Cystopas® (hyoscyamine), Lioresal® (baclofen), Hiprex® (methenamine), Mandelamine® (metheneamine), Macrodantin® (nitrofurantoin), Pyridium® (phenazopyridine), Cipro® (ciprofloxacin), Dulcolax® (bisacodyl), Bisacolax® (bisacodyl), Sani-Supp® (glycerin), Metamucil® (psyllium hydrophilic mucilloid), Fleet Enema® (sodium phosphate), Colace® (docusate), Therevac Plus®, Klonopin® (clonazepam), Rivotril® (clonazepam), Dantrium® (dantrolen sodium), Catapres® (clonidine), Botox® (botulinum toxin), Neurobloc® (botulinum toxin), Zanaflex® (tizanidine), Sirdalud® (tizanidine), Mysoline® (primidone), Diamox® (acetozolamide), Sinemet® (levodopa, carbidopa), Laniazid® (isoniazid), Nydrazid® (isoniazid), Antivert® (meclizine), Bonamine® (meclizine), Dramamine® (dimenhydrinate), Compazine® (prochlorperazine), Transderm® (scopolamine), Benadryl® (diphenhydramine), Antegren® (natalizumab), Campath-1H® (alemtuzumab), Fampridine® (4-aminopyridine), Gammagard® (IV immunoglobulin), Gammar-IV® (IV immunoglobulin), Gamimune N® (IV immunoglobulin), Iveegam® (IV immunoglobulin), Panglobulin® (IV immunoglobulin), Sandoglobulin® (IV immunoglobulin), Venoblogulin® (IV immunoglobulin), pregabalin, ziconotide, Badofen and AnergiX-MS®.

### Clinical Tests/Assessments for the Evaluation of Combination Avonex® and Anti-LINGO-1 Antibody Therapy

Efficacy endpoints of the therapy in any subject can be evaluated using tests and assessments known in the art. For example, for an RRMS patient, the subject can be evaluated by acquiring the subject's status using EDSS. In other embodiments where the subject has a progressive form of MS, *e.g.,* SPMS or PPMS, the subject can be evaluated by obtaining a measure of upper and/or lower extremity function, and/or a measure of ambulatory function, *e.g*., short distance ambulatory function, in addition to acquiring the subject's status using EDSS. In certain embodiments, an assessment of lower extremity ambulatory function (*e.g*., Timed Walk of 25 Feet (T25FW)), and/or an assessment of upper extremity function (*e.g*., 9 Hole Peg Test (9HP)) can be performed.

Additional exemplary efficacy endpoints that can be evaluated include one or more of the following.

### Efficacy Endpoints

### Exemplary Primary Endpoints

Subjects can be evaluated for confirmed improvement of neurophysical and/or cognitive function over treatment as measured by a composite endpoint comprising the Expanded Disability Status Scale (EDSS), Timed 25-Foot Walk (T25FW), 9-Hole Peg Test (9HPT), and (3-Second) Paced Auditory Serial Addition Test (PASAT). Improvement on neurophysical and/or cognitive function can be defined as at least 1 of the following:
a) A ≥1.0 point decrease in EDSS from a baseline score of ≤6.0 (decrease sustained for 3 months or greater);
b) A ≥15% improvement from baseline in T25FW (improvement sustained for 3 months or greater);
c) A ≥15% improvement from baseline in 9HPT (improvement sustained for 3 months or greater); and
d) A ≥15% improvement from baseline in PASAT (improvement sustained for 3 months or greater).

### Exemplary Secondary Endpoints

Subjects can be evaluated for confirmed worsening of neurophysical and/or cognitive function and/or disability treatment as measured by a composite endpoint of the EDSS, T25FW, 9HPT, and PASAT. Progression of disability or worsening of neurophysical and/or cognitive function is defined as at least 1 of the following:
a) A ≥1.0 point increase in EDSS from a baseline score of ≤5.5 or a ≥0.5 point increase from a baseline score equal to 6.0 (increase sustained for 3 months or greater);
b) A ≥15% worsening from baseline in T25FW (worsening sustained for 3 months or greater);
c) A ≥15% worsening from baseline in 9HPT (worsening sustained for 3 months or greater); and
d) A ≥15% worsening from baseline in PASAT (worsening sustained for 3 months or greater).

### Additional Clinical Efficacy Endpoints

In certain embodiments, subjects can be evaluated using additional clinical measures, including:
a) A change from baseline in cognitive function as measured by an MS cognitive composite endpoint comprising 2 tests of processing speed (the PASAT and the Symbol-Digit Modalities Test [SDMT]) and 2 tests of memory and learning (the Selective Reminding Test [SRT] for verbal memory and the Brief Visuospatial Memory Test-Revised [BVMT-R] for visual memory);
b) Severity of clinical relapses as determined by the Scripps Neurological Rating Scale (SNRS); and/or
c) A ≥10% (*e.g.,* ≥15%, >20% , ≥30%) worsening from baseline in Six Minute Walk (6MW) walking time (worsening sustained for 3 months or greater).

### Exemplary MRI Efficacy Endpoints

Analysis of brain MRI focused on measures of repair at the focal and diffuse levels with both new and preexisting lesions can include one or more of:
(i) Analysis of new brain lesions:
   a) Percentage Gd lesion volume with increased and decreased magnetization transfer ratio (MTR);
   b) A change from onset in new Gd lesion mean MTR relative to the normal appearing white matter (NAWM) with lesions per subject as the unit of measure;
   c) A change from onset in MTR signal for voxels per scan whose MTR drops below the normal value (new MTR lesions) with subject as the unit of measure;
   d) A change from onset in new Gd lesion radial diffusivity with subject as the unit of measure;
   e) A change from onset in radial diffusivity for voxels per scan whose MTR drops below the normal value (new MTR lesions) with subject as the unit of measure; or
   f) Percentage conversion from new Gd brain lesions to chronic black hole with chronic black holes defined as T1 hypointensity still visible after at least 6 months from onset.
(ii) Analysis of pre-existing brain lesions (lesions that are present at baseline scan):
   a. A change in MTR from baseline for abnormal T1 volume;
   b. A change in MTR from baseline for abnormal T2 volume;
   c. A change in MTR from baseline for abnormal T2 volume not associated with T1 hypointensity;
   d. A change in diffusion tensor imaging (DTI) from baseline for abnormal T1 volume;
   e. A change in DTI from baseline for abnormal T2 volume; or
   f. A change in DTI from baseline for abnormal T2 volume not associated with T1 hypointensity.
(iii) Analysis of diffuse brain MRI metrics:
   a) Percentage brain volume change;
   b) A change from baseline in cerebral cortical brain volume;
   c) A change from baseline in thalamic volume; or
   d) A change from baseline in whole brain radial diffusivity.

### Exemplary Patient-Reported Outcomes (PROs) Efficacy Endpoints

In certain embodiments, subjects can be evaluated by patient reported outcomes, including one or more of:
a) 12-Item Multiple Sclerosis Walking Scale (MSWS-12).
b) ABILHAND 56-Item Questionnaire.
c) 29-Item Multiple Sclerosis Impact Scale (MSIS-29).
d) The Short Form (36) Health Survey (SF-36).
e) MSNQ-informant and MSNQ-patient

### Efficacy Endpoint Analysis

### General Methods of Analysis

Summary statistics may be presented. For continuous endpoints, the summary statistics may generally include: the number of subjects randomized or dosed; or the number of subjects with data, mean, SD, median, and range. For categorical endpoints, the summary statistics may generally include: the number of subjects randomized or dosed; the number of subjects with data, or the percent of subjects with data in each category.

### Primary Endpoint Analyses

The primary efficacy endpoint can include the percentage of subjects with confirmed clinical improvement in 1 or more of the components of the composite endpoint (EDSS, T25FW, 9HPT, or PASAT). The percentage of confirmed improvers can be presented by treatment groups, and the data analyzed by a logistic regression model. Time to confirmed improvement may be analyzed using the Cox proportional hazards model. Baseline EDSS, T25FW, 9HPT (both dominant and non-dominant hands), PASAT, and stratification factors may be included in both logistic regression and Cox models as covariates. If 2 baseline EDSS assessments are performed, the higher EDSS score can be used for analysis. MRI activity may be explored as potential covariates as well.

### Secondary Endpoint Analyses

The secondary efficacy endpoint may include the percentage of subjects with confirmed clinical worsening in 1 or more of the components of the composite endpoint (EDSS, T25FW, 9HPT, or PASAT). The percentage of confirmed worseners can be presented by treatment groups, and the data analyzed by a logistic regression model. Time to confirmed worsening may be analyzed using the Cox proportional hazards model. Baseline EDSS, T25FW, 9HPT (both dominant and non-dominant hands), PASAT, and stratification factors may be included in both logistic regression and Cox models as covariates. If 2 baseline EDSS assessments are performed, the higher EDSS score can be used for analysis. MRI activity may be explored as potential covariates as well.

### Exploratory Endpoint Analyses

The exploratory endpoints may include clinical metrics, MRI metrics, and PRO variables. They can be summarized by presenting summary statistics for continuous variables or frequency distributions for categorical variables. The statistical methods used will depend on the nature of the variables. Binary variables can be analyzed by using a logistic regression model; continuous variables can be analyzed by using the analysis of covariance model, adjusting for the corresponding baselines and stratification factors. Time-to-event variables can be analyzed using the Cox proportional hazards regression model, by adjusting for the corresponding baselines and stratification factors. Count variables can be analyzed by a Negative Binomial regression model or a Wilcoxon rank-sum test.

### Ambulatory Assessments

### T25FW

The T25FW is a timed walk of 25 feet. The T25W is a measure of quantitative ambulatory capacity over a short distance that is responsive to deterioration mostly for subjects who are very disabled, *e.g*., EDSS steps 6-6.5. It can be used as quantitative measure of lower extremity function. Broadly, the patient is directed to one end of a clearly labeled 25-foot course and is instructed to walk 25 feet as quickly as possible, but safely. The task can be immediately administered again by having the patient walk back the same distance. Patients may use assistive devices when completing the T25W. A time limit of 3 minutes to complete the test is usually used. The test is discontinued if the patient cannot complete Trial 2 of the T25W after a 5 minute rest period, or if the patient cannot complete a trial in 3 minutes.

### 9HP

The 9HP is a 9-hole peg test. It is a quantitative measure that captures a clinically important aspect of upper extremity (*e.g*., arm and hand) function that is not measured by the EDSS or the T25FW. Unlike the EDSS and the T25FW, the 9HP is responsive across a wide EDSS range. Broadly, a patient is asked to pick up 9 pegs one at a time, using their hands only, and put the pegs into the holes on a peg board as quickly as possible until all of the holes are filled. The patient must then, without pausing, remove the pegs one at a time and return them to the container as quickly as possible. Both the dominant and non-dominant hands are tested twice (two consecutive trials of the dominant hand, followed immediately by two consecutive trials of the non-dominant hand). A time limit of 5 minutes to complete the test is usually used. The test is discontinued if the patient cannot complete one trial of the 9HP test in 5 minutes; if the patient cannot complete a trial with his or her dominant hand within 5 minutes, the patient is usually instructed to move onto the trials with the non-dominant hand.

### 6MW

The 6 minute walking test (6MW) is used to assess walking distance. Broadly, the patient is asked to walk the fastest speed possible without physical assistance for 6 minutes and the distance is measured. Assistive devices can be used but should be kept consistent and documented from test to test. The patient should walk continuously if possible, but the patient can slow down to stop or rest during the test.

### SNRS

The Scripps Neurological Rating Scale (SNRS) measures several parameters, including, mentation and mood; eyes and related cranial nerves, *e.g*., visual acuity, visual fields, eye movements, nystagmus; lower cranial nerves; motor function in each extremity, *e.g.,* right upper, left upper, right lower, left lower; deep tendon reflexes, *e.g.,* upper extremities, lower extremities; Babinski sign, *e.g.,* left side, right side; sensory function in each extremity, *e.g*., right upper, left upper, right lower, left lower; cerebellar signs, *e.g.,* upper extremities, lower extremities; and gait trunk balance, *e.g.,* special category for autonomic dysfunction, *e.g*., bladder dysfunction, sexual dysfunction.

### EDSS

As described above, the EDSS is based on a standardized neurological examination, focusing on the symptoms that occur frequently in MS. The EDSS assess the seven functional systems: visual, brainstem, pyramidal, cerebellar, sensory, bowel/bladder and cerebral; through neurological examination. In addition the EDSS also includes an assessment of walking range. Based on the functional system scores and the walking range, an EDSS step is determined. The range of the EDSS includes 19 steps from 0 to 10, with EDSS step 0 corresponding to a completely normal examination and EDSS step 10 to death due to MS. For EDSS ratings between 0 and 4, the scale relies mainly on the scores of the individual FS. For ratings over 4, the EDSS is primarily determined by the ability and range of walking.

### Patient Reported Outcome Assessments

### MSWS-12

The Multiple Sclerosis Walking Scale-12 (MSWS-12) test is a self rated measure of walking ability. The test contains 12 questions with Likert-type responses, describing the impact of MS on walking. The questions were generated from 30 MS patient interviews, expert opinions, and literature reviews.

### ABILHAND 56-Item Questionnaire

The ABILHAND 56-Item Questionnaire is a measure of manual ability designed to measure a patient's experience of problems in performing everyday tasks such as feeding, dressing, or managing tasks. The ABILHAND contains 56 unbiased and bimanual activities, which the patients are asked to judge on a four-level scale: 0=impossible, 1=very difficult, 2=difficult, 3=easy.

### MSIS-29

The Multiple Sclerosis Impact Scale 29 (MSIS-29) is a 29 item self report rating scale which measures physical and psychological parameters of MS. Three of the items deal with limited abilities, and the remaining 26 items are related to being impacted by symptoms or consequences of disease. Twenty of the items refer to physical function. Responses use a 5 point Likert scale range from 1 to 5.

### SF-36

The short form 36 (SF-36) test measures overall health related quality of life. The SF-36 is a structured, self report questionnaire that the patient can generally complete with little to no intervention from a physician. There is no single overall score for the SF-36, instead it generates 8 subscales and two summary scores. The 8 subscales include physical functioning, role limitations due to physical problems, bodily pain, general health perceptions, vitality, social functioning, role limitations due to emotional problems, and mental health. The two summary scores include a physical component summary and a mental health component summary.

### Cognitive Test Assessments

Several cognitive test instruments can be used to determine the value of the composite parameter, as follows.

### Symbol Digit Modalities Test (SDMT)

The SDMT is a test that evaluates processing speed and working memory in which the subject is given 90 seconds to pair specific numbers with given geometric figures based on a reference key. It is modeled after the Digit Symbol or Coding Tasks tests, which have been included in the Wechsler intelligence scales for many years (*e.g.,* Wechsler et al. (1974) Manual for the Wechsler Intelligence Scale for Children-Revised. New York: Psychological Corporation; Wechsler et al. (1981) WAIS-R Manual. New York: Psychological Corporation). Recognizing the limitations some patients have with manual dexterity, Rao and colleagues modified the SDMT to include only an oral response (Rao et al. (1991) Neurology 41: 685-691). In this oral SDMT selected in the present invention, participants are presented with an 8.5 x 11 inch sheet that contains the numbers and symbols to be processed. The top row of stimuli includes nine symbols, each of which is paired with a single digit in the key. The remainder of the page has a pseudo-randomized sequence of these symbols, and the participant's task is to respond orally with the digit associated with each of the symbols as quickly as possible. The score is the total number of correct matches (out of 110) made by the subject within the 90 second time frame.

Good test-retest reliability (r = 0.93 - 0.97, p < .001) has been established in MS subjects (Benedict et al. (2006) Journal of the International Neuropsychological Society 12: 549-558; Benedict et al. (2008) Multiple Sclerosis 14: 940-946). Good discriminative validity for distinguishing between MS patients and normal controls (d= 1.0 - 1.5, p < .001) (see *e.g.,* Deloire et al. (2005) Journal of Neurology, Neurosurgery & Psychiatry 76: 519-526; Benedict et al. (2006) Journal of the International Neuropsychological Society 12: 549-558; Houtchens et al. (2007) Neurology 69: 113-123; Strober et al. (2009) Multiple Sclerosis 15: 1077-1084; Parmenter et al. (2010) J Int Neuropsychol Soc 16: 6-16) and for distinguishing between RRMS and SPMS patients (d= 0.8, p < 0.001) (see Benedict et al. (2006) Archives of Neurology 63: 1301-1306) has also been confirmed. In addition, correlations between performance and brain MRI have also been documented (see *e.g.,* Benedict et al. (2007) Multiple Sclerosis 13: 722-730; Houtchens et al. (2007) Neurology 69: 113-123; Tekok-Kilic et al. (2007) NeuroImage 36: 1294-1300).

### Paced Serial Addition Test (PASAT)

First developed by Gronwall *et al.* to assess patients recovering from concussion, the PASAT requires patients to monitor a series of 61 audiotaped digits while adding each consecutive digit to the one immediately preceding it (Gronwall et al. (1977) Perceptual and Motor Skills 44: 367-373). The PASAT requires both rapid information processing and simultaneous allocation of attention to two tasks, as well as reasonably intact calculation ability. In its original format, the PASAT was administered at four inter-stimulus intervals (2.4 seconds, 2.0 seconds, 1.6 seconds, and 1.2 seconds). The number of inter-stimulus intervals and presentation rates were subsequently modified by Rao and colleagues for use with MS patients to 3.0 seconds and 2.0 seconds (Rao et al. (1991) A Manual for the Brief, Repeatable Battery of Neuropsychological Tests in Multiple Sclerosis: National Multiple Sclerosis Society; Rao et al. (1991) Neuropsychological Screening Battery for Multiple Sclerosis: National Multiple Sclerosis Society; Rao et al. (1991) Neurology 41: 685-691; Rao et al. (1991) Neurology 41: 692-696).

This latter version of the test was selected to be a component of the MS Functional Composite (MSFC) and the MACFIMS battery (Benedict et al. (2002) Clinical Neuropsychologist 16: 381-397). Test-retest reliability in MS populations ranges from r= 0.78 to 0.93 (Benedict et al. (2006) Journal of the International Neuropsychological Society 16: 228-237; Drake et al. (2010) Multiple Sclerosis 16: 228-237). Good discriminative validity for distinguishing between MS patients and normal controls (d= 0.5 - 0.7, p < 0.001 to 0.34) (Deloire et al. (2005) Journal of Neurology, Neurosurgery & Psychiatry 76: 519-526; Benedict et al. (2006) Journal of the International Neuropsychological Society 12: 549-558; Houtchens et al. (2007) Neurology 69: 113-123; Strober et al. (2009) Multiple Sclerosis 15: 1077-1084; Parmenter et al. (2010) J Int Neuropsychol Soc 16: 6-16; Drake et al. (2010) Multiple Sclerosis 16: 228-237) and for distinguishing between RRMS and SPMS patients (d= 0.5, p < 0.002) (Benedict et al. (2006) Archives of Neurology 63: 1301-1306) has been confirmed. The PASAT score of interest is the total number of correct responses at each presentation rate. Two alternate forms of the Rao version of the PASAT are available (PASAT 3" and PASAT 2") and were selected in the current invention. In the PASAT 3", the stimulus is presented every 3 seconds, where as in the PASAT 2", the stimulus is presented every 2 seconds.

### Selective Reminding Test (SRT)

The SRT was first developed by Buschke *et al.* (see Buschke et al. (1974) Neurology 24: 1019-1025) who conducted research in the area of anterograde amnesia. Rather than ask patients to recall an entire word list on each successive learning trial, the experimenter only repeated words not recalled on each successive learning trial. Subsequently, several memory investigators developed normative data for the test, and alternate forms. Note, the original versions were based on a form of the test using 15 words and 12 learning trials. Such an administration is arduous and time consuming, and therefore there has been much interest in shorter forms of the SRT. The administration procedure widely used in MS research is a six-trial form developed by Rao *et al.* (see *e.g.,* Rao et al. (1991) A Manual for the Brief, Repeatable Battery of Neuropsychological Tests in Multiple Sclerosis: National Multiple Sclerosis Society; Rao et al. (1991) Neuropsychological Screening Battery for Multiple Sclerosis: National Multiple Sclerosis Society; Rao et al. (1991) Neurology 41: 685-691; Rao et al. (1991) Neurology 41: 692-696). This six-trial format is utilized in the current invention. A number of different versions of SRT word lists exist. Hannay and Levin's word lists for adults, test forms 1 and 3, are utilized in the current invention (Hannay et al. (1985) J Clin Exp Neuropsychol. 7: 251-263). Discriminative validity of the SRT has been established in several studies, with SRT discriminating between MS subjects and normal controls d = 0.6 to d= 1.0 (see *e.g.,* Rao et al. (1991) A Manual for the Brief, Repeatable Battery of Neuropsychological Tests in Multiple Sclerosis: National Multiple Sclerosis Society; Deloire et al. (2005) Journal of Neurology, Neurosurgery & Psychiatry 76: 519-526; Strober et al. (2009) Multiple Sclerosis 15: 1077-1084). It has also been shown that SRT findings can be associated with ventricular enlargement as seen on brain MRI (R² = 0.14; p=0.05) (Christodoulou et al. (2003) Neurology 60: 1793-1798).

### Brief Visuospatial Memory Test - Revised (BVMT-R)

The BVMT-R is based on an initial effort to develop an equivalent alternate form visual memory test along the lines of the visual reproduction subtest from the Wechsler Memory Scale (Benedict et al. (1993) Neuropsychological Rehabilitation 3: 37-51; Benedict et al. (1995) Clinical Neuropsychologist 9: 11-16; Wechsler et al. (1987) Wechsler Memory Scale-Revised Manual. New York: Psychological Corporation). Initially, the BVMT included just a single exposure to a one-page presentation of six visual designs. The revised version includes three 10-second exposures to the stimulus (Benedict et al. (1997) Brief Visuospatial Memory Test - Revised: Professional Manual. Odessa, Florida: Psychological Assessment Resources, Inc.; Benedict et al. (1996) Psychological Assessment 8: 145-153). After each exposure, the subject is asked to reproduce the matrix using a pencil on a blank sheet of paper. There are rigid scoring criteria for accuracy and location. After a 25 minute delay, the patient is asked to reproduce the information again without another exposure. Finally a yes/no recognition task is presented. The BVMT-R has excellent reproducibility, with test-retest reliability ranging from r=0.85 to r=.91 (Benedict et al. (1996) Psychological Assessment 8: 145-153; Benedict et al. (2005) Journal of the International Neuropsychological Society 11: 727-736); as well as good discriminative validity between MS and normal control subjects (d= 0.9, p<0.) (Strober et al. (2009) Multiple Sclerosis 15: 1077-1084; Parmenter et al. (2010) J Int Neuropsychol Soc 16: 6-16) and RRMS and SPMS patients (d=0.6, p<0.001) (Benedict et al. (2006) Archives of Neurology 63: 1301-1306). Predictive validity, in the form of correlation between BVMT-R performance and brain MRI findings, has also been established. Further, there is extensive research showing that all 6 forms of the test are of equivalent difficulty. Variables of interest in the current invention are the Total Learning and Delayed Recall scores.

This invention is further illustrated by the following examples which should not be construed as limiting. The contents of all references, figures, sequence listing, patents and published patent applications cited throughout this application are hereby incorporated by reference.

### Exemplification

### Example 1. LINGO-1 antagonism reduces morbidity and mortality from MOG-EAE in mice and promotes axonal protection in the inflamed optic nerve.

Acute optic neuritis (AON) is an inflammatory disease of the optic nerve that often occurs in multiple sclerosis. AON is caused by inflammatory injury to the optic nerve and presents with visual loss due to edema, inflammation, and damage to the myelin sheath covering the optic nerve and axons. As a result of AON, there is often a significant loss of the retinal nerve fiber layer and retinal ganglion cell layer. Current treatment of AON is limited to intravenous treatment with high dose corticosteroids which fasten the resolution of edema but do not promote central nervous system (CNS) remyelination or provide neuroaxonal protection from CNS inflammatory demyelination.

Animal models for the study of optic neuritis include the rat and mouse experimental autoimmune encephalomyelitis (EAE) models; in which EAE induction results in the development of optic nerve neuritis. In the present example, the effects of LINGO-1 antagonism were analyzed using an EAE mouse model. Briefly, EAE was induced in C57BL/6 male and female mice at 8-12 weeks of age by subcutaneous injection of 250 µl into both flanks at the tail base with 125 µg of MOG 35-55 emulsified in complete Freund's adjuvant (CFA) followed by intravenous injection of 300 ng pertussis toxin in phosphate buffered saline (PBS) immediately afterwards and three days later. Efficacy on motor system impairment was measured using EAE severity scores on a range from 0-7.

Two separate cohorts of 14 mice each were blindly treated with intraperitoneal injections of 10 mg/kg of an antagonistic anti-LINGO-1 mouse antibody or a control monoclonal antibody (N=7 per treatment group per cohort). Mice were treated on 4 different occasions every three days starting on day six post EAE induction and prior to the onset of clinical disease (days 6, 9, 12, and 15). Mice were sacrificed at EAE-disease peak.

The optic nerve was imaged once at EAE-disease peak using diffusion tensor imaging (DTI) on a Bruker 4,7T MRI system. MRI images were acquired with the following parameters: TR of 1 s, TE of 30 ms, Δ of 10 ms, 8 NEX, slice thickness 0.5 mm, field of view 2x2 cm² , data matrix 256x128. B values of 0s/mm² (non-diffusion weighted image) and 700 s/mm² for one parallel and one perpendicular diffusion sensitizing gradient directions (Wu, Butzkueven et al. (2007) Neuroimage 37: 13138-1147) were employed.

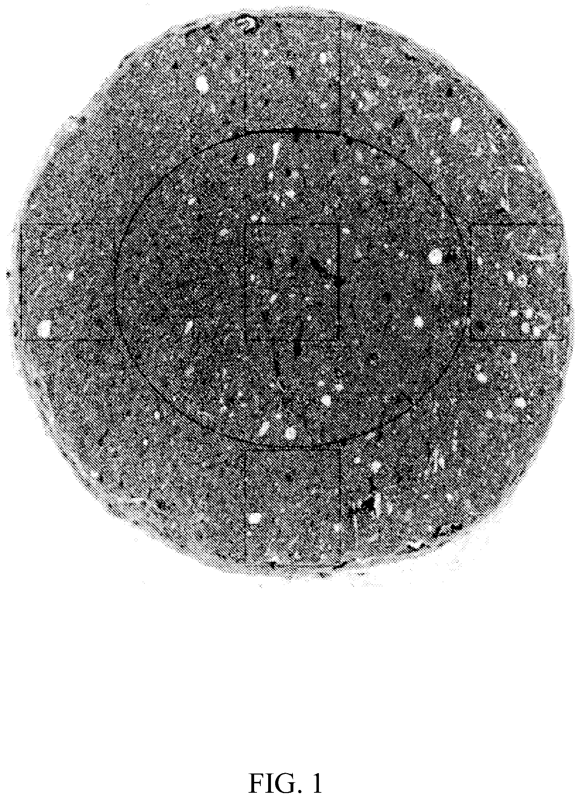

Immediately following MRI analysis, mice were euthanized with pentobarbital, perfused with PBS, fixed with 4% paraformaldhyde (PFA) in 0.1M PBS, and the optic nerves removed and post fixed in 4% PFA solution containing 2.5% glutaraldhyde and 0.1M sodium cacodylate buffer and processed for electron microscopy. Whole prechiasmal cross-sectional optic nerve images were taken at 10X and 100X magnification and merged on Photoshop CS3 software (Adobe Systems Incorporated, San Jose, CA, USA). Five pre-chiasmal cross-sectional optic nerve ROIs (regions of interest) were chosen for analysis, four peripheral and one central, each measuring approximately 3600 µm² (FIG. 1). Analysis was conducted using image Pro Plus software (Media Cybernetics, Inc., Rockville, MD, USA) to assess axonal number, axonal area, and axoplasmal area of each identified axon on each ROI. The periphery of the nerve contained the majority of the inflammatory infiltrate. The heavily infiltrated peripheral area and the central nerve areas were assessed separately for each nerve.

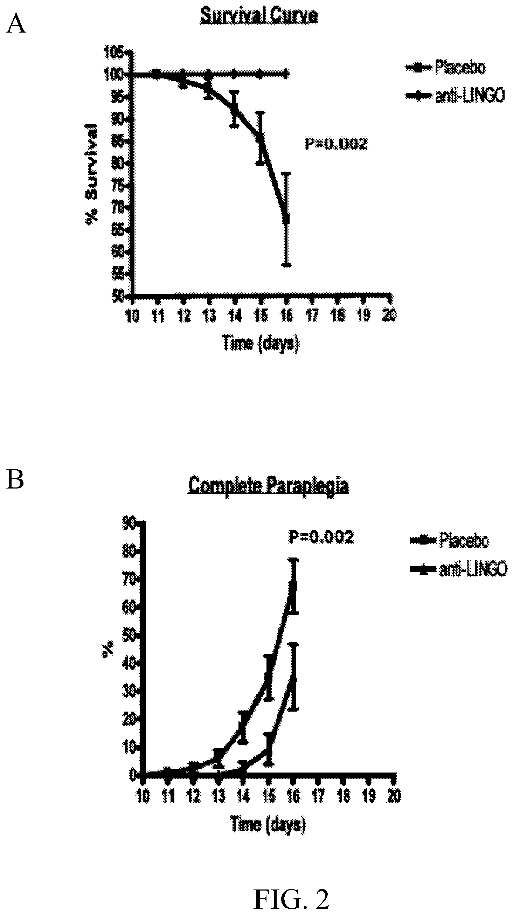

There was no EAE mortality observed in the anti-LINGO antibody treated mice, whereas 5/14 placebo treated mice had to be euthanized due to EAE severity (FIG.2A). In addition, a significantly lower proportion of anti-LINGO-1 antibody treated mice reached complete hindlimb paralysis (paraplegia) (grade 5 disease severity) (4/14 mice) compared to placebo-treated mice (7/14 mice) (FIG.2B).

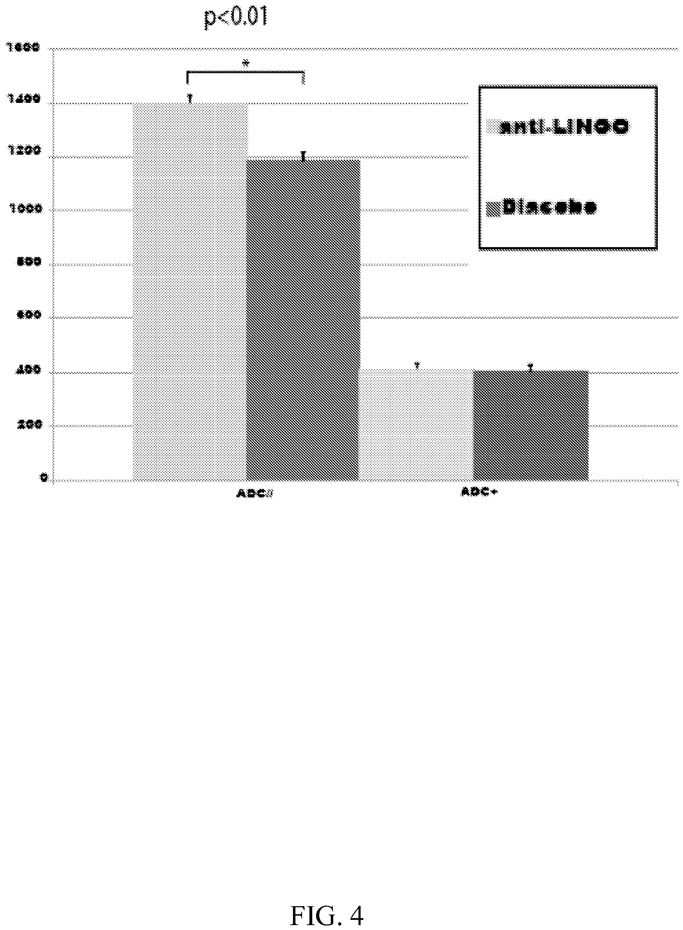

Optic nerve diffusion MRI scans were conducted on 16 surviving control-treated mice and 18 surviving anti-LINGO-1 antibody treated mice at peak EAE severity on days 16-17 post induction. The optic nerve ROI images comprised 10 voxels in the center of the optic nerves at the prechiasmal level. Diffusion tensor imaging (DTI) showed significantly higher apparent diffusion coefficient (ADC) values parallel to the long axis (longitudinal, axial, or parallel diffusivity or λII) in anti-LINGO-1 antibody treated mice (mean 1,400, SD 27 msec) than in control-treated mice (mean 1,183, SD 36 msec) (FIG. 3, FIG. 4). In contrast, there was no difference by treatment group in ADC values perpendicular to the optic nerve long axis (radial or perpendicular diffusivity or λ) (415 ± 19msec in anti-LINGO-1 antagonist treated mice versus 403 ± 25msec in control treated mice) (FIG. 3, FIG. 4).

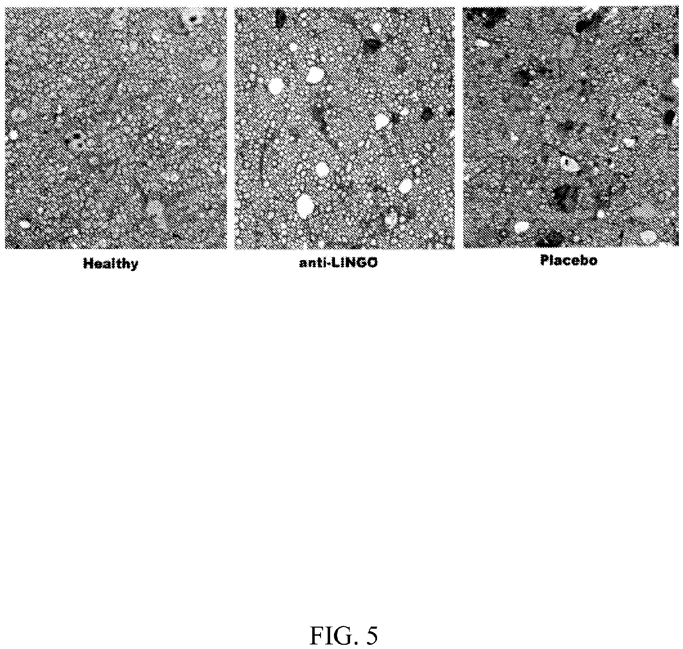

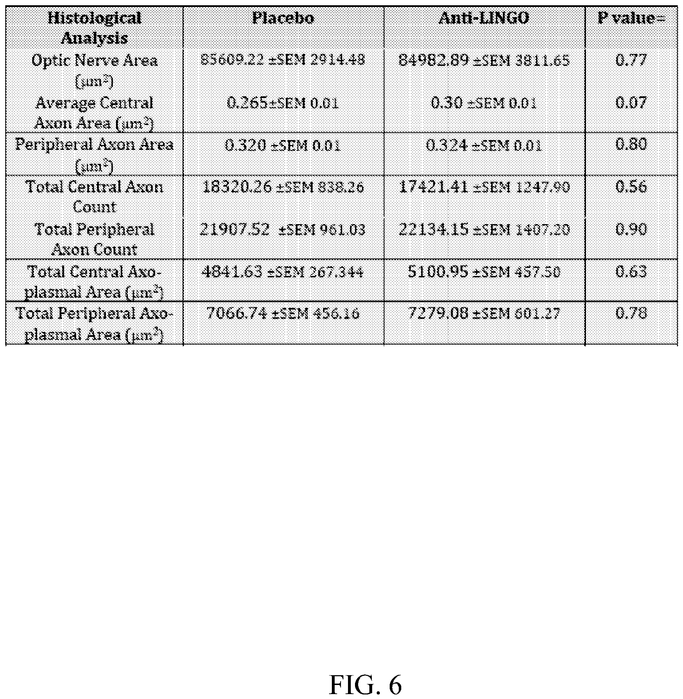

Assessment of central and peripheral axonal areas, axonal counts, and axoplasmal cross sectional areas in the 5 ROIs for each optic nerve examined were tabulated for comparison (FIG. 5; FIG. 6). Nineteen EAE nerves per condition and 5 healthy nerves were analyzed. The results showed there was no difference in the total optic nerve area or the number of axons in the ROIs with the central or peripheral (FIG. 5; FIG. 6). However, the individual axonal area (a measure of axonal health) in the central optic nerve ROI was 13% lower in the control-treated mice relative to the anti-LINGO-1 antibody treated mice (FIG. 5; FIG. 6). Overall, LINGO-1 antagonism reduced the morbidity and mortality from MOG-EAE in mice; and promoted axonal protection in the inflamed optic nerve by reducing the loss of axonal area and reducing the loss of axial diffusivity. In summary, damage to the optic nerve was seen in MOG-EAE histologically and by MRI, and it appeared to be reduced by LINGO-1 blockade.

### Example 2. LINGO-1 antagonism in combination with corticosteroid treatment provides increased axonal protection compared to LINGO-1 antagonism alone in the rat EAE model.

Acute optic neuritis (AON) is an inflammatory disease of the optic nerve that often occurs in multiple sclerosis. AON is caused by inflammatory injury to the optic nerve and presents with visual loss due to edema, inflammation, and damage to the myelin sheath covering the optic nerve and axons. There is significant loss of the retinal nerve fiber layer and retinalganglion cell layer as a result of AON. Current treatment of AON is limited to intravenous treatment with high dose corticosteroids which fasten the resolution of edema, but do not promote central nervous system (CNS) remyelination or provide neuroaxonal protection from CNS inflammatory demyelination.

Animal models for the study of optic neuritis include the rat and mouse experimental autoimmune encephalomyelitis (EAE) models; in which EAE induction results in the development of optic nerve neuritis. In the present example, the effects of LINGO-1 antagonism alone and in combination with corticosteroid treatment were analyzed using the EAE rat model. Briefly, female Brown Norway (BN) rats 8 to 10 weeks of age were anesthetized by inhalation of isoflurane and injected intradermally at the base of the tail with a total volume of 200 µl of inoculums, containing 100 µg rMOG1-125 in saline emulsified (1:1) with complete Freud's adjuvant containing 400 µg heat inactivated mycobacterium tuberculosis.

After the onset of clinical symptoms (15-16 days post EAE induction), rats were treated with 30mg/kg/day of methylprednisolone (MP) in saline solution or saline solution alone (Veh) intravenously for three consecutive days. On the second day of MP injection, rats were given either 6mg/kg of the anti-LINGO-1 monoclonal antibody or control antibody, administered intrapetitoneally once a week for three weeks. There were a total of four different treatment groups: (1) control treatment group (Veh + control Antibody (Ab)); (2) MP treatment group (MP + control antibody); (3) anti-LINGO-1 monoclonal antibody treatment group (Veh + anti-LINGO-1 monoclonal antibody); and (4) combination treatment group (MP + anti-LINGO-1 monoclonal antibody).

One week post the last treatment (4 weeks post the onset of symptoms and 6 weeks post EAE induction), the rats were perfused with 4% paraformaldehyde (PFA) in PBS and cryostat microtome sections of optic nerves (ONs) were stained with anti-βIII tubulin antibody to analyze axonal pathology using DAPI and visualized by fluorescence microscopy at 40X magnification. For axonal quantification, 3 different sections per optic nerve were analyzed, and 3-5 animals were counted per treatment group.

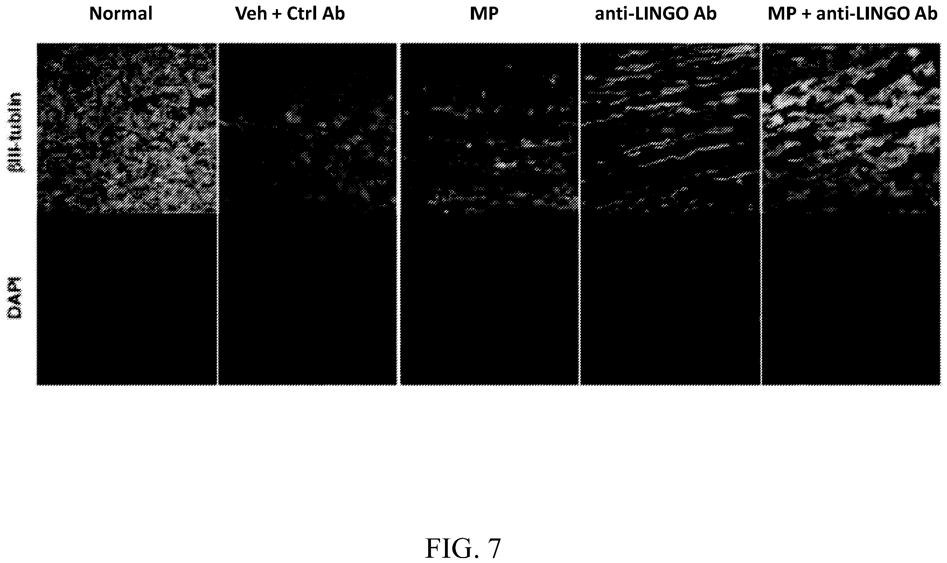

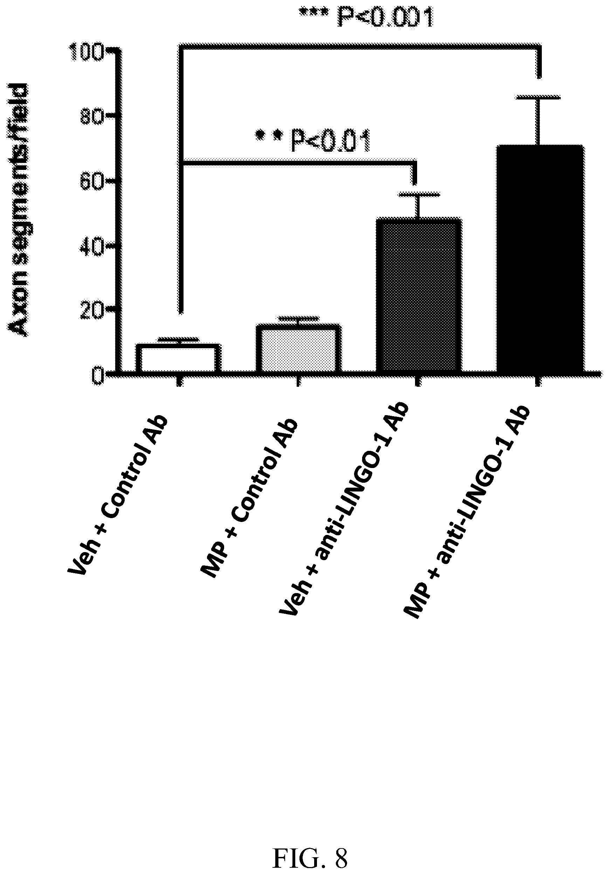

As shown in FIG. (FIG. 7), axonal loss was detected in the sections of optic nerves of the control treatment group (Veh + control Antibody) by anti-βIII tubulin staining, suggesting severe axonal loss. Inflammatory infiltration was also observed ion these areas as shown by DAPI staining (FIG. 7). The number of axons was slightly higher in the MP treatment group (MP + control antibody) (p value = non significant) (FIG. 8). The anti-LINGO-1 monoclonal antibody treatment group (Veh + anti-LINGO-1 monoclonal antibody) showed 5-fold higher axonal numbers, suggesting that anti-LINGO-1 monoclonal antibody treatment prevented axonal loss (FIG. 8). The combination treatment group (MP + anti-LINGO-1 monoclonal antibody) showed an 8-fold increase in axonal numbers compared with the control treatment group (Veh + control Antibody; suggesting that combination treatment of anti-LINGO-1 antagonist such as an anti-LINGO-1 monoclonal antibody with high dose corticosteroids can have a synergistic effect (FIG. 8).

Overall, LINGO-1 antagonism reduced axonal loss in the optic nerve in rat EAE. While axonal protection was not seen with daily treatment for three days with high dose intravenous methylprednisolone, anti-LINGO-1 monoclonal antibody treatment resulted in axonal protection in inflammatory demyelination; and combination treatment of the anti-LINGO-1 monoclonal antibody with high dose IV methylprednisolone resulted in even greater axonal protection.

In summary, LINGO-1 blockade with the monoclonal anti-LINGO-1 antibody reduced axonal loss in rat MOG-EAE; and the neuroprotective effects of LINGO-1 blockade in rat MOG-EAE were seen in the presence or absence of anti-inflammatory treatment with high-dose steroids.

### Incorporation by Reference

The contents of all references, figures, sequence listing, patents and published patent applications cited throughout this application are hereby incorporated by reference. All publications, patents, and patent applications mentioned herein are hereby incorporated by reference in their entirety as if each individual publication, patent or patent application was specifically and individually indicated to be incorporated by reference. In case of conflict, the present application, including any definitions herein, will control.

Also incorporated by reference in their entirety are any polynucleotide and polypeptide sequences which reference an accession number correlating to an entry in a public database, such as those maintained by The Institute for Genomic Research (TIGR) on the worldwide web at tigr.org and/or the National Center for Biotechnology Information (NCBI) on the worldwide web at ncbi.nlm.nih.gov.

### Equivalents

Those skilled in the art will recognize, or be able to ascertain using no more than routine experimentation, many equivalents to the specific embodiments of the invention described herein. Such equivalents are intended to be encompassed. Preferred embodiments of the present invention are described below and are referred to as embodiments E1 to E65.
E1. A method of treating a CNS demyelinating disease chosen from multiple sclerosis or an inflammatory condition of the optic nerve, in a subject in need thereof, said method comprising administering to the subject an anti-LINGO-1 antibody molecule and an immunosuppressive agent in an amount sufficient to treat the CNS demyelinating disease, wherein the immunosuppressive agent is chosen from one or more of:
   an IFN-β 1 molecule;
   a corticosteroid;
   a polymer of glutamic acid, lysine, alanine and tyrosine or glatiramer;
   an antibody or fragment thereof against alpha-4 integrin or natalizumab;
   an anthracenedione molecule or mitoxantrone;
   a fingolimod or FTY720 or other SIP1 functional modulator;
   a dimethyl fumarate;
   an antibody to the alpha subunit of the IL-2 receptor of T cells or daclizumab;
   an antibody against CD52 or alemtuzumab;
   an antibody against CD20; or
   an inhibitor of a dihydroorotate dehydrogenase or teriflunomide;
   thereby treating the CNS demyelinating disease.
E2. The method of claim E1, wherein the CNS demyelinating disease is multiple sclerosis.
E3. The method of claim E1, wherein the inflammatory condition of the optic nerve is optic neuritis.
E4. The method of claim E3, wherein the optic neuritis is acute optic neuritis.
E5. A method of treating multiple sclerosis or optic neuritis, in a subject in need thereof, said method comprising administering to the subject an anti-LINGO-1 antibody molecule, and an IFN-β 1 molecule, in an amount sufficient to treat the multiple sclerosis or optic neuritis.
E6. A method of treating multiple sclerosis or optic neuritis, in a subject in need thereof, said method comprising administering to the subject an anti-LINGO-1 antibody molecule and a corticosteroid, in an amount sufficient to treat the multiple sclerosis or the optic neuritis.
E7. The method of any of E1-6, wherein the anti-LINGO-1 antibody molecule causes one or more of: enhances myelination, enhances neuroaxonal protection, promotes differentiation and survival of oligodendrocytes, enhances synapse number or synapse efficiency, or accelerates conduction velocity.
E8. The method of any of E1-7, wherein the anti-LINGO-1 antibody molecule is a monoclonal antibody against human LINGO-1.
E9. The method of E8, wherein the anti-LINGO-1 antibody molecule is a human, humanized, a CDR-grafted, or an *in vitro-generated* antibody against human LINGO-1.
E10. The method of either of E8 or E9, wherein the anti-LINGO-1 antibody molecule is an immunoglobulin G subclass 1 (IgG1).
E11. The method of any of E1-10, wherein the anti-LINGO-1 antibody molecule comprises an aglycosyl (IgG1) framework.
E12. The method of any of E1-11, wherein the anti-LINGO-1 antibody molecule is modified to reduce effector cell and complement function compared to wild-type IgG1.
E13. The method of any of E1-12, wherein the anti-LINGO-1 antibody molecule comprises one, two or three CDRs of a heavy chain variable domain comprising the amino acid sequence of SEQ ID NO: 6, 7 or 8, or SEQ ID NO: 2, 3 or 30, or a sequence substantially identical thereto.
E14. The method of any of E1-12, wherein the anti-LINGO-1 antibody molecule comprises one, two or three CDRs of a light chain variable domain comprising the amino acid sequence of SEQ ID NO: 14, 15 or 16, or SEQ ID NO: 10, 11 or 12, or a sequence substantially identical thereto.
E15. The method of any of E1-12, wherein the anti-LINGO-1 antibody molecule comprises a heavy chain variable domain comprising the amino acid sequence of SEQ ID NO: 5 or SEQ ID NO: 66, or a sequence substantially identical thereto.
E16. The method of any of E1-12, wherein the anti-LINGO-1 antibody molecule comprises a light chain variable domain comprising the amino acid sequence of SEQ ID NO: 13 or SEQ ID NO: 9, or a sequence substantially identical thereto.
E17. The method of any of E1-12, wherein the anti-LINGO-1 antibody molecule comprises a heavy chain comprising the amino acid sequence of SEQ ID NO: 275, or a sequence substantially identical thereto; and a light chain comprising the amino acid sequence of SEQ ID NO: 276, or a sequence substantially identical thereto.
E18. The method of any of E1-17, wherein the IFN-β 1 molecule comprises one or more of an IFN-β 1a or IFN-β 1b polypeptide, a variant, a homologue, a fragment or a pegylated variant thereof.
E19. The method of any of E1-17, wherein the IFN-β 1 molecule comprises an IPNβ agent chosen from an IFN-β la molecule, an IFN-β 1b molecule, or a pegylated variant of an IFN-β 1a molecule or an IFN-β 1b molecule.
E20. The method of E19, wherein the IFN-β la molecule is Avonex® or Rebif®; and the IFNβ-1b molecule is Betaseron® or Betaferon® or Extavia®.
E21. The method of any of E1-20, wherein the anti-LINGO-1 antibody molecule comprises a heavy chain variable domain comprising the amino acid sequence of SEQ ID NO: 5 or SEQ ID NO: 66, and a light chain variable domain comprising the amino acid sequence of SEQ ID NO: 13 or SEQ ID NO: 9; and the immunosuppressive agent is Avonex®.
E22. The method of any of E1-21, wherein the subject has one of: relapsing-remitting multiple sclerosis (RRMS), primary progressive MS, secondary progressive MS, clinically isolated syndrome (CIS), clinically defined MS (CDMS), or benign MS.
E23. The method of E22, wherein the subject has one or more newly developed lesions.
E24. The method of E22, wherein the subject has one or more pre-existing lesion.
E25. The method of any of E1-21, wherein the subject has relapsing-remitting multiple sclerosis (RRMS).
E26. The method of any of E1-21, wherein the subject has secondary progressive MS (SPMS).
E27. The method of E25 or E26, wherein the subject is a patient with active disease.
E28. The method of any of E1-27, wherein said treating step comprises reducing one or more symptoms associated with the disease; or reducing retarding or preventing, a relapse, or the worsening of a disability, in the subject.
E29. The method of any of E1-28, wherein the anti-LINGO-1 antibody molecule and the immunosuppressive agent are administered concurrently.
E30. The method of any of E1-28, wherein the anti-LINGO-1 antibody molecule and the immunosuppressive agent are administered sequentially.
E31. The method of any of E1-28, wherein the administration of the anti-LINGO-1 antibody molecule and the immunosuppressive agent overlaps in part with each other.
E32. The method of any of E1-28, wherein initiation of the administration of the immunosuppressive agent and the anti-LINGO-1 antibody molecule occurs at the same time.
E33. The method of any of E1-28, wherein the immunosuppressive agent is administered before initiating treatment with the anti-LINGO-1 antibody molecule.
E34. The method of any of E1-28, wherein the anti-LINGO-1 antibody molecule is administered before initiating treatment with the immunosuppressive agent.
E35. The method of any of E1-28, wherein administration of the immunosuppressive agent continues after cessation of administration of the anti-LINGO-1 antibody molecule.
E36. The method of any of E1-28, wherein administration of the anti-LINGO-1 antibody molecule continues after cessation of administration of the immunosuppressive agent.
E37. The method of any of E1-36, wherein the anti-LINGO-1 antibody molecule is an antibody molecule against LINGO-1 and is administered intravenously, subcutaneously, intravitreally, intrathecally or intramuscularly.
E38. The method of E40, wherein the anti-LINGO-1 antibody molecule is administered intravenously.
E39. The method of any of E1-38, wherein the anti-LINGO-1 antibody molecule is dosed at 1 to 100 mg/kg.
E40. The method of any of E1-38, wherein the anti-LINGO-1 antibody molecule is dosed at about 3 mg/kg, about 10 mg/kg, about 30 mg/kg, about 50mg/kg, or about 100 mg/kg.
E41. The method of any of E1-40, wherein the anti-LINGO-1 antibody molecule is administered once every one, two, three, four or five weeks by IV infusion.
E42. The method of any of E1-41, wherein the immunosuppressive agent is an IFN-β 1 molecule is administered intravenously, subcutaneously or intramuscularly.
E43. The method of E42, wherein the IFN-β 1 molecule is administered at one or more of:
   (i) at 20-45 microgram once a week via intramuscular injection;
   (ii) at 20-30 microgram three times a week, or at 40-50 micrograms three times a week, via subcutaneous injection; or
   (iii) in an amount of between 10 and 50 µg intramuscularly, three times a week, or every five to ten days once a week.
E44. The method of any of E1-43, wherein:
   the anti-LINGO-1 antibody molecule is administered once every four weeks by IV infusion dosed at about 3 mg/kg, about 10 mg/kg, about 30 mg/kg, about 50mg/kg, or about 100 mg/kg; and
   the immunosuppressive agent is IFN-β 1 and is administered at one or more of:
      (i) at 20-45 microgram once a week via intramuscular injection;
      (ii) at 20-30 microgram once or three times a week, or at 40-50 micrograms once or three times a week, via subcutaneous injection; or
      (iii) in an amount of between 10 and 50 µg intramuscularly, e.g., three times a week, or every five to ten days.
E45. The method of any of E1-44, wherein the subject has been, or is being evaluated by one or more of:
   performing a neurological examination;
   acquiring the subject's status on the Expanded Disability Status Scale (EDSS);
   acquiring the subject's status on the Multiple Sclerosis Functional Composite (MSFC);
   detecting the subject's lesion status;
   acquiring a measure of upper and/or lower extremity function;
   acquiring a measure of short distance ambulatory function;
   acquiring a measure of long distance ambulatory function;
   acquiring a measure of cognitive function; or
   acquiring a measure of visual function.
E46. The method of any of E1-44, further comprising one or more of:
   acquiring the subject's status on the MSFC;
   performing a neurological examination;
   acquiring the subject's status on the Expanded Disability Status Scale (EDSS);
   detecting the subject's lesion status;
   acquiring a measure of upper and/or lower extremity function;
   acquiring a measure of short distance ambulatory function;
   acquiring a measure of long distance ambulatory function;
   acquiring a measure of cognitive function; or
   acquiring a measure of visual function.
E47. The method of E45 or £46, wherein the measure of upper extremity function is acquired using a 9 Hole Peg Test (9HP).
E48. The method of E45 or £46, wherein the measure of short distance ambulatory function is acquired using a Timed Walk of 25 Feet (T25FW).
E49. The method of E45 or £46, wherein the measure of cognitive function comprises an evaluation of a learning test, a memory test and/or an attention/processing speed test.
E50. The method of any of E1-44, wherein the subject is evaluated using an EDSS assessment and an assessment of ambulatory function chosen from one, two, three, or all of an assessment of : short distance ambulatory function, long distance ambulatory function, upper extremity function or lower extremity function.
E51. The method of E50, wherein an increase by at least 10%, 15%, 20%, 25% or higher in a measure of extremity and/or ambulatory function is indicative of disease progression in the subject; and a decrease of at least 10%, 15%, 20%, 25% or more in a measure of extremity and/or ambulatory function is indicative of an improved outcome in the subject.
E52. The method of E49, wherein the measure of cognitive function comprises an evaluation of one or more of auditory memory, verbal learning and/or remembering verbal information (e.g., Selective Reminding Test (SRT)); tests for evaluating auditory/verbal memory (e.g., California Verbal Learning Test Second Edition (CVLT2)), the Rey Auditory Verbal Learning Test (RAVLT); tests for evaluating visual/spatial memory (e.g., Brief Visuospatial Memory Test Revised (BVMTR)); cognition tests, e.g., PASAT, SDMT; and patient reported outcome measures (e.g. MSWS-12, MSIS-29, ABILHAND, MSNQ, and/or SF-36).
E53. The method of either of E45 or E46, wherein the measure of cognitive function is performed using a composite of MS cognitive endpoint that comprises SDMT, PASAT-3 and -3, SRT-Total Learned (SRT-TL), SRT Delayed Recall (SRT-DR), and BVMTR Delayed Recall (BVMTR-DR).
E54. The method of E53, wherein the measure of cognitive function comprises an MS-COG.
E55. The method of any of E45-54, wherein an improvement in the subject is defined by one or more of:
   a. ≥1.0 point decrease in EDSS from a baseline score of ≤6.0;
   b. ≥15% improvement from baseline in T25FW;
   c. ≥15% improvement from baseline in 9HPT; or
   d. ≥15% improvement from baseline in PASAT.
E56. The method of any of E45-54, wherein the subject's lesion status is evaluated using magnetic resonance imaging.
ES7. The method of E56, wherein the magnetic resonance imaging comprises magnetic transfer and diffusion tensor imaging.
E58. A kit comprising an antibody molecule against human LINGO-1 and an IFN-β 1 molecule with instructions for use in treating multiple sclerosis or an inflammatory condition of the optic nerve.
E59. A packaged composition comprising an antibody molecule against human LINGO-1 and an IFN-β 1 molecule with instructions for use in treating multiple sclerosis or an inflammatory condition of the optic nerve.
E60. A method of treating acute optic neuritis, in a subject in need thereof, said method comprising administering to the subject an anti-LINGO-1 antibody molecule in an amount sufficient to treat the acute optic neuritis.
E61. The method of E60, wherein the anti-LINGO-1 antibody molecule comprises one, two or three CDRs of a heavy chain variable domain comprising the amino acid sequence of SEQ ID NO: 6, 7 or 8, or SEQ ID NO: 2, 3 or 30, or a sequence substantially identical thereto.
E62. The method of E60, wherein the anti-LINGO-1 antibody molecule comprises one, two or three CDRs of a light chain variable domain comprising the amino acid sequence of SEQ ID NO: 14, 15 or 16, or SEQ ID NO: 10, 11 or 12, or a sequence substantially identical thereto.
E63. The method of E60, wherein the antibody molecule comprises a heavy chain variable domain comprising the amino acid sequence of SEQ ID NO: 5 or SEQ ID NO: 66, or a sequence substantially identical thereto.
E64. The method of E60, wherein the antibody molecule comprises a light chain variable domain comprising the amino acid sequence of SEQ ID NO: 13 or SEQ ID NO: 9, or a sequence substantially identical thereto.
E65. The method of E60, wherein the antibody molecule comprises a heavy chain comprising the amino acid sequence of SEQ ID NO: 275, or a sequence substantially identical thereto; and a light chain comprising the amino acid sequence of SEQ ID NO: 276, or a sequence substantially identical thereto.

## Claims

1. An anti-LINGO-1 antibody molecule for use in a method of treating a CNS demyelinating disease chosen from multiple sclerosis or an inflammatory condition of the optic nerve in a subject in need thereof,
wherein said method comprises administering the anti-LINGO-1 antibody molecule and an immunosuppressive agent to the subject in an amount sufficient to treat the CNS demyelinating disease, wherein the immunosuppressive agent is chosen from one or more of:
an IFN-β 1 molecule;
a corticosteroid;
a polymer of glutamic acid, lysine, alanine and tyrosine or glatiramer;
an antibody or fragment thereof against alpha-4 integrin or natalizumab;
an anthracenedione molecule or mitoxantrone;
a fingolimod or FTY720 or other SIP1 functional modulator;
a dimethyl fumarate;
an antibody to the alpha subunit of the IL-2 receptor of T cells or daclizumab;
an antibody against CD52 or alemtuzumab;
an antibody against CD20; or
an inhibitor of a dihydroorotate dehydrogenase or teriflunomide.

2. The anti-LINGO-1 antibody for use according to claim 1, wherein:
(i) the CNS demyelinating disease is multiple sclerosis, optionally wherein the multiple sclerosis is relapsing-remitting multiple sclerosis (RRMS), primary progressive MS, secondary progressive MS, clinically isolated syndrome (CIS), clinically defined MS (CDMS), or benign MS; or
(ii) the inflammatory condition of the optic nerve is optic neuritis, optionally wherein the optic neuritis is acute optic neuritis.

3. The anti-LINGO-1 antibody for use according to claim 2, wherein the immunosuppressive agent is an IFN-β 1 molecule; or is a corticosteroid.

4. The anti-LINGO-1 antibody for use according to any of claims 1-3, wherein the anti-LINGO-1 antibody molecule comprises an aglycosyl (IgG1) framework and/or wherein the anti-LINGO-1 antibody molecule is modified to reduce effector cell and complement function compared to wild-type IgG1.

5. The anti-LINGO-1 antibody for use according to any of claims 1-4, wherein the anti-LINGO-1 antibody molecule comprises:
(i) a heavy chain variable domain comprising the amino acid sequence of SEQ ID NO: 5 or SEQ ID NO: 66, and a light chain variable domain comprising the amino acid sequence of SEQ ID NO: 13 or SEQ ID NO: 9; and wherein the immunosuppressive agent is Avonex®;
(ii) one, two or three CDRs of a heavy chain variable domain comprising the amino acid sequence of SEQ ID NO: 6, 7 or 8, or SEQ ID NO: 2, 3 or 30, or a sequence substantially identical thereto;
(iii) one, two or three CDRs of a light chain variable domain comprising the amino acid sequence of SEQ ID NO: 14, 15 or 16, or SEQ ID NO: 10, 11 or 12, or a sequence substantially identical thereto;
(iv) a heavy chain variable domain comprising the amino acid sequence of SEQ ID NO: 5 or SEQ ID NO: 66, or a sequence substantially identical thereto;
(v) a light chain variable domain comprising the amino acid sequence of SEQ ID NO: 13 or SEQ ID NO: 9, or a sequence substantially identical thereto; or
(vi) a heavy chain comprising the amino acid sequence of SEQ ID NO: 275, or a sequence substantially identical thereto; and a light chain comprising the amino acid sequence of SEQ ID NO: 276, or a sequence substantially identical thereto.

6. The anti-LINGO-1 antibody for use according to any of claims 1-5, wherein the IFN-β1 molecule comprises:
(i) one or more of an IFN-β 1a or IFN-β 1b polypeptide, a variant, a homologue, a fragment or a pegylated variant thereof; or
(ii) an IPNβ agent chosen from an IFN-β la molecule, an IFN-β 1b molecule, or a pegylated variant of an IFN-β la molecule or an IFN-β 1b molecule, optionally wherein the IFN-β 1a molecule is Avonex® or Rebif®; and the IFNβ-1b molecule is Betaseron® or Betaferon® or Extavia®.

7. The anti-LINGO-1 antibody for use according to any of claims 2-6, wherein the subject has:
(i) one or more newly developed lesions, or one or more pre-existing lesion; or
(ii) relapsing-remitting multiple sclerosis (RRMS) or secondary progressive MS (SPMS), optionally wherein the subject is a patient with active disease.

8. The anti-LINGO-1 antibody for use according to any of claims 1-7, wherein said treating comprises reducing one or more symptoms associated with the CNS demyelinating disease; and/or reducing retarding or preventing, a relapse, or the worsening of a disability, in the subject.

9. The anti-LINGO-1 antibody for use according to any of claims 1-8, wherein:
(i) the anti-LINGO-1 antibody molecule and the immunosuppressive agent are administered concurrently;
(ii) the anti-LINGO-1 antibody molecule and the immunosuppressive agent are administered sequentially;
(iii) the administration of the anti-LINGO-1 antibody molecule and the immunosuppressive agent overlaps in part with each other;
(iv) initiation of the administration of the immunosuppressive agent and the anti-LINGO-1 antibody molecule occurs at the same time;
(v) the immunosuppressive agent is administered before initiating treatment with the anti-LINGO-1 antibody molecule;
(vi) the anti-LINGO-1 antibody molecule is administered before initiating treatment with the immunosuppressive agent;
(vii) administration of the immunosuppressive agent continues after cessation of administration of the anti-LINGO-1 antibody molecule; or
(viii) administration of the anti-LINGO-1 antibody molecule continues after cessation of administration of the immunosuppressive agent.

10. The anti-LINGO-1 antibody for use according to any of claims 1-9, wherein the anti-LINGO-1 antibody molecule is an antibody molecule against LINGO-1 and is:
(i) administered intravenously, subcutaneously, intravitreally, intrathecally or intramuscularly, preferably administered intravenously;
(ii) dosed at 1 to 100 mg/kg;
(iii) dosed at about 3 mg/kg, about 10 mg/kg, about 30 mg/kg, about 50mg/kg, or about 100 mg/kg; and/or
(iv) administered once every one, two, three, four or five weeks by IV infusion.

11. The anti-LINGO-1 antibody for use according to any of claims 1-10, wherein the immunosuppressive agent is an IFN-β 1 molecule and is administered intravenously, subcutaneously or intramuscularly, optionally wherein the IFN-β 1 molecule is administered at one or more of:
(i) at 20-45 microgram once a week via intramuscular injection;
(ii) at 20-30 microgram three times a week, or at 40-50 micrograms three times a week, via subcutaneous injection; or
(iii) in an amount of between 10 and 50 µg intramuscularly, three times a week, or every five to ten days once a week.

12. The anti-LINGO-1 antibody for use according to any of claims 1-11, wherein:
the anti-LINGO-1 antibody molecule is administered once every four weeks by IV infusion dosed at about 3 mg/kg, about 10 mg/kg, about 30 mg/kg, about 50mg/kg, or about 100 mg/kg; and
the immunosuppressive agent is IFN-β 1 and is administered at one or more of:
(i) at 20-45 microgram once a week via intramuscular injection;
(ii) at 20-30 microgram once or three times a week, or at 40-50 micrograms once or three times a week, via subcutaneous injection; or
(iii) in an amount of between 10 and 50 µg intramuscularly, *e.g.*, three times a week, or every five to ten days.

13. The anti-LINGO-1 antibody for use according to any of claims 1-12, wherein the subject is evaluated using an EDSS assessment and an assessment of ambulatory function chosen from one, two, three, or all of an assessment of: short distance ambulatory function, long distance ambulatory function, upper extremity function or lower extremity function; optionally wherein an increase by at least 10%, 15%, 20%, 25% or higher in a measure of extremity and/or ambulatory function is indicative of disease progression in the subject; and a decrease of at least 10%, 15%, 20%, 25% or more in a measure of extremity and/or ambulatory function is indicative of an improved outcome in the subject.

14. The anti-LINGO-1 antibody for use according to claim 13, wherein an improvement in the subject is defined by one or more of:
a. ≥1.0 point decrease in EDSS from a baseline score of ≤6.0;
b. ≥15% improvement from baseline in T25FW;
c. ≥15% improvement from baseline in 9HPT; or
d. ≥15% improvement from baseline in PASAT.

15. A kit or a packaged composition comprising an anti-LINGO-1 antibody molecule and an immunomodulatory agent, wherein the immunomodulatory agent is chosen from one or more of:
an IFN-β 1 molecule;
a corticosteroid;
a polymer of glutamic acid, lysine, alanine and tyrosine or glatiramer;
an antibody or fragment thereof against alpha-4 integrin or natalizumab;
an anthracenedione molecule or mitoxantrone;
a fingolimod or FTY720;
a Sphingosine 1-phosphate modulating agent;
a dimethyl fumarate;
an antibody to the alpha subunit of the IL-2 receptor of T cells or daclizumab;
an antibody against CD52 or alemtuzumab;
an antibody against CD20 or ocrelizumab; or
an inhibitor of a dihydroorotate dehydrogenase or leflunomide or teriflunomide.
